# EUROPEAN PATENT APPLICATION

(11) **EP 4 763 975 A1**
(43) Date of publication of application: **24.06.2026**
(21) Application number: 24854088.2
(22) Date of filing: 09.07.2024
(51) Int. Cl.: C12N 5/071, A61L 27/38, C12M 1/00, C12N 5/073, C12Q 1/02, C12Q 1/70, G01N 33/569, G01N 33/573

(54) **METHOD FOR PRODUCING SMALL INTESTINE TISSUE**

(30) Priority: 15.08.2023 JP 2023132372
(71) Applicant: Kyoto University, Kyoto-shi, Kyoto 606-8501 (JP)
(72) Inventor: TAKAYAMA, Kazuo, Kyoto-shi, Kyoto 606-8501 (JP); DEGUCHI, Sayaka, Kyoto-shi, Kyoto 606-8501 (JP)
(74) Representative: Hoffmann Eitle
(86) International application number: PCT/JP2024/024841
(87) International publication number: WO 2025/037497

(57) **Abstract**

The present invention provides a method for producing a small intestine epithelial cell or a small intestine tissue from a midgut cell, the method comprising: (1) a step of culturing a midgut cell in a medium containing a Wnt agonist, a BMP inhibitor, and a growth factor to obtain an intestinal progenitor cell; and (2) a step of culturing the intestinal progenitor cell obtained in step (1) in a medium containing a growth factor, an adenylate cyclase activator, an MEK inhibitor, a DNA methylation inhibitor, a TGF-β inhibitor, a GSK3β inhibitor, and a γ-secretase inhibitor to obtain a small intestine epithelial cell or a small intestine tissue.

## Description

### FIELD

The present invention provides a method for producing small intestinal epithelial cells or small intestine tissue. In particular, the present invention relates to a method for producing small intestinal epithelial cells or small intestine tissue, including the step of culturing midgut cells under specific conditions, uses of the small intestinal epithelial cells or small intestine tissue obtained by such a method, and the like.

### BACKGROUND

The intestine, one of the major parts of the digestive tract, is an organ where openings of ducts of numerous digestive glands are present and digestion and absorption take place. The intestine is broadly divided into the small intestine and the large intestine, and the small intestine is further divided into the duodenum, jejunum, and ileum in this order from the oral side. There are no definitive treatments for small-intestinal dysfunction, and in many cases, symptomatic treatments such as restricting oral intake and administering total parenteral nutrition are only possible therapeutic approaches. When continuation of total parenteral nutrition becomes difficult due to complications or the like, small intestine transplantation is indicated. However, the small intestine used for transplantation has complex immune defense functions because it is an organ constituting part of the gastrointestinal tract, which is directly exposed to the external environment and performs digestion and absorption. Accordingly, as compared with transplantation of other organs, small intestine transplantation has problems in that controlling rejection is more difficult and post-transplant infectious complications are more frequent.

In addition, absorption, metabolization, and excretion of orally administered drugs take place first in the small intestine. Accordingly, evaluating pharmacokinetics using small intestinal epithelial cells and small intestine tissue is important for effective development of safe pharmaceutical products. However, it is difficult to obtain primary cultured human intestinal epithelial cells, and even if they are obtained, variations in their properties due to donor-to-donor differences give rise to a problem. Moreover, it is also difficult to perform long-term culture of the obtained cells while preserving their functions. On this account, the development of methods for inducing small intestinal epithelial cells or small intestine tissue from pluripotent stem cells has been attracting attention.

For example, a previous study has reported a method for inducing intestinal epithelial cells by culturing definitive endoderm cells derived from human ES cells in a medium containing a GSK inhibitor, BIO (6-bromoindirubin-3'-oxime), and a γ-secretase inhibitor, DAPT (N-[(3,5-Difluorophenyl)acetyl]-L-alanyl-2-phenylglycine-1,1-dimethylethyl ester) (NPL 1). Another previous study has reported a method for inducing small intestinal epithelial cells by culturing intestinal stem cells derived from human iPS cells first in a medium containing Forskolin and then in the medium further supplemented with EGF, PD98059, 5-aza-2'-dC, and A-83-01 (NPL 2). Also, it has been reported that, by isolating crypts from mouse small intestines and culturing preparations containing the crypts on Matrigel, organoids containing at least 40 crypt domains surrounding a central lumen lined with a villus-like epithelium can be generated (NPL 3). Further, it has been reported that small intestine tissue-on-a-chip can be generated by obtaining epithelial cells from a human intestinal tract organoid derived from induced pluripotent stem cells and then incorporating these cells into a microengineered chip (NLP 4). However, these small intestine models are composed only of small intestinal epithelial cells and lack mesenchymal cells that support the epithelial cells, and thus they differ greatly from native small intestine tissue.

### [CITATION LIST]

### [NON PATENT LITERATURE]

[NPL 1] Ogaki et al., Stem Cells. 31(6):1086-1096 (2013)
[NPL 2] Kabeya et al., Drug Metab Pharmacokinet. 35(4):374-382 (2020)
[NPL 3] Sato et al., Nature. 459(7244):262-265 (2009)
[NPL 4] Workman et al., Cell Mol Gastroenterol Hepatol. 5(4):669-677 (2017)

### SUMMARY

### [TECHNICAL PROBLEM]

As described above, in vitro small intestine models fabricated by conventional methods include only small intestinal epithelial cells. Furthermore, there have been no successful examples of constructing a small intestine tissue model that includes both small intestinal epithelial cells having crypt-like structures and villus-like structures and mesenchymal cells (in particular, fibroblasts) that support the small intestinal epithelial cells. With the foregoing in mind, it is an object of the present invention to provide a novel method capable of producing small intestinal epithelial cells or small intestine tissue, in particular, a method capable of producing a novel small intestine tissue model that includes both small intestinal epithelial cells and mesenchymal cells that support the small intestinal epithelial cells.

### [SOLUTION TO PROBLEM]

The inventors of the present invention first made an attempt to develop a novel method for inducing differentiation into small intestinal epithelial cells by improving the method disclosed in NLP 2. The inventors conducted intensive studies and found that, in differentiation induction from midgut cells into small intestinal epithelial cells, the small intestinal epithelial cells are obtained after undergoing a differentiation process involving the following two steps: first, inducing midgut cells into intestinal progenitor cells; and then, inducing these intestinal progenitor cells into small intestinal epithelial cells. More specifically, in the former step, the midgut cells are cultured in the presence of a Wnt agonist and a BMP inhibitor, and in the latter step, the intestinal progenitor cells obtained in the former step are cultured in the presence of a GSK inhibitor, a γ-secretase inhibitor, and the like. The Wnt agonist and the BMP inhibitor are substances that are commonly used for proliferation of intestinal progenitor cells, whereas the GSK inhibitor and the γ-secretase inhibitor are substances that are used, in conventional methods, immediately after induction into definitive endoderm cells. On the other hand, surprisingly, the method according to the present invention could produce small intestinal epithelial cells despite the fact that these substances were used at timings completely different from those in conventional methods.

Through further studies, the inventors found that performing the above-described culture method in a microfluidic device allows small intestine tissue including fibroblasts to be obtained and that flow culture enables the production of small intestine tissue having morphology closer to that of native small intestine tissue, as compared with tissue obtained by conventional methods. Based on these findings, the inventors conducted further studies, which led to completion of the present invention.

Specifically, the present invention provides the following.
[1] A method for producing a small intestinal epithelial cell or a small intestine tissue from a midgut cell, comprising the following steps (1) and (2):
   (1) culturing a midgut cell in a medium containing a Wnt agonist, a BMP inhibitor, and a growth factor to obtain an intestinal progenitor cell; and
   (2) culturing the intestinal progenitor cell obtained in step (1) in a medium containing a growth factor, an adenylate cyclase activator, an MEK inhibitor, a DNA methylation inhibitor, a TGF-β inhibitor, a GSK3β inhibitor, and a γ-secretase inhibitor to obtain a small intestinal epithelial cell or a small intestine tissue.
[2-1] The method according to [1], wherein the Wnt agonist includes a Wnt protein and an R-spondin.
[2-2] The method according to [2-1], wherein at least one Wnt protein is Wnt3A.
[2-3] The method according to [2-1] or [2-2], wherein at least one R-spondin is R-spondin 3.
[2-4] The method according to any one of [2-1] to [2-3], wherein at least one BMP inhibitor is noggin.
[3] The method according to any one of [1] to [2-4], wherein the Wnt agonist and the BMP inhibitor are obtained from a cell expressing them.
[4-1] The method according to any one of [1] to [3], wherein the growth factor used in each of steps (1) and (2) includes EGF.
[4-2] The method according to [4-1], wherein the growth factor used in step (1) includes IGF.
[4-3] The method according to any one of [1] to [4-2], wherein at least one adenylate cyclase activator is Forskolin.
[4-4] The method according to any one of [1] to [4-3], wherein at least one MEK inhibitor is PD98059.
[4-5] The method according to any one of [1] to [4-4], wherein at least one DNA methylation inhibitor is 5-aza-2'-deoxycytidine.
[4-6] The method according to any one of [1] to [4-5], wherein at least one TGF-β inhibitor is A-83-01.
[4-7] The method according to any one of [1] to [4-6], wherein at least one GSK3β inhibitor is BIO.
[4-8] The method according to any one of [1] to [4-7], wherein at least one γ-secretase inhibitor is DAPT.
[5] The method according to any one of [1] to [4-8], wherein steps (1) and (2) are performed in a microfluidic device.
[6] The method according to [5], wherein steps (1) and (2) each includes a step of performing flow culture.
[7] The method according to [6], wherein the flow culture is performed by a peristaltic pump or a shaker.
[8] The method according to any one of [1] to [7], further including, prior to step (1), a step of inducing differentiation of a definitive endoderm cell into a midgut cell.
[9-1] The method according to [8], further including the step of inducing differentiation of a pluripotent stem cell into a definitive endoderm cell.
[9-2] The method according to [9-1], wherein the pluripotent stem cell is an induced pluripotent stem cell or an embryonic stem cell.
[9-3] The method according to [9-1] or [9-2], wherein the pluripotent stem cell is a human pluripotent stem cell.
[10] A small intestinal epithelial cell or a small intestine tissue obtainable by the method according to any one of [1] to [9-3], or a microfluidic device supporting the small intestinal epithelial cell or the small intestine tissue.
[11-1] A small intestine tissue having all of the following features (A) to (F) or a microfluidic device supporting the small intestine tissue:
   (A) the small intestine tissue has an enterocyte;
   (B) the small intestine tissue has a fibroblast;
   (C) the small intestine tissue has a collagen fiber;
   (D) the small intestine tissue expresses one or more (preferably all) genes selected from the group consisting of TGFB1, Wnt5A, and IGF2;
   (E) the small intestine tissue expresses one or more (preferably all) genes selected from the group consisting of MMP1, COL3A1, and TIMP1; and
   (F) the small intestine tissue has drug-metabolizing enzyme activity.
[11-2] The small intestine tissue according to [11-1] having at least one (preferably all) of the following features (G) to (K) or a microfluidic device supporting the small intestine tissue:
   (G) the small intestine tissue is derived from a pluripotent stem cell;
   (H) the small intestine tissue has a goblet cell;
   (I) the small intestine tissue has a Paneth cell;
   (J) the small intestine tissue has an enteroendocrine cell; and
   (K) the small intestine tissue has a crypt-like structure and a villus-like structure.
[11-3] The small intestine tissue according to [11-1] or [11-2] having a feature (L): the small intestine tissue expresses a transporter gene, or a microfluidic device supporting the small intestine tissue.
[11-4] The small intestine tissue according to any one of [11-1] to [11-3] having a feature (M): the small intestine tissue having a tight junction (TJ), an adherens junction (AJ), and a desmosome (DS), or a microfluidic device supporting the small intestine tissue.
[12-1] An agent for transplantation therapy, comprising the small intestinal epithelial cell or a small intestine tissue according to any one of [10] to [11-4].
[12-2] The agent for transplantation therapy according to [12-1] for treatment or prevention of small intestinal diseases.
[13] A method for screening a therapeutic or prophylactic medicament for a viral infection, comprising the following steps (I) to (III):
   (I) contacting the small intestine tissue according to [10] to [11-4] with a virus in the presence or absence of a test substance, and culturing the small intestine tissue;
   (II) evaluating the degree of infection with the virus in the small intestine tissue; and
   (III) when it is evaluated in step (II) that the degree of infection with the virus is lower in the presence of the test substance than in the absence of the test substance, selecting the test substance as a candidate substance for a therapeutic or prophylactic medicament for the viral infection.
[14] The method according to claim 13, wherein the virus belongs to the family *Coronaviridae.*
[15] A method for evaluating the degree of metabolism of a test substance, comprising the following steps (i) to (iii):
   (i) contacting a test substance with the small intestine tissue according to any one of [10] to [11-4];
   (ii) measuring the activity level of a drug-metabolizing enzyme; and
   (iii) based on the activity level of the drug-metabolizing enzyme measured in step (ii), evaluating the degree of metabolism of the test substance catalyzed by the drug-metabolizing enzyme.
[16-1] The method according to [15], wherein the drug-metabolizing enzyme is at least one selected from the group consisting of CYP3A4, UGT1A1, and CYP2C9.
[16-2] The method according to [15] or [16-1], wherein at least one drug-metabolizing enzyme is CYP3A4.
[17] A method for treating or preventing small intestinal diseases, comprising administering or transplanting an effective amount of the small intestinal epithelial cell or a small intestine tissue according to any one of [10] to [11-4] to a mammal.
[18] The small intestinal epithelial cell or the small intestine tissue according to any one of [10] to [11-4] for use in the treatment or the prevention of small intestinal diseases.
[19] Use of the small intestinal epithelial cell or the small intestine tissue according to any one of [10] to [11-4] in the manufacture of a therapeutic or a prophylactic medicament for small intestinal diseases.

### [ADVANTAGEOUS EFFECTS OF INVENTION]

According to the present invention, small intestinal epithelial cells can be produced from midgut cells using a method distinct from conventional methods. Also, according to the method using a microfluidic device, small intestinal epithelial cells and mesenchymal cells such as fibroblasts can be concurrently differentiated from pluripotent stem cells within a single microfluidic device. The mesenchymal cells produce niche factors and extracellular matrix components such as collagen, thereby allowing the small intestinal epithelial cells to exhibit polarity and functions similar to those observed in vivo.

### BRIEF DESCRIPTION OF DRAWINGS

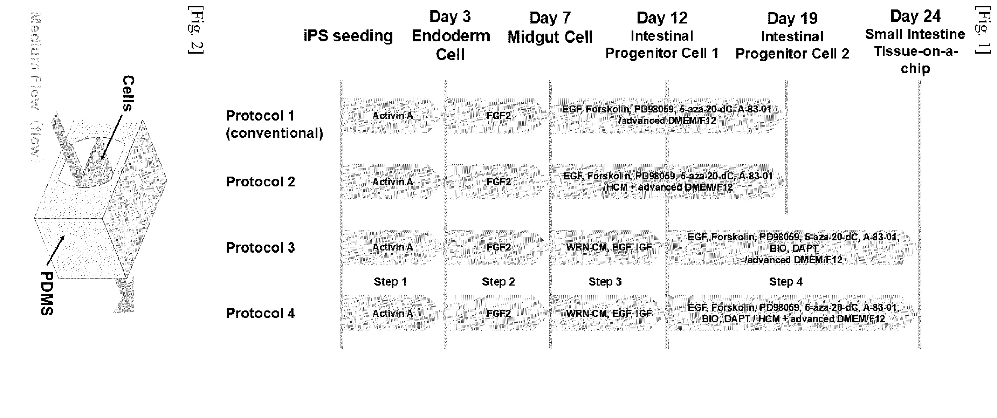

FIG. 1 shows overviews of differentiation induction protocols of pluripotent stem cells into small intestinal epithelial cells or small intestine tissue. Cells were seeded onto a 24-well plate or a microfluidic device on day 3, and then cultured until day 19 or day 24.
FIG. 2 is a schematic diagram of a microfluidic device.

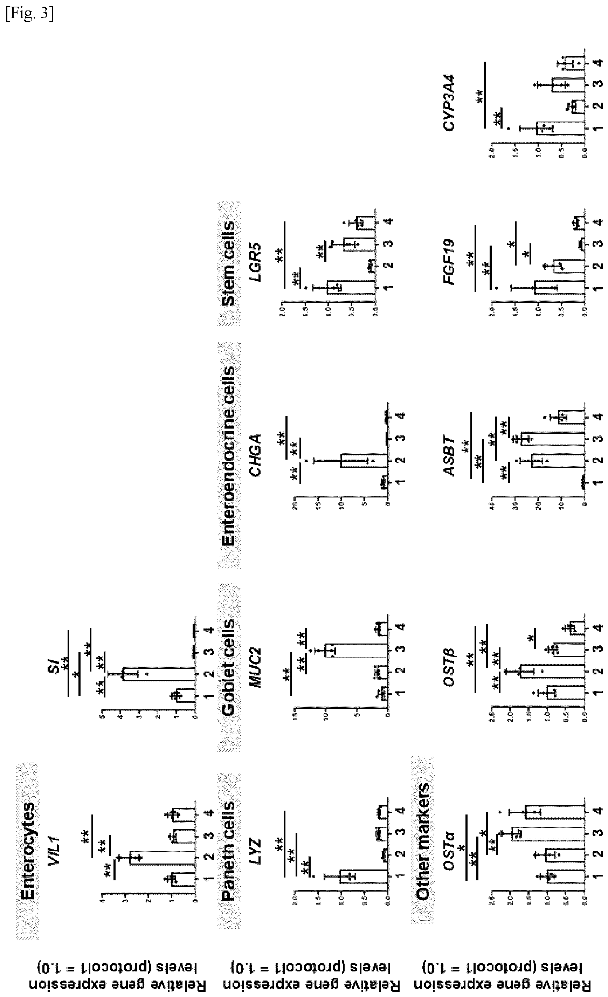

FIG. 3 illustrates differentiation induction into small intestinal epithelial cells using a 24-well plate. Data on the mRNA expression levels of Villin (hereinafter also referred to as Villin 1 (VIL1)), MUC2, LGR5, OSTa, ASBT, and CYP3A4 demonstrate that differentiation into small intestinal epithelial cells can be induced under any of the conditions of protocols 1 to 4 (The overviews of protocols 1 to 4 are as shown in FIG. 1. The same applies hereinafter). n = 5, error bars: standard deviation.

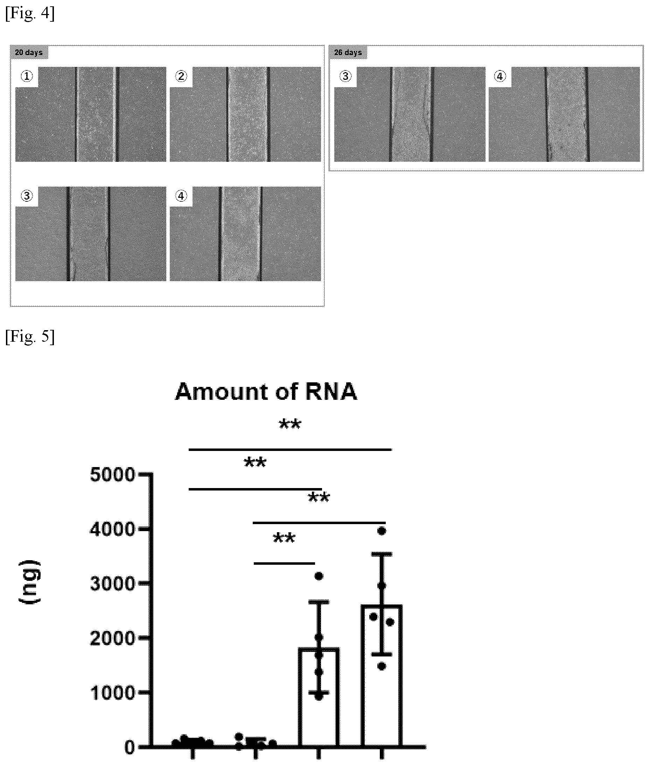

FIG. 4 relates to differentiation induction into small intestine tissue using a microfluidic device. FIG. 4 shows bright field images on day 19 and day 24 in the respective protocols. In protocols 1 and 2, the cells died during culture, and further culture became impossible. On the other hand, in protocols 3 and 4, viable cells were observed on day 19 and day 24.
FIG. 5 relates to differentiation induction into small intestine tissue using a microfluidic device. FIG. 5 shows the amounts of RNA extracted from cells collected after the differentiation induction by the respective protocols. The cells were collected on day 19 in protocols 1 and 2, and on day 24 in protocols 3 and 4. n = 5, error bars: standard deviation.

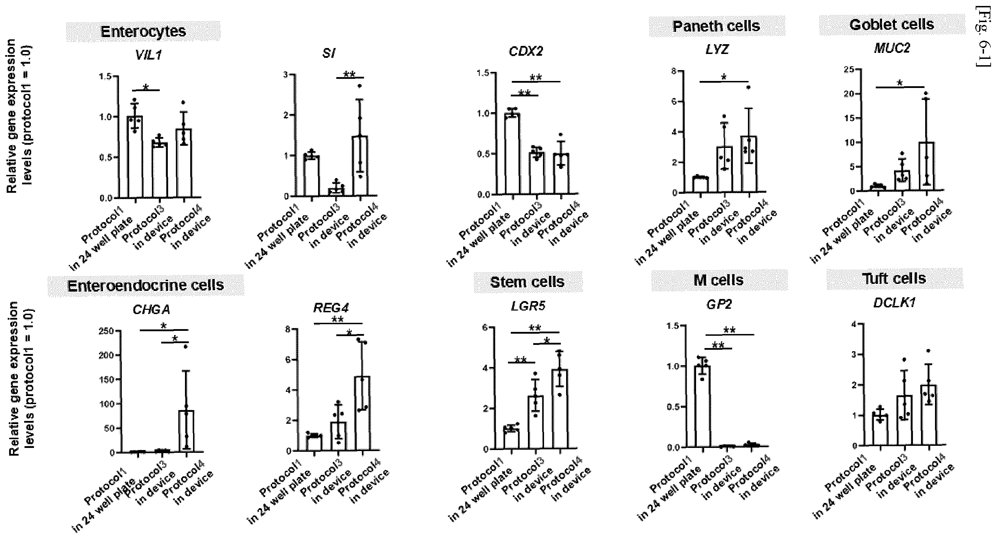

FIG. 6-1 relates to differentiation induction into small intestine tissue using a microfluidic device. FIG. 6-1 shows the results of measuring the mRNA expression levels of small intestinal markers in the respective protocols. n = 5, error bars: standard deviation.

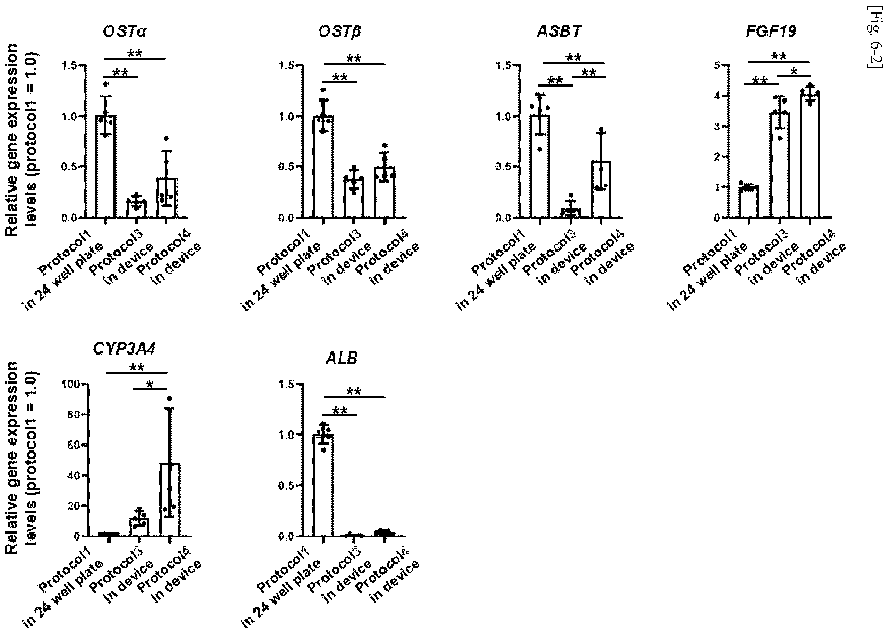

FIG. 6-2 relates to differentiation induction into small intestine tissue using a microfluidic device. FIG. 6-2 shows the results of measuring the mRNA expression levels of small intestinal markers in the respective protocols. Based on the data on the mRNA expression levels of SI, CHGA, REG4, LGR5, ASBT, and CYP3A4, it can be determined that protocol 4 is superior to protocol 3. Moreover, as compared with cells produced on a 24-well plate using protocol 1, more small intestinal marker genes showing high expression were observed in cells obtained using protocol 4. Accordingly, protocol 4 was employed for the following experiments. n = 5, error bars: standard deviation.

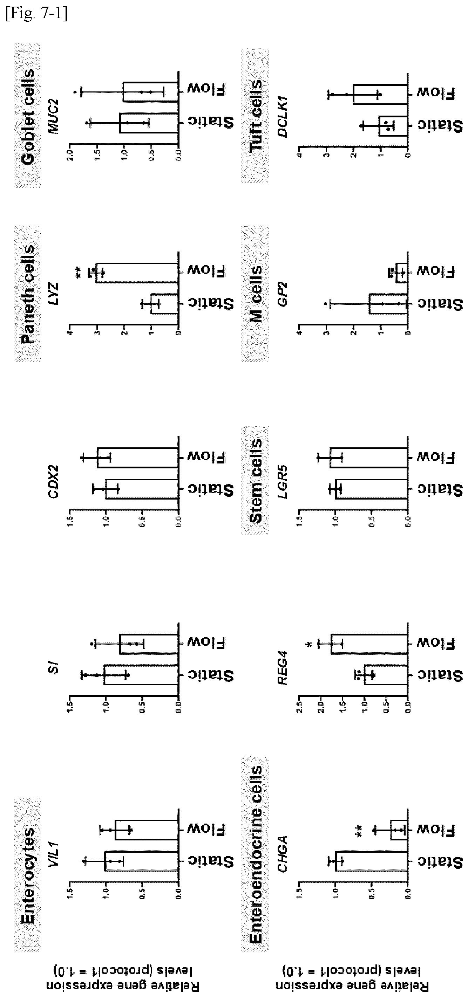

FIG. 7-1 relates to effects of medium flow in small intestine tissue-on-a-chip. The mRNA expression levels of small intestinal markers were compared between static culture (Static) and flow culture (Flow) conditions. n = 3, error bars: standard deviation.

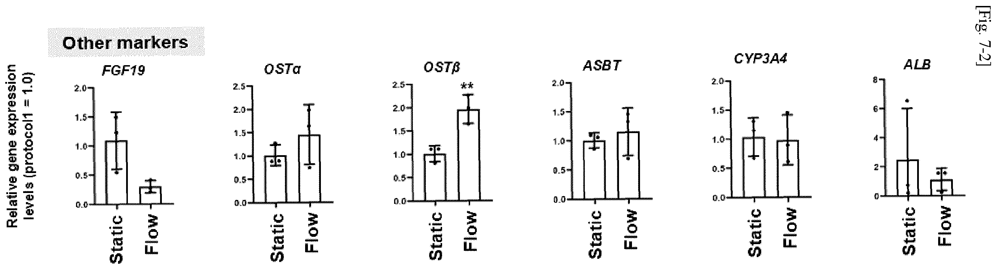

FIG. 7-2 relates to effects of medium flow in small intestine tissue-on-a-chip. The mRNA expression levels of small intestinal markers were compared between static culture (Static) and flow culture (Flow) conditions. For all the markers examined, no marked differences were observed between these conditions. The results also demonstrate that flow culture has minor effect on the expression levels of the small intestinal markers. n = 3, error bars: standard deviation.

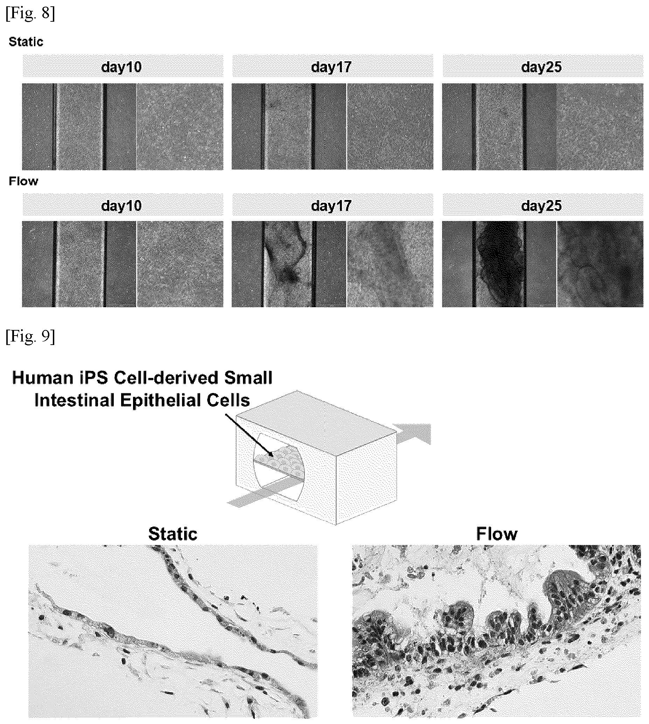

FIG. 8 relates to effects of medium flow in small intestine tissue-on-a-chip. FIG. 8 shows bright-field images on days 10, 17, and 25. The results were compared between static culture (Static) and flow culture (Flow) conditions. The results revealed that medium flow included the formation of three-dimensional small intestine-like tissue. The results further revealed that not only flow into the bottom channel but also flow caused using a shaker can result in the formation of three-dimensional intestine-like tissue.
FIG. 9 relates to effects of medium flow in small intestine tissue-on-a-chip. FIG. 9 shows HE-stained images taken under static or flow culture conditions. Under static culture conditions, a single layer of small intestinal epithelial cells was formed. Under flow culture conditions, structures similar to crypts and villi in small intestine were observed, and also, a lamina propria-like structure and an extracellular matrix-like structure were also observed beneath them.

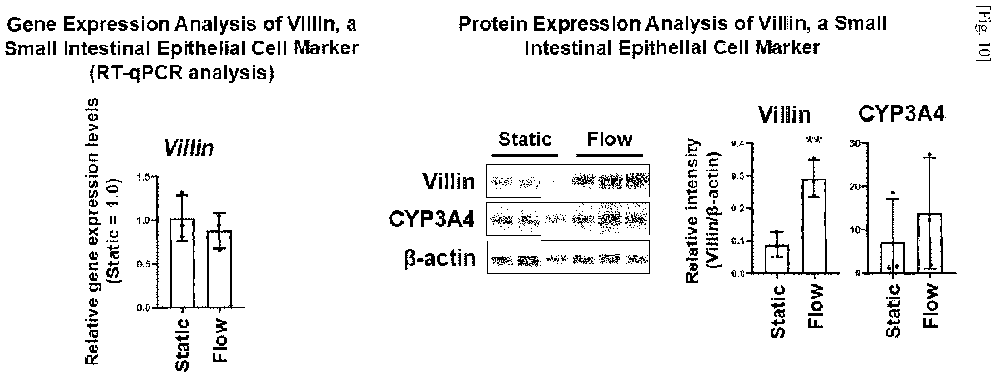

FIG. 10 relates to effects of medium flow in small intestine tissue-on-a-chip. FIG. 10 shows the results of analysis of Villin gene expression (left) and Villin protein expression (right) under static or flow culture conditions. The Villin gene expression level was not changed by the action of flow, whereas the Villin protein expression levels was increased by flow. The protein expression levels of CYP3A4 did not change with or without flow. n = 3, error bars: standard deviation, *: P < 0.05, **: P < 0.01.

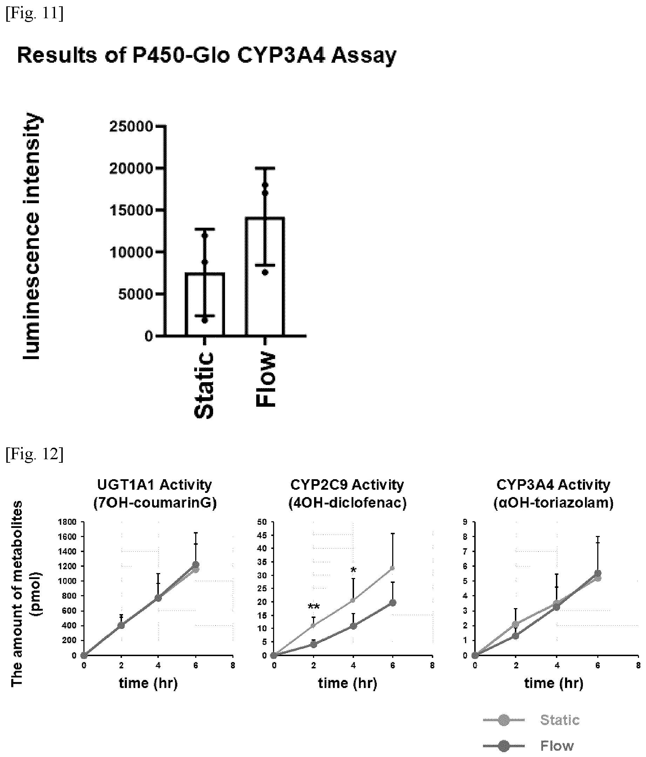

FIG. 11 shows the results of CYP3A4 activity assay in small intestine tissues-on-chips. The small intestine tissues exhibited high CYP3A4 activity under both static and flow culture conditions, with no significant difference between these conditions. This indicates that the flow did not affect the CYP3A4 activity, which is consistent with the results of gene expression analysis and immunostaining. n = 3, error bars: standard deviation.
FIG. 12 shows the results of measuring drug-metabolizing enzyme activity in small intestine tissues-on-chips. Drug-metabolizing enzyme activity was assessed using UGT1A1 activity, CYP2C9 activity, and CYP3A4 activity as indicators. The small intestine tissue exhibited high drug-metabolizing enzyme activity under both static and flow culture conditions. n = 6, error bars: standard deviation.

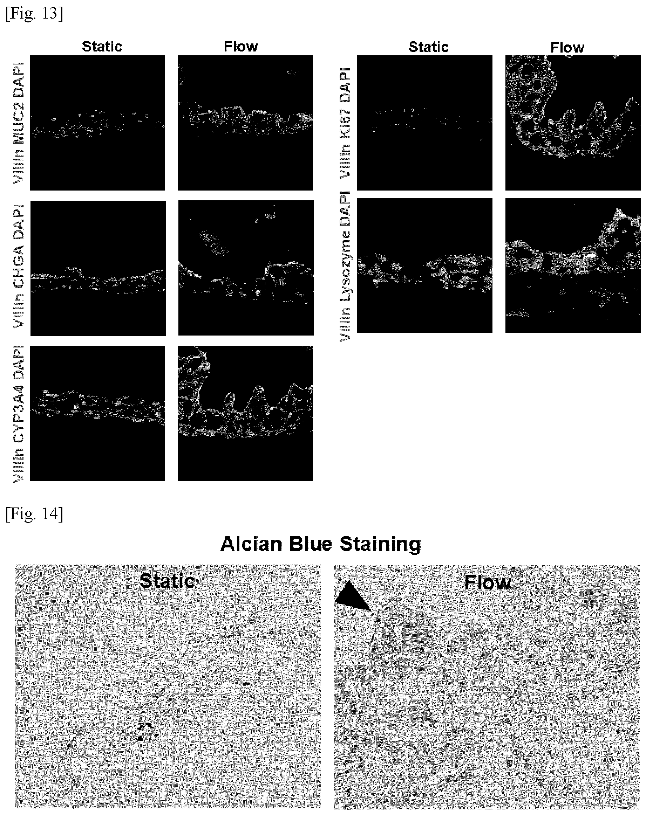

FIG. 13 relates to effects of medium flow in small intestine tissue-on-a-chip. FIG. 13 shows the results of immunostaining for Villin, MUC2, CHGA, CYP3A4, Ki67, and Lysozyme under static or flow culture conditions. Owing to the action of flow, MUC2-positive goblet cells, Lysozyme-positive Paneth cells, CHGA-positive endocrine cells, and Ki67-positive proliferating cells were observed abundantly in the small intestine tissue-on-a-chip. Villin was localized on the apical side of the small intestine tissue. For CYP3A4, the expression level was not changed by the action of flow.
FIG. 14 relates to effects of medium flow in small intestine tissue-on-a-chip. The results of alcian blue staining (to stain mucin) under static or flow culture conditions. Owing to the action of flow, alcian blue-positive mucin-producing cells (goblet cells) were observed abundantly. A thin mucin layer was observed on the apical side of small intestinal epithelial cells (indicated by a black arrowhead).

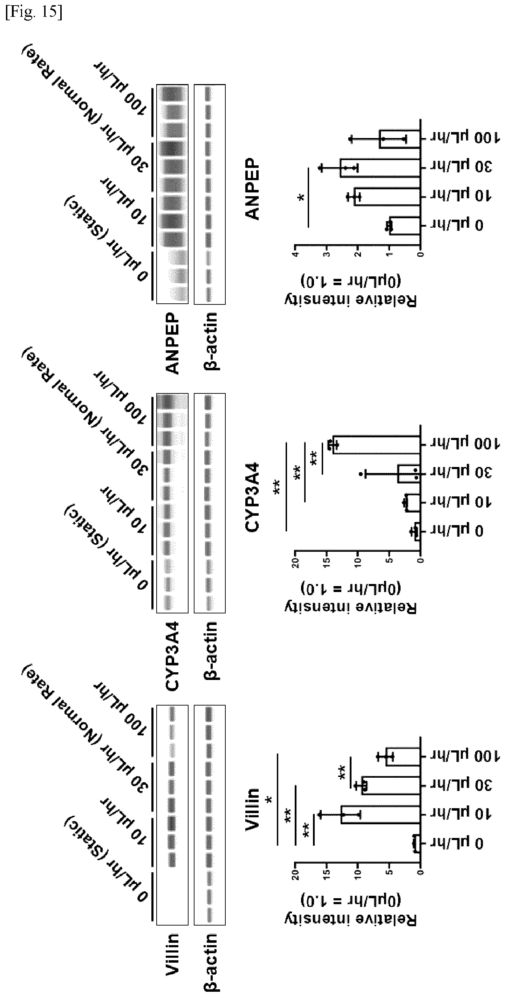

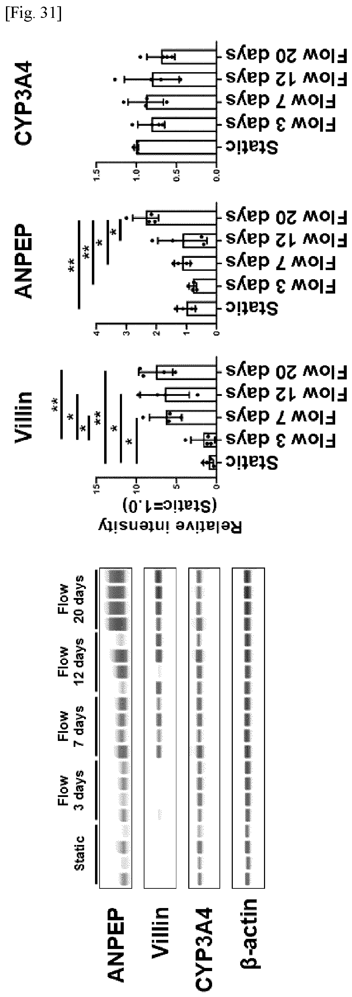

FIG. 15 relates to analysis of the effects of medium flow rate on various small intestinal markers. FIG. 31 shows the results of fully automated capillary electrophoresis immunoassays for Villin, CYP3A4, ANPEP, VIL1, and β-actin. n = 3, error bars: standard deviation.

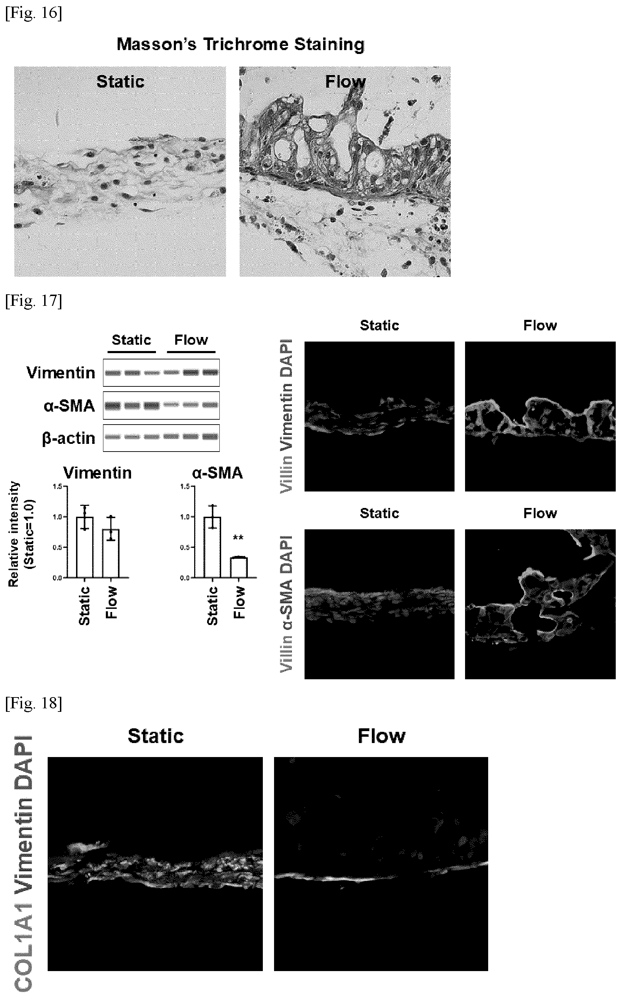

FIG. 16 relates to effects of medium flow in small intestine tissue-on-a-chip. FIG. 16 shows the results of Masson's trichrome staining under static or flow culture conditions. In Masson's trichrome staining, collagen fibers and their main component, collagen, are stained blue. The formation of a Masson's trichrome-positive collagen layer aligned on the basement membrane side was induced by the action of flow. The small intestinal epithelial layer is supported by the collagen layer. Since fibroblasts are expected to be primary collagen-producing cells, experiments to confirm the presence of fibroblasts were performed subsequently.
FIG. 17 relates to localization of fibroblasts in small intestine tissue-on-a-chip. The results of fully automated capillary electrophoresis immunoassay for Vimentin, α-SMA, and β-actin (left) and immunostaining for Villin, Vimentin, and α-SMA (right). The expression of Vimentin, an intestinal fibroblast marker, was maintained, and its localization on the basolateral side was caused by flow. Also, the expression of α-SMA, a fibroblast marker, was decreased by flow. n = 3, error bars: standard error, **: P < 0.01.
FIG. 18 relates to localization of fibroblasts in small intestine tissue-on-a-chip. FIG. 18 shows the results of immunostaining for COL1A1 and Vimentin. Colocalization of COL1A1 with Vimentin was observed.

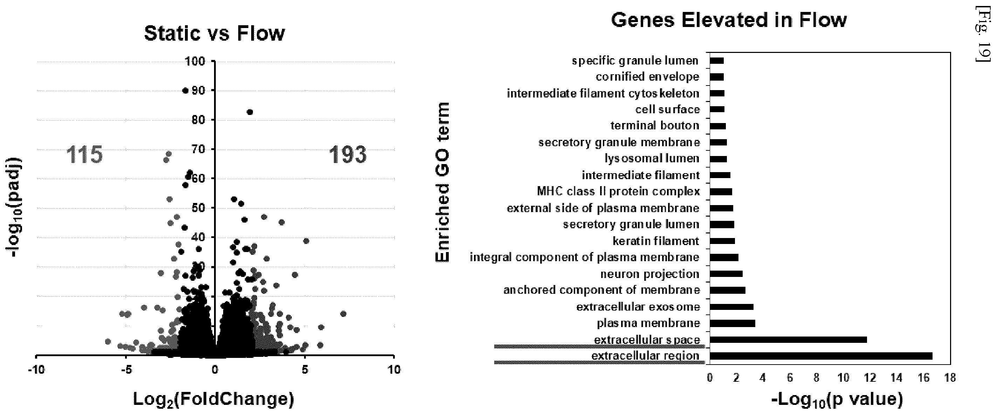

FIG. 19 relates to effects of medium flow in small intestine tissue-on-a-chip (RNA-seq analysis). Flow increased the expression of genes that regulate extracellular structures and secretions and may serve as factors controlling the mucin layer and the collagen layer adjacent to small intestinal epithelial cells.

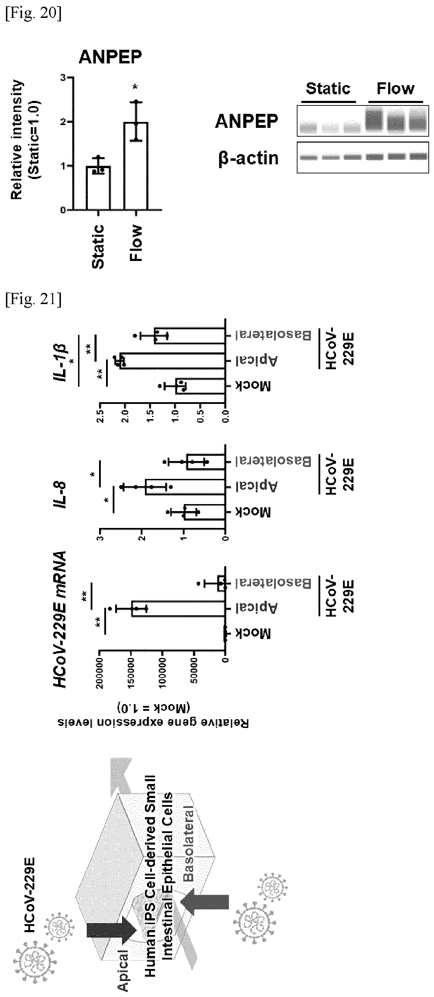

FIG. 20 relates to expression analysis of the viral receptor ANPEP in small intestine tissue-on-a-chip. FIG. 20 shows the results of fully automated capillary electrophoresis immunoassay for β-actin and ANPEP, which is one of infection receptors for human coronavirus 229E. n = 3, error bars: standard deviation, *: P < 0.05.
FIG. 21 relates to viral infection experiments using small intestine tissues-on-chips. Human coronavirus (HCoV-229E) infection experiments were performed using small intestine tissues-on-chips. The small intestine tissues-on-chips were infected with the virus from the top channel (apical) side or the bottom channel (basolateral) side, and viral infection efficiency and the viral response of host cells were evaluated. The amount of viral genome (HCoV-229E mRNA) and the mRNA amount of the genes involved in the immune response to the viral infection (IL-8 and IL-1β) were measured. The results indicate that the infection from the apical side allows efficient viral replication and host response. n = 4, error bars: standard deviation, *: P < 0.05, **: P < 0.01.

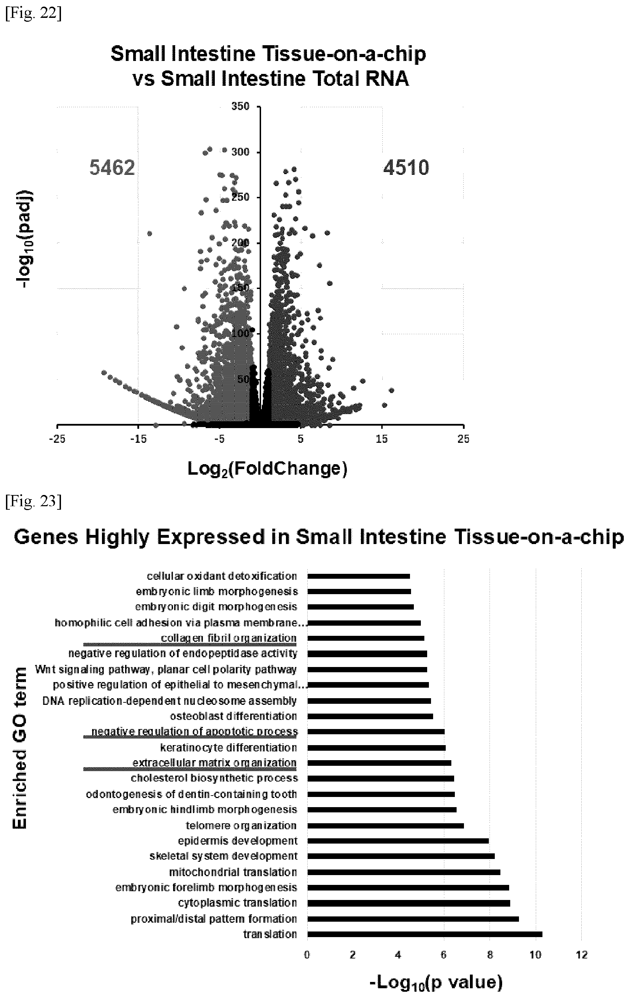

FIG. 22 relates to comparison between the newly developed small intestine tissue-on-a-chip and human native small intestine (based on small intestine total RNA) (RNA-seq analysis). Gene expression profiles were compared between small intestine tissue-on-a-chip subjected to flow culture and the small intestine total RNA. In the small intestine tissue-on-a-chip, 4,510 genes exhibited at least a two-fold increase in expression, whereas 5,462 genes exhibited a decrease in expression to one-half or less.
FIG. 23 relates to comparison between the newly developed small intestine tissue-on-a-chip and human native small intestine. Data indicate that, in the small intestine tissue-on-a-chip, a group of genes related to collagen fibrils, negative regulation of the apoptosis pathway, and ECM formation exhibited increased expression.

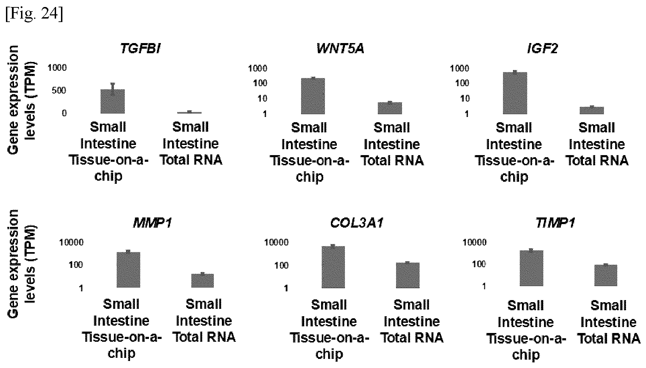

FIG. 24 relates to comparison between the newly developed small intestine tissue-on-a-chip and human native small intestine. The gene expression levels of extracellular matrix (ECM) and ECM degradation regulatory factors (MMP1, COL3A1, and TIMP1), and also the gene expression levels of growth factors (TGFB1, WNT5A, and IGF2) (WNT5A is essential for small intestinal regeneration. IGF2 is essential for maintaining intestinal stem cells.) were measured. In the small intestine tissue-on-a-chip, ECM, the ECM degradation regulatory factors, and the growth factors exhibited increased expression. n = 3, error bars: standard deviation.

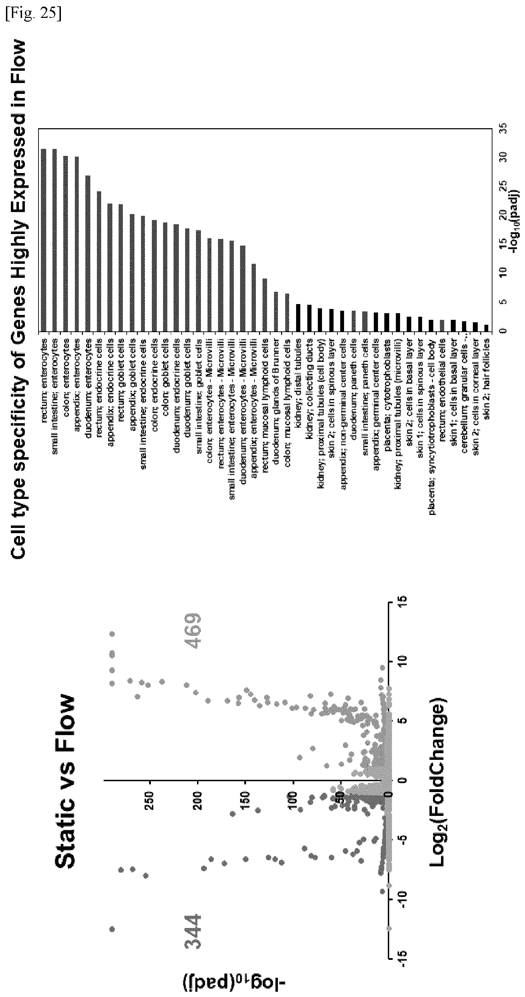

FIG. 25 relates to comparison of the protein expression changes in small intestine tissue-on-a-chip subjected to static culture (Static) or flow culture (Flow) (left) (proteome analysis), and also shows the results of cell type analysis using the Human Protein Atlas (right).

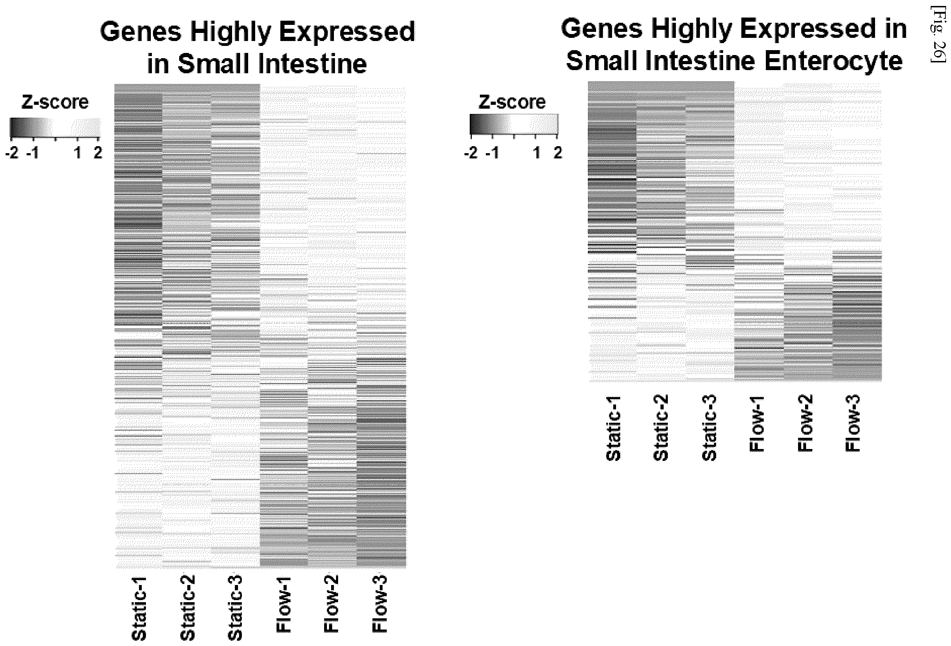

FIG. 26 shows a heatmap of a group of genes highly expressed in the small intestine (left) and a heatmap of group of genes highly expressed in enterocytes of the small intestinal (right).

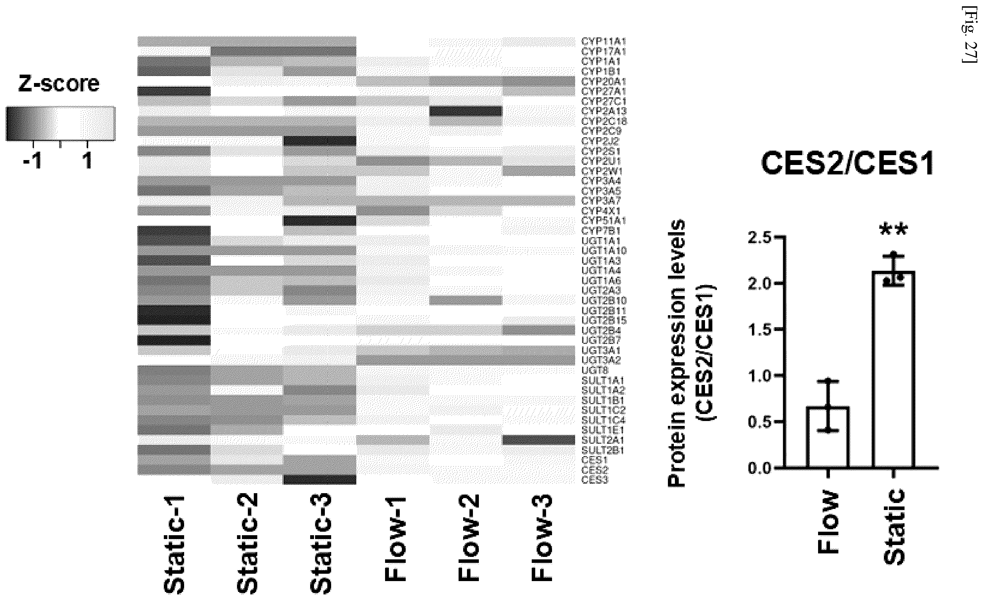

FIG. 27 shows a heatmap representing changes in protein expression of cytochrome P450s (CYPs), uridine diphosphate-glucuronosyltransferases (UGTs), sulfotransferases (SULTs), and carboxylesterases (CESs), which are drug-metabolizing enzymes (left), and also relates to comparison of the expression level between CES2 highly expressed in human small intestine and CES1 highly expressed in liver (right). n = 3, error bars: standard error, **: P < 0.01.

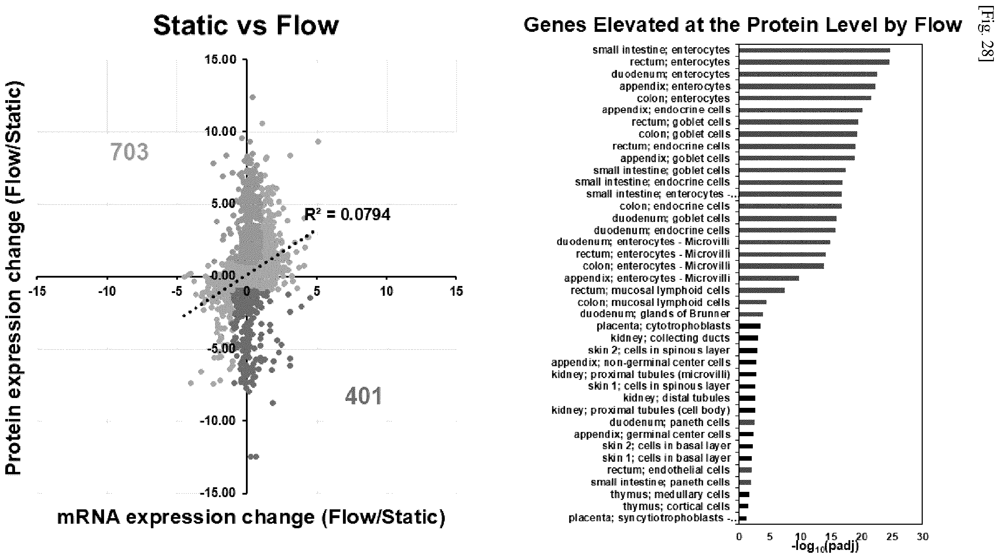

FIG. 28 relates to comparison of changes in mRNA expression and protein expression between small intestine tissue-on-a-chip subjected to static culture (Static) and small intestine tissue-on-a-chip subjected to flow culture (Flow), and the results of analysis using the Human Protein Atlas (right).

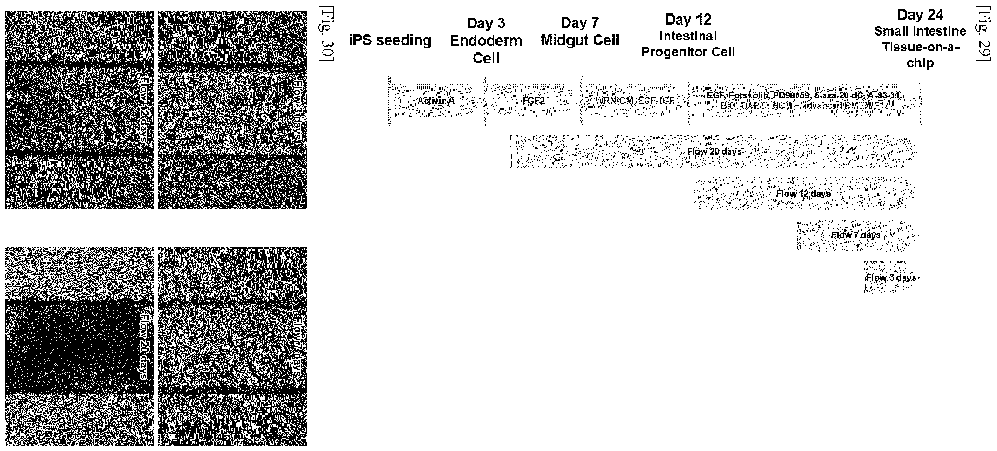

FIG. 29 shows an overview of a differentiation induction protocol from pluripotent stem cells into small intestine tissue with different periods of flow culture.
FIG. 30 shows bright-field images of small intestine tissues-on-chips.
FIG. 31 relates to analysis of the effects of the period of flow culture (Flow) on functions of small intestine tissue-on-a-chip. FIG. 31 shows the results of fully automated capillary electrophoresis immunoassays for Villin, CYP3A4, ANPEP, VIL1, and β-actin. n = 3, error bars: standard deviation, *: P < 0.05, **: P < 0.01.

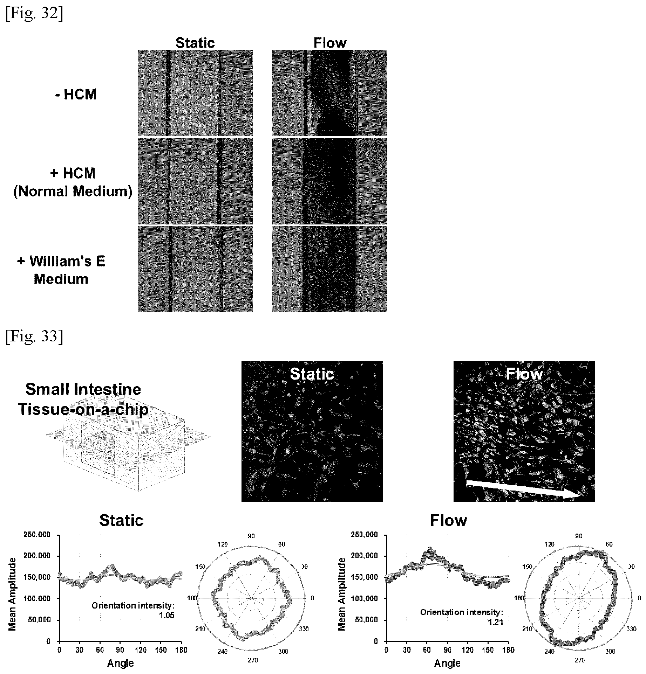

FIG. 32 shows bright-field image of small intestine tissues-on-chips. Small intestine tissue with three-dimensional morphology was constructed without using HCM. Also, small intestine tissue with three-dimensional morphology was constructed when William's E medium was used instead of HCM.
FIG. 33 shows the results of immunofluorescence staining for Vimentin under static or flow culture conditions (upper right panels), and the degree of orientation of Vimentin calculated using FiberOri8single03 (lower panels).

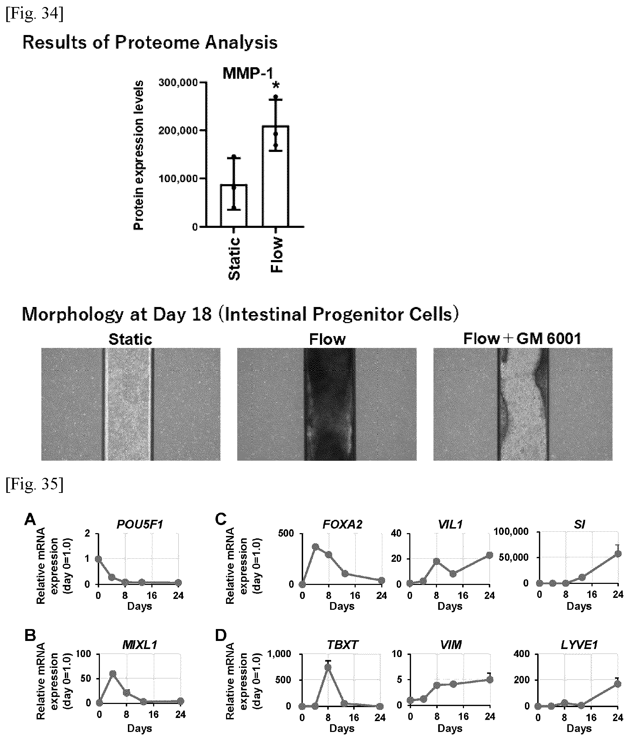

FIG. 34 shows the results of measuring MMP-1 expression levels by proteomics analysis (upper panel) and bright-field images on day 18 (intestinal progenitor cells) (lower panels). The use of GM6001, an MMP inhibitor, prevented small intestine tissue from acquiring three-dimensional morphology. n = 3, error bars: standard deviation, *: P < 0.05.
FIG. 35 relates to gene expression over time in small intestine tissue-on-a-chip (RT-qPCR analysis). FIG. 35 shows the results of measuring: the relative mRNA expression level of POU class 5 homeobox 1 (POU5F1), which is an undifferentiated cell marker (A); the relative mRNA expression level of Mix paired-like homeobox (MIXL1), which is a mesendodermcell marker (B); and the relative mRNA expression levels of forkhead box A2 (FOXA2), which is an endoderm cell marker, Villin 1 (VIL1) and sucrase-isomaltase (SI), which are enterocyte markers, T-box transcription factor T (TBXT), which is a mesoderm cell marker, and Vimentin (VIM) and lymphatic vessel endothelial hyaluronan receptor 1 (LYVE1), which are mesenchymal cell markers (C). n = 3, error bars: standard deviation.

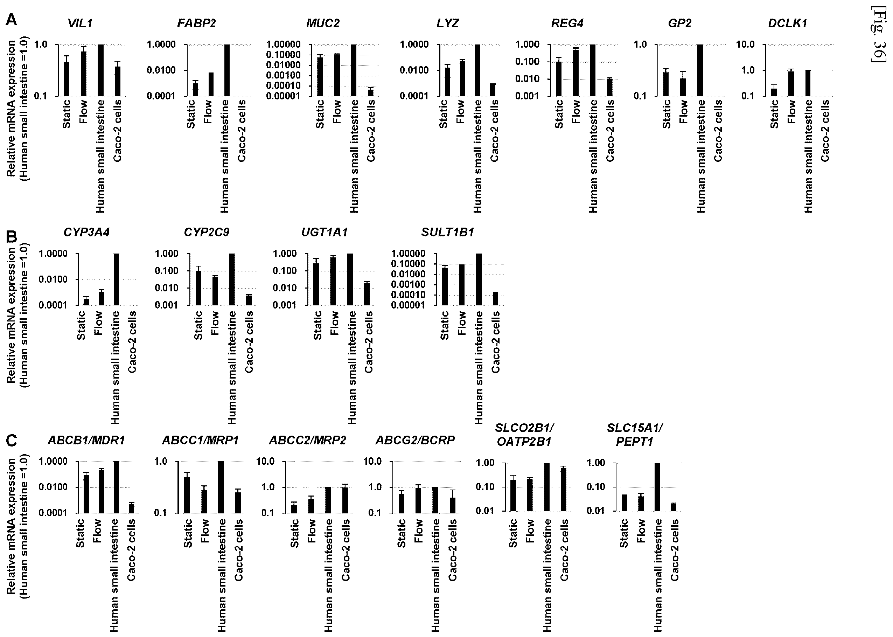

FIG. 36 relates to comparison of the gene expression in small intestine tissues-on-chips (prepared under static culture (Static) and flow culture (Flow) conditions) with the gene expression in human native small intestine and Caco-2 cells (RT-qPCR analysis). FIG. 36 shows the results of measuring the relative mRNA expression levels of: VIL1 and fatty acid-binding protein 2 (FABP2), which are enterocyte markers, mucin 2 (MUC2), which is a goblet cell marker, lysozyme (LYZ), which is a Paneth cell marker, regenerating family member 4 (REG4), which is a neuroendocrine cell marker, glycoprotein 2 (GP2), which is an M cell marker, and doublecortin-like kinase 1 (DCLK1), which is a tuft cell marker (A); cytochrome P450 family 3 subfamily A member 4 (CYP3A4), CYP family 2 subfamily C member 9 (CYP2C9), UDP glucuronosyltransferase family 1 member A1 (UGT1A1), and sulfotransferase family 1B member 1 (SULT1B1), which are drug-metabolizing enzymes (B); and ATP binding cassette subfamily B member 1 (ABCB1/MDR1), ABC subfamily C member 1 (ABCC1/MRP1), ABC subfamily C member 2 (ABCC2/MRP2), ABC subfamily G member 2 (ABCG2/BCRP), solute carrier organic anion transporter family member 2B1 (SLCO2B1/OATP2B1), and SLC family 15 member 1 (SLC15A1/PEPT1), which are transporters (C). n = 4 (small intestine tissues-on-chips and Caco-2 cells) or n = 1 (human native small intestine), error bars: standard deviation.

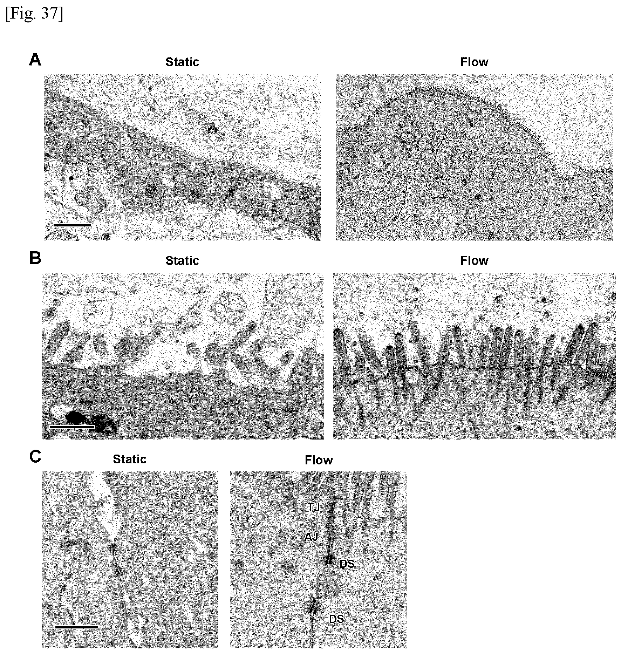

FIG. 37 relates to morphological analysis of small intestine tissues-on-chips (electron microscopic analysis). The morphology of small intestine tissue-on-a-chip prepared under static culture (Static) conditions was compared with the morphology of small intestine tissue-on-a-chip prepared under flow culture (Flow) conditions. TJ: tight junction, AJ: adherens junction, DS: desmosome. Scale bar: 6 µm (A) and 600 nm (B, C).

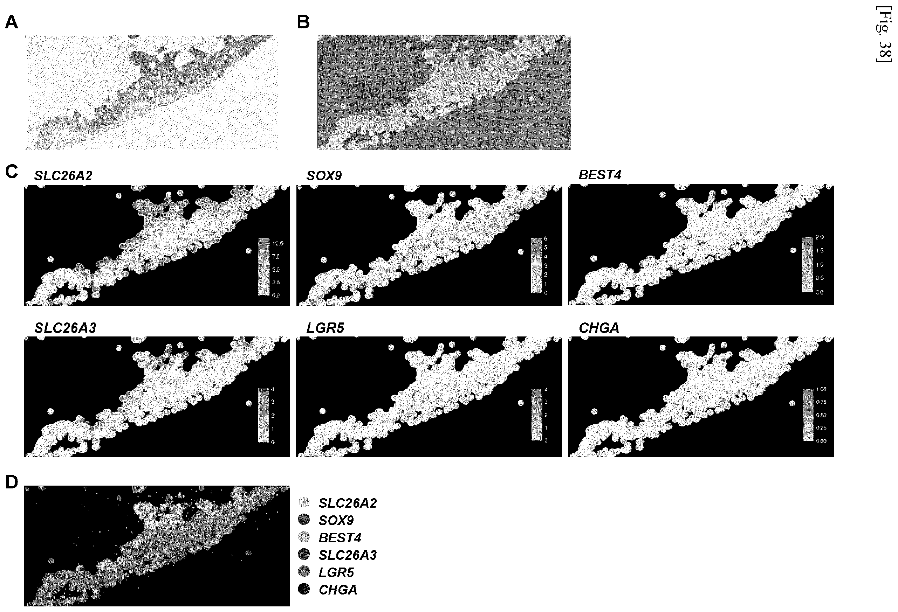

FIG. 38 relates to in situ gene expression profiling in small intestine tissue-on-a-chip. FIG. 38A shows an H&E-stained image of small intestine tissue-on-a-chip used for the analysis. FIG. 38B shows cell morphology inferred based on the positions of the cell nuclei. FIGs. 38C and 38D show the results of expression analysis of the following markers in the small intestine tissue-on-a-chip: solute carrier family 26 member 2 (SLC26A2) and solute carrier family 26 member 3 (SLC26A3), which are enterocyte markers, SRY-box transcription factor 9 (SOX9), which is an intestinal progenitor cell marker, leucine rich repeat containing G protein-coupled receptor 5 (LGR5) and Bestrophin 4 (BEST4), which are stem cell markers, and CHGA, which is a neuroendocrine cell marker.

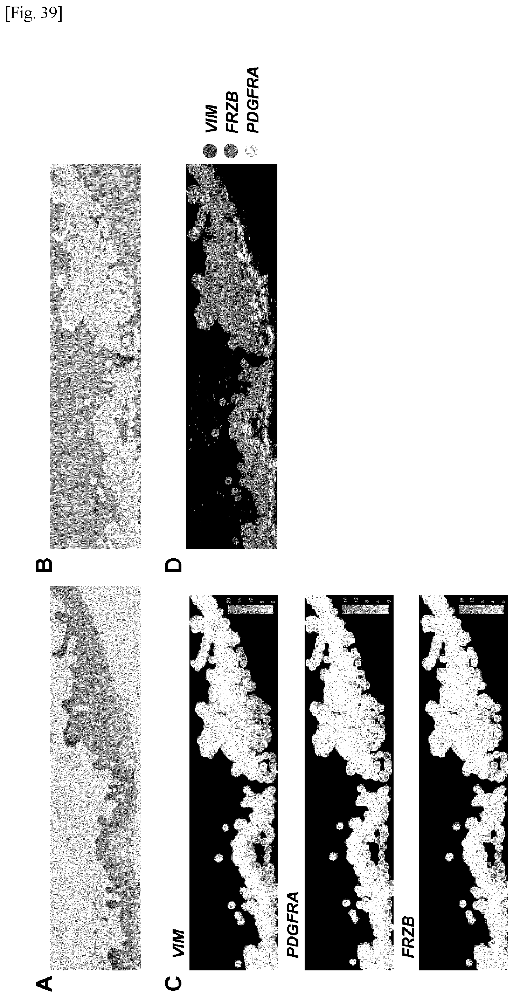

FIG. 39 relates to in situ gene expression profiling in small intestine tissues-on-chips. FIG. 39A shows an H&E-stained image of small intestine tissue-on-a-chip used for the analysis. FIG. 39B shows cell morphology inferred based on the positions of the cell nuclei. FIG. 39C and 39D show the results of transcriptome analysis for the following markers in the small intestine tissue-on-a-chip: Vimentin (VIM), which is an intestinal fibroblast marker, and platelet-derived growth factor receptor alpha (PDGFRA) and frizzled-related protein (FRZB, a Wnt antagonist), which are a telocyte marker or trophocyte marker.

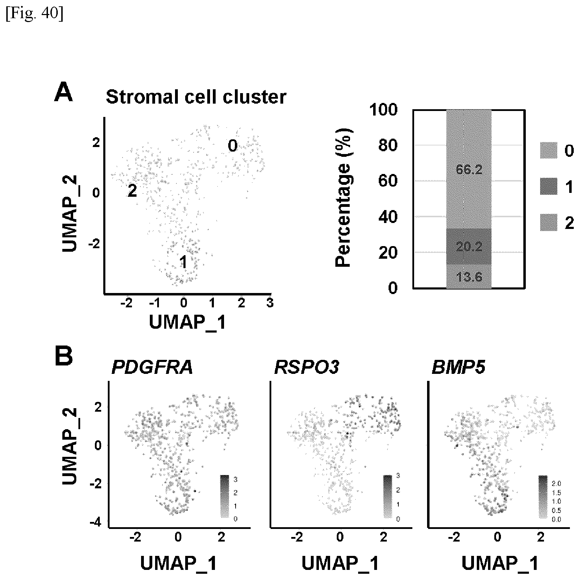

FIG. 40 relates to gene expression analysis of fibroblasts included in small intestine-tissue-on-a-chip (scRNA-seq analysis). FIGs. 40A and 40B show the results of subcluster analysis of stromal cell clusters. Stromal cells were classified into three subclusters (FIG. 40A). The numbers 0, 1, and 2 in FIG. 40 indicate subclusters 0, 1, and 2, respectively.

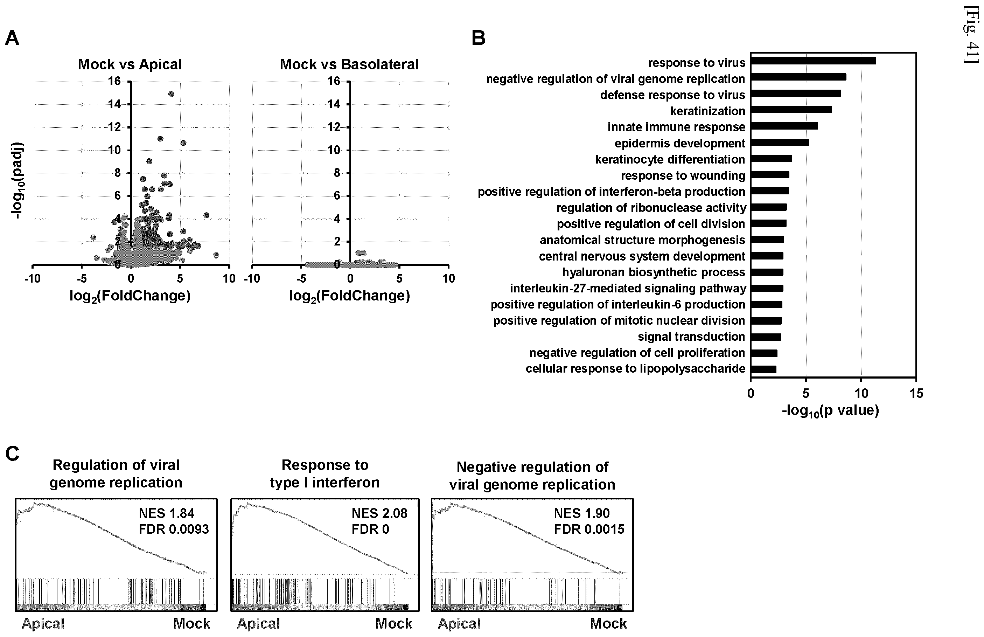

FIG. 41 shows the results of viral (HCoV-229E) infection experiment using small intestine tissues-on-chips (RNA-seq analysis). FIG. 41A shows the results obtained when the small intestine tissues-on-chips cultured under medium flow was infected with HCoV-229E from the apical side (Mock vs Apical) and from the basolateral side (Mock vs Basolateral). FIG. 41B and 41C show the change in expression of a group of host genes associated with viral infection in the small intestine tissue-on-a-chip infected with HCoV-229E from the apical side.

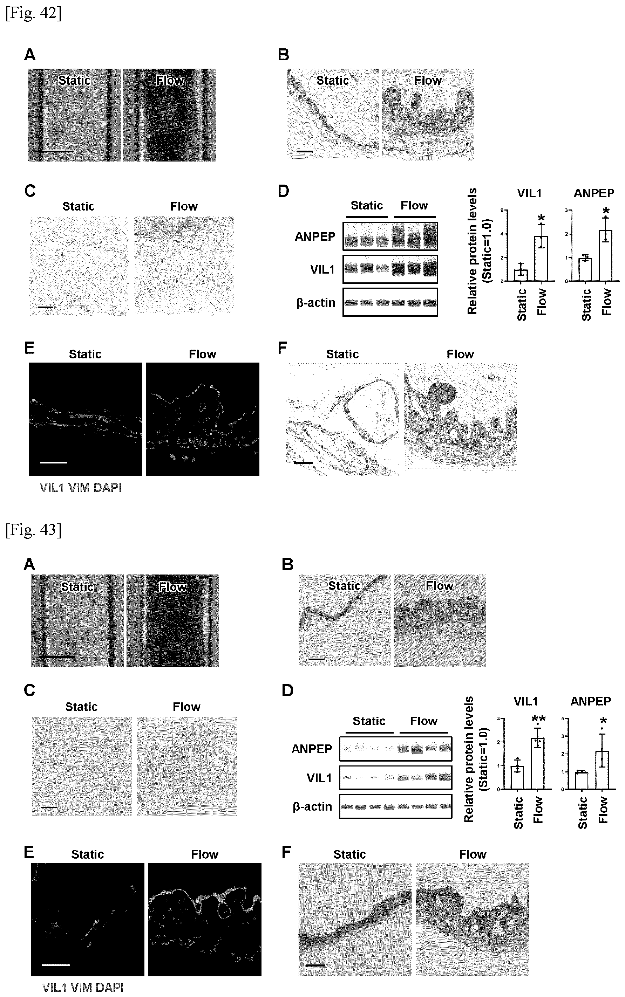

FIG. 42 relates to investigation using a human ES cell line (KhES-3) in small intestine tissues-on-chips prepared under static culture (Static) or flow culture (Flow) conditions. FIG. 42A shows bright-field images on day 24. Scale bar: 500 µm. FIG. 42B shows HE-stained images. Scale bar: 50 µm. FIG. 42C shows alcian blue-stained images. Scale bar: 50 µm. FIG. 42D shows the results of fully automated capillary electrophoresis immunoassays for ANPEP, VIL1, and β-actin. n = 3, error bars: standard deviation, *: P < 0.05. FIG. 42E shows immunostaining images for VIL1 and VIM. Nuclei were stained with DAPI. Scale bar: 50 µm. FIG. 42F shows Masson's trichrome staining images. Scale bar: 50 µm.
FIG. 43 relates to investigation using a human ES cell line (H9) in small intestine tissues-on-chips prepared under static culture (Static) or flow culture (Flow) conditions. FIG. 43A shows bright-field images on day 20. Scale bar: 500 µm. FIG. 43B shows HE-stained images. Scale bar: 50 µm. FIG. 43C shows alcian blue-stained images. Scale bar: 50 µm. FIG. 43D shows the results of fully automated capillary electrophoresis immunoassays for ANPEP, VIL1, and β-actin. n = 4, error bars: standard deviation, *: P < 0.05, **: P < 0.01. FIG. 43E shows immunostaining images for VIL1 and VIM. Nuclei were stained with DAPI. Scale bar: 50 µm. FIG. 43F shows Masson's trichrome staining images. Scale bar: 50 µm.

### DESCRIPTION OF EMBODIMENTS

### 1. Method for producing small intestinal epithelial cells and small intestine tissue

The present invention provides a method for producing small intestinal epithelial cells or small intestine tissue. Specifically, such a method includes:
(1) culturing midgut cells in a medium containing a Wnt agonist, a BMP inhibitor, and a growth factor to obtain intestinal progenitor cells; and
(2) culturing the intestinal progenitor cells obtained in the step (1) in a medium containing a growth factor, an adenylate cyclase activator, an MEK inhibitor, a DNA methylation inhibitor, a TGF-β inhibitor, a GSK3β inhibitor, and a γ-secretase inhibitor to obtain small intestinal epithelial cells or small intestine tissue (hereinafter such a method may also be referred to as "the production method of the present invention").

Midgut cells are cells constituting the midgut, which is formed during vertebrate development and serves as the precursor of the small intestine. As used herein, the term "midgut cells" refers to cells that express CDX2 and have a differentiation potency to intestinal progenitor cells. Small intestinal epithelial cells are cells constituting the small intestinal epithelium in vivo. Specific examples of the small intestinal epithelial cells include the following cells obtainable through cell culture: enterocytes that express Villin, goblet cells that express MUC2, intestinal stem cells that express LGR5, Paneth cells that express LYZ, enteroendocrine cells that express CHGA, M cells that express GP2, and tuft cells that express DCLK1. As used herein, the term "small intestine tissue" refers to a layered assembly of cells, including, as small intestinal epithelial cells, at least enterocytes. The enterocytes express Villin, and preferably further express SI and/or FABP2.

The term "cells" as used herein shall be construed to encompass "cell populations", unless otherwise stated. Unless otherwise stated, the term "cells" refers to those obtainable through cell culture. A cell population may be composed of a single type of cell or two or more different types of cells. Accordingly, the term "small intestinal epithelial cells" encompasses not only a single type of small intestinal epithelial cells (e.g., enterocytes) but also cell populations including two or more types of small intestinal epithelial cells (e.g., the combination of enterocytes, goblet cells, and intestinal stem cells).

In the step (1) of the production method of the present invention, intestinal progenitor cells can be obtained by culturing midgut cells in a medium containing a Wnt agonist, a BMP inhibitor, and a growth factor. Intestinal progenitor cells are cells that arise from intestinal stem cells during vertebrate development and are capable of differentiating into cells constituting the small intestine, i.e., cells having a differentiation potency to small intestinal epithelial cells. As used herein, the term "intestinal progenitor cells" refers to cells that are induced from midgut cells or intestinal stem cells and has a differentiation potency to small intestinal epithelial cells. The term "intestinal progenitor cells" shall be construed to encompass not only cells without self-renewal capacity but also cells with self-renewal capacity (i.e., intestinal stem cells). Typically, intestinal progenitor cells weakly express Villin, MUC2, LYZ, and CHGA.

As used herein, the term "Wnt agonist" refers to a substance that activates transcription mediated by T-cell factor (hereinafter also referred to as TCF)/lymphoid enhancer factor (hereinafter also referred to as LEF) in cells. Accordingly, Wnt agonists are not limited to Wnt family proteins but also encompass Wnt agonists that are activated by binding to members of the Frizzled receptor family, intracellular β-catenin degradation inhibitors, and TCF/LEF activating substances. Examples of the Wnt agonist used in the step (1) of the production method of the present invention include Wnt proteins and R-spondins. It is preferable to use a Wnt protein (e.g., Wnt3A) and an R-spondin (e.g., R-spondin 3) in combination.

Examples of the Wnt protein used in the step (1) of the production method of the present invention include Wnt1, Wnt2, Wnt2B, Wnt3, Wnt3A, Wnt4, Wnt5A, Wnt5B, Wnt6, Wnt7A, Wnt7B, Wnt8A, Wnt8B, Wnt9A, Wnt9B, Wnt10A, Wnt10B, Wnt11, and Wnt16. Of these, Wnt3A is preferable. Two or more types of Wnt proteins may be used in combination.

Examples of the R-spondin used in the step (1) of the production method of the present invention include R-spondin 1, R-spondin 2, R-spondin 3, and R-spondin 4. Of these, R-spondin 3 is preferable. Two or more types of R-spondins may be used in combination.

Bone morphogenetic proteins (BMP) bind, in the form of dimer ligands, to a receptor complex composed of two different types of serine/threonine kinase receptors, namely, type I and type II receptors. The Type II receptor phosphorylates the type I receptor, whereby this receptor kinase is activated. Subsequently, this type I receptor phosphorylates specific receptor substrates (SMADs), thereby inducing transcriptional activation through the signal transduction pathway. In general, a BMP inhibitor is a substance that inhibits, for example, the binding of BMP molecules to BMP receptors and binds to BMP molecules to form complexes that neutralize BMP activity. Alternatively, a BMP inhibitor is, for example, a substance that binds to BMP receptors to inhibit the binding of BMP molecules to the BMP receptors and acts as an antagonist or inverse agonist.

Examples of the BMP inhibitor used in the step (1) of the production method of the present invention include: chordin-like proteins such as noggin, chordin, and chordin domains; Follistatin-related proteins such as Follistatin and Follistatin domains; DAN; DAN-like proteins such as DAN cysteine-knot domains; sclerostin/SOST; decorin; and α2-macroglobulin. Of these, chordin-like proteins or DAN-like proteins are preferable, and chordin-like proteins (in particular, noggin) are more preferable. Chordin-like proteins and DAN-like proteins are diffusible proteins, and they are capable of inhibiting the approach of BMP molecules to signaling receptors by binding to the BMP molecules with various degrees of affinity.

The growth factor used in the step (1) of the production method of the present invention may be, for example, an epidermal growth factor (EGF), insulin-like growth factor (IGF), transforming growth factor-α (TGF-α), fibroblast growth factor (FGF), brain-derived neurotrophic factor (BDNF), or keratinocyte growth factor (KGF). Examples of FGF include 23 types of proteins, namely, FGF1 to FGF23. IGF may be IGF-1 or IGF-2. The growth factor used in the step (1) of the production method of the present invention is preferably EGF or IGF, and more preferably the combination of EGF and IGF (in particular, IGF-1). Two or more types of growth factors may be used in combination.

The concentration of EGF used in the step (1) of the production method of the present invention in the medium is typically 5 ng/mL to 500 ng/mL, preferably 10 ng/mL to 300 ng/mL, and more preferably 30 ng/mL to 100 ng/mL (in particular, 50 ng/mL). The concentration of IGF-1 used in the step (1) of the production method of the present invention in the medium is typically 1 ng/mL to 200 ng/mL, preferably 2 ng/mL to 100 ng/mL, and more preferably 10 ng/mL to 50 ng/mL (in particular, 20 ng/mL). When a growth factor other than EGF or IGF-1 is used, the concentration of the growth factor in the medium is selected as appropriate.

The culture period in the step (1) of the production method of the present invention is typically 3 to 13 days, preferably 4 to 10 days, and more preferably 5 to 7 weeks (in particular, 6 days).

In the step (2) of the production method of the present invention, the small intestinal epithelial cells or small intestine tissue can be obtained by culturing the intestinal progenitor cells obtained in the step (1) in a medium containing a growth factor, an adenylate cyclase activator, an MEK inhibitor, a DNA methylation inhibitor, a TGF-β inhibitor, a GSK3β inhibitor, and a γ-secretase inhibitor.

Examples of the growth factor used in the step (2) are the same as those given above for the step (1). The growth factor used in the step (2) of the production method of the present invention is preferably EGF. Two or more types of growth factors may be used in combination.

The concentration of EGF used in the step (2) of the production method of the present invention in the medium is typically 0.5 ng/mL to 100 ng/mL, preferably 1 ng/mL to 50 ng/mL, and more preferably 5 ng/mL to 30 ng/mL (in particular, 10 ng/mL). When a growth factor other than EGF is used, the concentration of the growth factor in the medium is selected as appropriate.

The adenylate cyclase activator used in the step (2) of the production method of the present invention is not limited as long as it increases the intracellular cAMP concentration, and examples thereof include Forskolin, HJC0350, 8-Br-cAMP, Adenosine 3',5'-cyclic monophosphate (cAMP), and Dibutyryl-cAMP (Bucladesine). Of these, Forskolin is preferable. Two or more types of adenylate cyclase activators may be used in combination.

The concentration of Forskolin used in the step (2) of the production method of the present invention in the medium is typically 1 µM to 200 µM, preferably 3 µM to 100 µM, and more preferably 5 µM to 50 µM (in particular, 15 µM). When an adenylate cyclase activator other than Forskolin is used, the concentration of the adenylate cyclase activator in the medium is selected as appropriate.

Examples of the MEK inhibitor used in the step (2) of the production method of the present invention include PD98059 (2-(2-amino-3-methoxyphenyl)-4H-1-benzopyran-4-one), PD184352 (2-(2-chloro-4-iodoanilino)-N-(cyclopropylmethoxy)-3,4-difluorobenzamide), PD184161 (2-(2-chloro-4-iodophenylamino)-N-cyclopropylmethoxy-3,4-difluoro-5-bromobenzamide), PD0325901 (N-[(2R)-2,3-dihydroxypropoxy]-3,4-difluoro-2-(2-fluoro-4-iodoanilino)benzamide), U0126, MEK inhibitor I, MEK inhibitor II, NMK1/2 inhibitor II, and SL327. Of these, PD98059 is preferable. Two or more types of MEK inhibitors may be used in combination.

The concentration of PD98059 used in the step (2) of the production method of the present invention in the medium is typically 0.5 µM to 100 µM, preferably 1 µM to 50 µM, and more preferably 5 µM to 30 µM (in particular, 10 µM). When an MEK inhibitor other than PD98059 is used, the concentration of the MEK inhibitor in the medium is selected as appropriate.

Examples of the DNA methylation inhibitor used in the step (2) of the production method of the present invention include 5-aza-2'-deoxycytidine (5-aza-2'-dC), 5-azacytidine, RG108, and zebularine. Of these, 5-aza-2'-dC is preferable. Two or more types of DNA methylation inhibitors may be used in combination.

The concentration of 5-aza-2'-dC used in the step (2) of the production method of the present invention in the medium is typically 0.1 µM to 100 µM, preferably 0.5 µM to 50 µM, and more preferably 1 µM to 5 µM (in particular, 2.5 µM). When an DNA methylation inhibitor other than 5-aza-2'-dC is used, the concentration of the DNA methylation inhibitor in the medium is selected as appropriate.

Examples of the TGF-β inhibitor used in the step (2) of the production method of the present invention include SB431542 (4-(5-benzol[1,3]dioxol-5-yl-4-pyridin-2-yl-1H-imidazol-2-yl)-benzamide, 4-[4-(1,3-benzodioxol-5-yl)-5-(2-pyridinyl)-1H-imidazol-2-yl]-benzamide, 4-[4-(3,4-methylenedioxyphenyl)-5-(2-pyridinyl)-1H-imidazol-2-yl]-benzamide), A-83-01 (3-(6-methylpyridin-2-yl)-1-phenylthiocarbamoyl-4-quinolin-4-ylpyrazole), LDN193189 (4-[6-[4-(1-piperazinyl)phenyl]pyrazolo[1,5-a]pyrimidin-3-yl]-quinoline), GW788388 (4-[4-[3-(pyridin-2-yl)-1H-pyrazol-4-yl]-pyridin-2-yl]-N-(tetrahydro-2H-pyran-4-yl)benzamide), SM16 (4-[4-(1,3-benzodioxol-5-yl)-5-(6-methyl-2-pyridinyl)-1H-imidazol-2-yl]-bicyclo[2.2.2]octan-1-carboxamide), IN-1130 (3-[[5-(6-methyl-2-pyridinyl)-4-(6-quinoxalinyl)-1H-imidazol-2-yl]methyl]-benzamide), GW6604 (2-phenyl-4-[3-(pyridin-2-yl)-1H-pyrazol-4-yl]pyridine), SB505124 (2-(5-benzo[1,3]dioxol-5-yl-2-tert-butyl-3H-imidazol-4-yl)-6-methylpyridine), SB525334 (6-[2-(1,1-dimethylethyl)-5-(6-methyl-2-pyridinyl)-1H-imidazol-4-yl]quinoxaline), SD-208 (2-(5-chloro-2-fluorophenyl)pteridin-4-yl)pyridin-4-yl-amine), and LY-364947 (4-[3-(2-pyridinyl)-1H-pyrazol-4-yl]-quinoline). Of these, A-83-01 is preferable. Two or more types of TGF-β inhibitors may be used in combination.

The concentration of A-83-01 used in the step (2) of the production method of the present invention in the medium is typically 10 nM to 10 µM, preferably 50 nM to 5 µM, and more preferably 100 nM to 500 nM (in particular, 250 nM). When a TGF-β inhibitor other than A-83-01 is used, the concentration of the TGF-β inhibitor in the medium is selected as appropriate.

As used herein, the term "GSK3β inhibitor" refers to a substance having inhibitory activity against GSK3β (glycogen synthase kinase 3β). GSK3 (glycogen synthase kinase 3), which is a type of serine/threonine protein kinase, is involved in numerous signaling pathways related to glycogen production, apoptosis, and the maintenance of stem cells. There are two isoforms of GSK3, namely, GSK3α and GSK3β. The "GSK3β inhibitor" used in the present invention is not limited as long as it has GSK3β inhibitory activity, and may be a substance that has GSK3α inhibitory activity in addition to the GSK3β inhibitory activity.

Examples of the GSK3β inhibitor used in the step (2) of the production method of the present invention include CHIR98014 (2-[[2-[(5-nitro-6-aminopyridin-2-yl)amino]ethyl]amino]-4-(2,4-dichlorophenyl)-5-(1H-imidazol-1-yl)pyridazine), CHIR99021 (6-[[2-[[4-(2,4-dichlorophenyl)-5-(4-methyl-1H-imidazol-2-yl)-2-pyrimidinyl]amino]ethyl]amino]nicotinonitrile), CP21R7 (3-(3-amino-phenyl)-4-(1-methyl-1H-indol-3-yl)-pyrrole-2,5-dione), LY2090314 (3-[9-fluoro-1,2,3,4-tetrahydro-2-(1-piperidinylcarbonyl)pyrrolo[3,2,1-jk][1,4]benzodiazepin-7-yl]-4-imidazo[1,2-a]pyridin-3-yl-1h-pyrrole-2,5-dione), TDZD-8 (4-benzyl-2-methyl-1,2,4-thiadiazolidine-3,5-dione), SB216763 (3-(2,4-dichlorophenyl)-4-(1-methyl-1H-indol-3-yl)-1H-pyrrole-2,5-dione), TWS-119 (3-[6-(3-aminophenyl)-7H-pyrrolo[2,3-d]pyrimidin-4-yloxy]phenol), kenpaullone, 1-Azakenpaullone, SB415286 (3-[(3-chloro-4-hydroxyphenyl)amino]-4-(2-nitrophenyl)-1H-pyrrole-2,5-dione), AR-AO144-18 (1-[(4-methoxyphenyl)methyl]-3-(5-nitro-1,3-thiazol-2-yl)urea), CT99021, CT20026, BIO ((2'Z,3'E)-6-bromoindolybin-3'-oxime), BIO-acetoxime, a pyridocarbazole-cyclopentadienylruthenium complex, OTDZT, alpha-4-dibromoacetophenone, and lithium. Of these, BIO is preferable. Two or more types of GSK3 inhibitors may be used in combination.

The concentration of BIO used in the step (2) of the production method of the present invention in the medium is typically 0.1 µM to 100 µM, preferably 0.5 µM to 50 µM, and more preferably 1 µM to 5 µM (in particular, 2.5 µM). When a GSK3β inhibitor other than BIO is used, the concentration of the GSK3β inhibitor in the medium is selected as appropriate.

Examples of the γ-secretase inhibitor used in the step (2) of the production method of the present invention include DAPT, DBZ, LY450139, BMS-708163, NIC5-15, GSI-953, ELND006, and CHF-5074 Of these, DAPT is preferable. Two or more types of γ-secretase inhibitors may be used in combination.

The concentration of DAPT used in the step (2) of the production method of the present invention in the medium is typically 0.2 µM to 200 µM, preferably 1 µM to 100 µM, and more preferably 2 µM to 10 µM (in particular, 5 µM). When a γ-secretase inhibitor other than DAPT is used, the concentration of the γ-secretase inhibitor in the medium is selected as appropriate.

Each substance used in the production method of the present invention may be synthesized by a known method or may be obtained commercially. When the substance is a protein, such as a Wnt protein, or a peptide, this target protein or peptide may be obtained from cells that express it. Cells that express the target protein or peptide can be obtained by inserting DNA encoding the target protein or peptide into a known expression vector and transfecting appropriate host cells with the resulting expression vector.

Also, established cells that express the target protein or peptide may be used. Examples of Wnt protein-expressing cells include L cells that stably express mouse Wnt3a (ATCC CRL-2647) and L cells that stably express mouse Wnt5a (ATCC CRL-2814). In addition, cells that express a plurality of types of proteins or peptides may also be used, and specific examples of such cells include L-WRN cells that express Wnt-3A, R-spondin 3, and noggin (ATCC CRL-3276). Therefore, in one embodiment, the Wnt agonist (e.g., a combination of a Wnt protein and an R-spondin) and the BMP inhibitor (e.g., a chordin-like protein) used in the step (1) of the production method of the present invention are obtainable from cells that express them.

When the respective substances used in the production method of the present invention are proteins or peptides, their origins are not particularly limited, and they are preferably derived from a mammal (e.g., human, mouse, rat, monkey, cow, horse, pig, or dog). The proteins or peptides used in the production method of the present invention are not limited to wild-type proteins or peptides and shall be construed to encompass their variants having similar functions. Examples of such variants include proteins or peptides having a high degree of identity (e.g., at least 80%, at least 85%, at least 90%, at least 91%, at least 92%, at least 93%, at least 94%, at least 95%, at least 96%, at least 97%, at least 98%, or at least 99%) to the amino acid sequence of a specific wild-type protein or peptide.

The culture period in the step (2) of the production method of the present invention is not particularly limited, and may be 5 days or more (e.g., 6 days, 7 days, 8 days, 9 days, 10 days, or more). Since the small intestinal epithelial cells and small intestine tissue may be maintained by the step (2), the upper limit of the culture period in the step (2) is not particularly limited and typically is 20 days or less (e.g., 19, 18, 17, or 16 days, or less). In one embodiment, the culture period in the step (2) of the production method of the present invention is 5 to 15 days or 7 to 13 days.

Examples of the basal medium used in the production method of the present invention include, but not particularly limited to: RPMI-1640 medium, Eagle's MEM (EMEM), Dulbecco's modified MEM, Glasgow's MEM (GMEM), α-MEM, 199 medium, IMDM, DMEM, Hybridoma Serum-free medium, KnockOut^{™} DMEM, Advanced^{™} media (e.g., Advanced MEM, Advanced RPMI, and Advanced DMEM/F-12), Ham's Medium F-12, Ham's Medium F-10, Ham's Medium F12K, DMEM/F-12, ATCC-CRCM30, DM-160, DM-201, BME, Fischer, McCoy's 5A, Leibovitz's L-15, RITC80-7, MCDB105, MCDB107, MCDB131, MCDB153, MCDB201, NCTC109, NCTC135, Waymouth's Medium (e.g., Waymouth's MB752/1), CMRL medium (e.g., CMRL-1066), Williams' medium E, Brinster's BMOC-3 Medium, E8 Medium, StemPro 34, and MesenPRO RS (all from Thermo Fisher Scientific); ReproFF2, Primate ES Cell Medium, and ReproStem (all from ReproCELL Incorporated); Procul AD (ROHTO Pharmaceutical Co., Ltd.); MSCBM-CD and MSCGM-CD (both from Lonza); EX-CELL 302 medium (SAFC) or EX-CELL-CD-CHO (SAFC); ReproMed^{™} iPSC Medium (ReproCELL Incorporated); and mixtures thereof. Of these, Advanced DMEM/F-12 is preferable.

In particular, for culture under feeder-free and xeno-free conditions, the following media also can be used: StemFit^{®} AK02 medium (Ajinomoto Co., Inc.), StemFit^{®} AK03 medium (Ajinomoto Co., Inc.), StemFit^{®} Basic03 medium, CTS^{®} KnockOut SR XenoFree Medium (Gibco), mTeSR1 medium, TeSRI medium (Stem Cell Technologies), Iscove's modified Dulbecco's medium (GE HealthCare), Improved MEM (Thermo Fisher Scientific), and the like.

In addition, examples of the culture medium that is used, in particular, in the step (2) of the present invention include the above-described basal media (preferably Advanced DMEM/F-12) and the above-described media mixed with a hepatocyte culture medium. Examples of the hepatocyte culture medium include HCM (LONZA), William's E Medium (Thermo Fisher Scientific), and Leibovitz's L-15 Medium (Thermo Fisher Scientific).

When necessary, the medium may contain serum such as fetal bovine serum, or alternatively, one or more serum replacements such as Knockout Serum Replacement (KSR), N2 supplement (Invitrogen), B27 supplement (Invitrogen), albumin, transferrin, apotransferrin, fatty acids, insulin, collagen precursors, trace elements, 2-mercaptoethanol, and 3'-thioglycerol. In addition, the medium may further contain one or more substances such as lipids, amino acids, L-glutamine, Glutamax (Invitrogen), non-essential amino acids, vitamins, growth factors, low molecular weight compounds, antibiotics, antioxidants, pyruvate, buffers, inorganic salts, selenic acid, progesterone, and putrescine.

A culture vessel used in the present invention may be, for example, a flask, tissue culture flask, dish, Petri dish, tissue culture dish, multidish, microplate, microwell plate, micropore, multiplate, multiwell plate (e.g., 24-well plate or 96-well microplate), chamber slide, schale, tube, tray, culture bag, or roller bottle.

As shown in the Examples below, small intestine tissue can be produced efficiently by using a microfluidic device as a culture vessel. Accordingly, it is preferable to perform the steps (1) and (2) of the present invention in a microfluidic device. As used herein, the term "microfluidic device" refers to a cell culture device with microchannels, and the width and the depth of the microchannels can be designed freely. The width and the depth of each microchannel of the microfluidic device used in the present invention are typically 10 µm to 10 mm and preferably 100 µm to 1 mm.

The microfluidic device used in the present invention is preferably a device having two vertically arranged channels (a top channel and a bottom channel) separated by a semipermeable membrane. The material of substrates of the microfluidic device having two vertically arranged channels is typically PDMS (polydimethylsiloxane), and other examples of the material include cycloolefin polymers, polymethyl methacrylate, and cycloolefin copolymers. The material of the membrane of the microfluidic device having two vertically arranged channels is typically polyethylene terephthalate (PET), and other examples of the material include plastics such as polycarbonate and polytetrafluoroethylene. The microfluidic device can be fabricated by known methods. The channels can be fabricated using a method such as soft lithography (D. C. Duffy, J. C. McDonald, O. J. A. Schueller, G. M. Whitesides, Rapid prototyping of microfluidic systems in poly(dimethylsiloxane). Analytical Chemistry 70, 4974-4984 (1998).).

In the production method of the present invention, it is preferable to culture cells under adherent culture conditions. As used herein, the term "adherent culture" refers to culture under conditions that allow the formation of strong cell-substrate adhesion between cells and a culture tool or the like.

Culture in the microfluidic device is typically performed by seeding cells into the top channel and filling the bottom channel with a medium. At this time, the medium in the bottom channel may be caused to flow. Small intestine tissue that is more closely resembling native small intestine tissue can be produced under flow. Accordingly, in one embodiment of the present invention, flow culture is performed during at least part of the culture period (preferably over the entire culture period) in the step (1) and/or the step (2) of the production method of the present invention. In a preferred embodiment, flow culture is performed over the entire culture period in the steps (1) and (2) of the production method of the present invention.

The flow culture is typically performed using a peristaltic pump or a shaker (shaking device). A peristaltic pump, also called a tube pump, is a pump that conveys fluid by squeezing a soft tube made of silicone or the like with rollers. By connecting the soft tube to the bottom channel of the microfluidic device and rotating the rollers, the medium in the bottom channel is caused to flow. As demonstrated in the Examples below, small intestine tissue could be produced at any of the flow rates of 10 µL/h, 30 µL/h, and 100 µL/h. Accordingly, the flow rate is not particularly limited, and is preferably from 10 µL/h to 100 µL/h. In addition, since it was also demonstrated that the expression levels of certain genes vary depending on the flow rate, it is also preferable to adjust the flow rate as appropriate depending on the purpose.

Flow of the medium can also be caused by placing the microfluidic device on a shaker. The shaker used in the present invention may be an orbital shaker, a reciprocating shaker, a shaker employing figure-eight motion, or a shaker having a function of switching between these shaking modes as appropriate. The shaking direction may be horizontal or vertical, and is typically horizontal. The shaking speed is not particularly limited, and typically in the range from 50 to 450 rpm and preferably in the range from 100 to 200 rpm.

A culture vessel used for adherent culture may be, for example, a culture vessel with its surface artificially treated to enhance adhesion with cells or a microfluidic device with its channels artificially treated to enhance adhesion with cells (e.g., by performing a coating treatment with, for example, an extracellular matrix such as a basement membrane preparation, fibronectin, laminin or its fragments, entactin, collagen, gelatin, Synthemax, or vitronectin or with a polymer such as polylysine or polyornithine, or surface processing such as a positive charge treatment). Examples of commercially available basement membrane preparations include Matrigel and Geltrex. In particular, a culture vessel coated with Matrigel is preferable.

Alternatively, laminin or its fragments are also preferable in terms of providing xeno-free conditions. Examples of laminin or its fragments include laminin-111 or its fragment containing the E8 domain, laminin-211 or its fragment containing the E8 domain (e.g. iMatrix-211), laminin-121 or its fragment containing the E8 domain, laminin-221 or its fragment containing the E8 domain, laminin-332 or its fragment containing the E8 domain, laminin-3A11 or its fragment containing the E8 domain, laminin-411 or its fragment containing the E8 domain (e.g. iMatrix-411), laminin-421 or its fragment containing the E8 domain, laminin-511 or its fragment containing the E8 domain (e.g. iMatrix-511, iMatrix-511 silk), laminin-521 or its fragment containing the E8 domain, laminin-213 or its fragment containing the E8 domain, laminin-423 or its fragment containing the E8 domain, laminin-523 or its fragment containing the E8 domain, laminin-212/222 or its fragment containing the E8 domain, laminin-522 or its fragment containing the E8 domain, and so on.

In at least one of the steps (1) and (2) of the production method of the present invention, the cells may be cultured under feeder-free conditions and/or xeno-free conditions. In the production method of the present invention, all the steps may be performed under feeder-free and xeno-free conditions. Also, in any steps other than the step (1) or (2) of the production method of the present invention, the cells may be cultured under feeder-free conditions and/or xeno-free conditions. As used herein, the term "feeder-free" refers to a medium or culturing conditions free of auxiliary cells (i.e., feeder cells) that are different in type from cells to be cultured and used for adjusting culturing conditions for the cells to be cultured. The term "xeno-free" refers to a medium or culturing conditions free of components derived from organisms of biological species different from that of cells to be cultured.

The culturing temperature is not particularly limited and may be about 30°C to 40°C and preferably about 37°C, with the culturing being carried out in a CO₂-containing air atmosphere with the CO₂ concentration of preferably about 2% to 5%.

The seeding density of the cells is not particularly limited as long as the cells can proliferate. The seeding density is typically 1.0 × 10² to 1.0 × 10⁷ cells/cm², preferably 1.0 × 10³ to 1.0 × 10⁶ cells/cm², and more preferably 1.0 × 10⁴ to 1.0 × 10⁵ cells/cm².

Midgut cells used in the present invention can be obtained by known methods. For example, the midgut cells may be obtained by isolating them from biological tissue (e.g., intestine tissue) using known techniques, by inducing differentiation of definitive endoderm cells into midgut cells, or commercially obtaining them from companies such as ATCC. The midgut cells can be isolated from biological tissue, for example, by flow cytometry or mass cytometry using the expression of an antigen as a marker, by magnetic cell separation, or using an affinity column on which a desired antigen is immobilized. Midgut cells used in the present invention are preferably obtained by inducing differentiation of definitive endoderm cells into midgut cells.

As used herein, the term "definitive endoderm cells" refers to cells that express SOX17 and FOXA2 and have a differentiation potency to endodermal cells, such as cells of the gastrointestinal tract. Definitive endoderm cells used in the present invention can be obtained by known methods. For example, the definitive endoderm cells can be obtained by isolating them from biological tissue (e.g., intestine tissue) using known techniques, by inducing differentiation of pluripotent stem cells into definitive endoderm cells, or commercially obtaining them from companies such as ATCC. The definitive endoderm cells can be isolated from biological tissue, for example, by flow cytometry or mass cytometry using the expression of an antigen (e.g., E-cadherin, CXCR4, or CD55) as a marker, by magnetic cell separation, or using an affinity column on which a desired antigen is immobilized. The definitive endoderm cells used in the present invention are preferably obtained by inducing differentiation of pluripotent stem cells into definitive endoderm cells.

The term "pluripotent stem cells" refers to stem cells that can differentiate into tissue or cells having various different forms or functions in the body, and that can also differentiate into cells of any lineages of the three germ layers (endoderm, mesoderm and ectoderm). Examples of pluripotent stem cells to be used for the present invention include induced pluripotent stem cells (iPS cells), embryonic stem cells (ES cells), embryonic stem cells from cloned embryos obtained by nuclear transfer (nuclear transfer Embryonic stem cells; ntES cells), multipotent germline stem cells (mGS cells) and embryonic germ cells (EG cells), of which iPS cells (and more preferably human iPS cells) are preferred. When the pluripotent stem cells are ES cells or arbitrary cells derived from human embryos, the cells may be obtained by destruction of the embryo or they may be obtained without destruction of the embryo. From an ethical perspective, they are preferably cells obtained without destruction of the embryo.

ES cells are stem cells having pluripotency and auto-replicating proliferation potency, established from the inner cell mass of an early embryo (such as the blastocyst) of a mammal such as a human or mouse. ES cells were discovered in mice in 1981 (M.J. Evans and M.H. Kaufman (1981), Nature 292:154-156), and ES cell lines were later established in primates including humans and monkeys (J.A. Thomson et al. (1998), Science 282:1145-1147; J.A. Thomson et al. (1995), Proc. Natl. Acad. Sci. USA, 92:7844-7848; J.A. Thomson et al. (1996), Biol. Reprod., 55:254-259; J.A. Thomson and V.S. Marshall (1998), Curr. Top. Dev. Biol., 38:133-165). ES cells can be established by extracting the inner cell mass from the blastocyst of a fertilized egg of a target animal and culturing the inner cell mass on a fibroblast feeder. Alternatively, ES cells can be established using a single embryo blastomere alone during the cleavage stage before the blastocyst stage (Chung Y. et al. (2008), Cell Stem Cell 2: 113-117), or they can be established using a developmentally arrested embryo (Zhang X. et al. (2006), Stem Cells 24: 2669-2676.).

ntES cells are ES cells derived from a clone embryo prepared by nuclear transfer, and they have approximately the same properties as fertilized egg-derived ES cells (Wakayama T. et al. (2001), Science, 292:740-743; S. Wakayama et al. (2005), Biol. Reprod., 72:932-936; Byrne J. et al. (2007), Nature, 450:497-502). In other words, ntES (nuclear transfer ES) cells are ES cells established from the inner cell mass of a cloned embryo-derived blastocyst obtained by exchanging an unfertilized egg nucleus with a somatic cell nucleus. Combinations of nuclear transfer (Cibelli J.B. et al. (1998), Nature Biotechnol., 16:642-646) and ES cell preparation techniques (described above) are used to prepare ntES cells (Wakayama, S. (2008), Jikken Igaku, Vol. 26, No. 5 (special edition), pp. 47-52).
For nuclear transfer, a somatic cell nucleus may be implanted into a mammalian enucleated unfertilized egg and cultured for several hours for reprogramming.

The ES cell line used for the present invention may be mouse ES cells, and for example, the different mouse ES cell lines established by the inGenious Targeting Laboratory, Riken (Riken Research Institute), etc., may be used, or for human ES cell lines, the different human ES cell lines established by the University of Wisconsin, NIH, Riken, Kyoto University, the National Center for Child Health and Development, Cellartis, etc., for example, may be used. Specific examples of human ES cell lines include: CHB-1 to CHB-12 lines, RUES1 line, RUES2 line, HUES1 to HUES28 lines, and the like distributed by ESI Bio Co.; H1 line, H9 line, and the like distributed by WiCell Research; KhES-1 line, KhES-2 line, KhES-3 line, KhES-4 line, KhES-5 line, SSES1 line, SSES2 line, SSES3 line, and the like distributed by RIKEN.

iPS cells are cells obtained by reprogramming of mammalian somatic cells or undifferentiated stem cells by transfer of specific factors (nuclear reprogramming factors). A large number of iPS cells currently exist, among which there may be used iPSCs established by introduction of the 4 factors Oct3/4 Sox2 Klf4 c-Myc into mouse fibroblasts by Yamanaka et al. (Takahashi K, Yamanaka S., Cell, (2006) 126: 663-676), iPSCs derived from human cells established by introduction of the same 4 factors into human fibroblasts (Takahashi K, Yamanaka S., et al. Cell, (2007) 131:861-872.), Nanog-iPSCs established by selecting of Nanog expression markers after introduction of the 4 factors (Okita, K., Ichisaka, T. and Yamanaka, S. (2007). Nature 448, 313-317.), iPSCs produced by methods without c-Myc (Nakagawa M, Yamanaka S., et al. Nature Biotechnology, (2008) 26, 101-106), iPSCs established by introduction of 6 factors by a virus-free method (Okita K et al. Nat. Methods 2011 May; 8(5):409-12, Okita K et al. Stem Cells. 31(3):458-66.), and so on. The induced pluripotent stem cells established by introduction of the 4 factors OCT3/4·SOX2·NANOG·LIN28, created by Thomson et al. (Yu J., Thomson JA. et al., Science (2007) 318: 1917-1920), the induced pluripotent stem cells created by Daley et al. (Park IH, Daley GQ. et al., Nature (2007) 451:141-146) and the induced pluripotent stem cells created by Sakurata et al. (Japanese Unexamined Patent Publication No. 2008-307007), etc., may also be used.

In addition, any induced pluripotent stem cells publicly known in the field as described in published journal (for example, Shi Y., Ding S., et al., Cell Stem Cell, (2008) Vol. 3, Issue 5, 568-574;, Kim JB., Scholer HR., et al., Nature, (2008) 454, 646-650; Huangfu D., Melton, DA., et al., Nature Biotechnology, (2008) 26, No 7, 795-797), or patent publications (for example, Japanese Unexamined Patent Publication No. 2008-307007, Japanese Unexamined Patent Publication No. 2008-283972, US2008-2336610, US2009-047263, WO2007-069666, WO2008-118220, WO2008-124133, WO2008-151058, WO2009-006930, WO2009-006997 and WO2009-007852), may also be used.

Induced pluripotent stem cell lines that are iPSC lines established by NIH, Riken, Kyoto University, etc., for example, may be used as well. Examples include human iPSC lines such as HiPS-RIKEN-1A line, HiPS-RIKEN-2A line, HiPS-RIKEN-12A line, Nips-B2 line, and the like by RIKEN and 253G1 line, 253G4 line, 1201C1 line, 1205D1 line, 1210B2 line, 1383D2 line, 1383D6 line, 201B7 line, 409B2 line, 454E2 line, 606A1 line, 610B1 line, 648A1 line, 1231A3 line, FfI-01s04 line, and the like by Kyoto University.

The induced pluripotent stem cells used in the production method of the present invention may be derived from cells of a patient with a hereditary disease (e.g., a patient with a hereditary small intestinal disease). Cells induced to differentiate from pluripotent stem cells derived from a patient with a hereditary small intestinal disease can serve as a disease model reflecting the pathology of the disease, and thus is suitable for screening of therapeutic or prophylactic medicaments for the disease. Alternatively, pluripotent stem cells derived from a patient with a hereditary small intestinal disease may be subjected to genome editing using a CRISPR-Cas system or the like to repair the gene of interest, and then differentiated into target cells or tissue, whereby the resulting cells or tissue can be used as a therapeutic medicament for the disease.

mGS cells are pluripotent stem cells derived from the testes, and they serve as a source for spermatogenesis. Similar to ES cells, these cells can also be induced to differentiate to cells of various cell series, and have properties that allow creation of a chimeric mouse when they are grafted into a mouse blastocyst, for example (Kanatsu-Shinohara M. et al. (2003) Biol. Reprod., 69:612-616; Shinohara K. et al. (2004), Cell, 119:1001-1012). The mGS cells are also capable of auto-replication in culture medium containing glial cell line-derived neurotrophic factor (GDNF), and their repeated subculturing under culturing conditions similar to those of ES cells allows germline stem cells to be obtained (Takebayashi, M. et al. (2008), Jikken Igaku, Vol. 26, No. 5 (Special Edition) pp. 41-46, Yodosha (Tokyo, Japan)).

EG cells are established from the primordial germ cells in the embryonic period and have pluripotency similar to that of ES cells. The EG cells can be established by culturing primordial germ cells in the presence of substances such as LIF, bFGF and stem cell factors (Matsui Y. et al. (1992), Cell, 70:841-847; J. L. Resnick et al. (1992), Nature, 359:550-551).

The source species of the pluripotent stem cells is not limited to particular species, and for example, the cells may be derived from a rodent such as a rat, mouse, hamster or guinea pig, a lagomorph such as a rabbit, an ungulate such as a pig, cow, goat or sheep, a dog, a feline such as a cat, or a primate such as a human, monkey, rhesus monkey, marmoset, orangutan or chimpanzee. The preferred source species is human.

Differentiation induction of pluripotent stem cells into midgut cells can be performed using known methods such as those described in NPLs 1 to 4, Toyoda T, et al. Stem Cell Reports 2017, and International Patent Publication No. WO2017/047797. Specifically, differentiation of pluripotent stem cells into midgut cells can be induced, for example, by a method including the steps of:
A) inducing differentiation of pluripotent stem cells into definitive endoderm cells; and
B) inducing differentiation of the definitive endoderm cells into midgut cells.

Accordingly, the production method of the present invention may include, prior to the step (1), the step A) and/or the step B).

Basal media and medium additives used in the steps A) and B) may be the same as those used in the production method of the present invention. The basal medium used in the step A) is preferably RPMI 1640 medium. The basal medium used in the step B) is preferably advanced DMEM/F12. The culturing temperature in the steps A) and B) is typically about 30°C to 40°C and preferably about 37°C, with the culturing being carried out in a CO₂-containing air atmosphere with a CO₂ concentration of preferably about 2% to 5%. Preferably, the steps A) and B) are performed under adherent culture conditions. The definition and the method of adherent culture are as described above. In a preferable embodiment, the step B) is performed in a microfluidic device.

Differentiation of the pluripotent stem cells into the definitive endoderm cells in the step A) can be induced, for example, by culturing the pluripotent stem cells in a medium containing activin A at a low dose. The medium may further contain a ROCK inhibitor and a GSK3β inhibitor. The culture period is typically 2 to 8 days and preferably 2 to 4 days (in particular, 3 days).

The concentration of activin A in the medium used in the step A) is, for example, 5 to 1000 ng/mL, preferably 20 to 500 ng/mL, and more preferably 50 to 150 ng/mL.

Examples of the GSK3β inhibitor used in the step A) are the same as those given above for the step (2) of the production method of the present invention. Of these, CHIR99021 is preferable. When CHIR99021 is used as the GSK3β inhibitor, the concentration thereof in the medium is typically 0.5 to 5 µM and preferably 1 to 4 µM.

Examples of the ROCK inhibitor used in the step A) include Y-27632 (e.g., see Ishizaki et al., Mol. Pharmacol. 57, 976-983 (2000); and Narumiya et al., Methods Enzymol. 325, 273-284 (2000)), fasudil/HA1077 (e.g., see Uenata et al., Nature 389: 990-994 (1997)), SR3677 (e.g., see Feng Y et al., J Med Chem. 51: 6642-6645 (2008)), GSK269962 (e.g., see Stavenger RA et al., J Med Chem. 50: 2-5 (2007) or WO2005/037197), GSK429286A, H1152 (e.g., see Sasaki et al., Pharmacol. Ther. 93: 225-232 (2002)), Wf-536 (e.g., see Nakajima et al., Cancer Chemother Pharmacol. 52(4): 319-324 (2003)), thiazovivin, and salts or derivatives thereof. Of these, Y-27632 is preferable. When Y-27632 is used as the ROCK inhibitor, the concentration thereof in the medium is typically 1 to 20 µM and preferably 5 to 15 µM.

The medium may be further supplemented with insulin. The concentration of insulin in the medium is typically 0.01 to 20 µM, preferably 0.1 to 10 µM, and more preferably 0.5 to 5 µM. The concentration of insulin in the medium may be, but not limited to, the concentration of insulin contained in B-27 supplement added thereto.

Differentiation into the midgut cells in the step B) can be induced, for example, by culturing the definitive endoderm cells in a medium containing a growth factor. The culture period is typically 2 to 8 days and preferably 3 to 5 days (in particular, 4 days).

In one embodiment of the present invention, flow culture is performed during at least part of the culture period (preferably over the entire culture period) in the step B). In a preferred embodiment, flow culture is performed over the entire culture period in the step B). Specific methods etc. for performing flow culture are as described above.

Examples of the growth factor used in the step B) are the same as those given above for the step (1) of the production method of the present invention. Of these, EGF, KGF, FGF2, and FGF10 are preferable, and FGF2 is more preferable. The concentration of the growth factor in the medium is set as appropriate depending on the type of the growth factor to be used, and is typically about 0.1 nM to 1000 µM and preferably about 0.1 nM to 100 µM. When the growth factor is EGF, the concentration thereof is about 5 to 2000 ng/mL (i.e., about 0.8 to 320 nM), preferably about 5 to 1000 ng/mL (i.e., about 0.8 to 160 nM), and more preferably about 10 to 1000 ng/mL (i.e., about 1.6 to 160 nM). When the growth factor is FGF10, the concentration thereof is about 5 to 2000 ng/mL (i.e., about 0.3 to 116 nM), preferably about 10 to 1000 ng/mL (i.e., about 0.6 to 58 nM), and more preferably about 10 to 1000 ng/mL (i.e., about 0.6 to 58 nM). For example, when KGF is used as the growth factor, the concentration of the growth factor is typically 5 to 150 ng/mL, preferably 30 to 100 ng/mL, and particularly preferably about 50 ng/mL.

The step A) and/or the step B) may be performed under feeder-free conditions and/or xeno-free conditions.

The production method of the present invention may also include the step of collecting target cells or tissue obtained in each step. The collected cells may be cryopreserved using a cell cryopreservation solution. Further, the cells collected in a container may be counted using a cell counter, and may be labeled with an antibody against the surface marker of the cells and sorted by flow cytometry, mass cytometry, magnetic cell separation, or the like.

### 2. Small intestinal epithelial cells and small intestine tissue

Small intestinal epithelial cells, small intestine tissue, or a microfluidic device supporting them is obtainable by the step (2) of the production method of the present invention. Accordingly, in another aspect, the present invention also provides small intestinal epithelial cells or small intestine tissue obtainable (the term "obtainable" may be read as "obtained" as appropriate) by the production method of the present invention, or a microfluidic device supporting the small intestinal epithelial cells or the small intestine tissue. The small intestine tissue obtainable by the production method of the present invention (hereinafter also referred to as "the small intestine tissue of the present invention") typically has all of the following features (A) to (F):
(A) the small intestine tissue has enterocytes;
(B) the small intestine tissue has fibroblasts;
(C) the small intestine tissue has collagen fibers;
(D) the small intestine tissue expresses one or more (preferably all) genes selected from the group consisting of TGFB1, Wnt5A, and IGF2;
(E) the small intestine tissue expresses one or more (preferably all) genes selected from the group consisting of MMP1, COL3A1, and TIMP1; and
(F) the small intestine tissue has drug-metabolizing enzyme activity.

The small intestine tissue of the present invention also has at least one (preferably all) of the following features (G) to (K):
(G) the small intestine tissue is derived from pluripotent stem cells;
(H) the small intestine tissue has goblet cells;
(I) the small intestine tissue has Paneth cells;
(J) the small intestine tissue has enteroendocrine cells; and
(K) the small intestine tissue has crypt-like structures and villus-like structures.

The small intestine tissue of the present invention further may have a feature (L): the small intestine tissue expresses a transporter gene. Examples of the transporter gene include ABCB1/MDR1, ABCC1/MRP1, ABCC2/MRP2, ABCG2/BCRP, SLCO2B1/OATP2B1, and SLC15A1/PEPT1. In one embodiment, the small intestine tissue of the present invention expresses ABCB1/MDR1, ABCC1/MRP1, ABCC2/MRP2, ABCG2/BCRP, SLCO2B1/OATP2B1, and SLC15A1/PEPT1.

In one embodiment, the small intestine tissue of the present invention is supported by the microfluidic device. In this case, in the small intestine tissue, the fibroblasts may be arranged parallel to the channels of the microfluidic device.

In addition, the small intestine tissue of the present invention may be composed of epithelial cells that are uniform and columnar, and may have regularly arranged microvilli on its surface. Further, the small intestine tissue of the present invention may have tight junctions (TJs), adherens junctions (AJs), and desmosomes (DSs), and preferably, TJs, AJs, and DSs are orderly arranged. In one embodiment, the small intestine tissue of the present invention has enterocytes that express SLC26A2 or SLC26A3 predominantly on the apical side and has intestinal progenitor cells that express SOX9 and intestinal stem cells that express LGR5 predominantly on the basolateral side.

In another aspect, the present invention also provides small intestine tissue having all of the above-described features (A) to (F). The present invention also provides the small intestine tissue further having at least one (preferably all) of the above-described features (G) to (K) and/or the above-described feature (L). Such small intestine tissue may be obtainable by the production method of the present invention, or may be obtainable by other methods. Hereinafter, the above-described small intestine tissue may also be referred to as "the small intestine tissue of the present invention".

As used herein, the terms "expressed" or "positive" with respect to a gene shall be construed to encompass, unless otherwise stated, at least "the production of mRNA encoded by the gene" and preferably further encompass "the production of a protein encoded by the mRNA". Accordingly, it can be determined that a gene is expressed when production of mRNA encoded by the gene is detected at least by the methods described in the Examples below (RT-qPCR or RNA-seq).

As used herein, the term "positive" encompasses both strong expression (also referred to as "strongly positive") and weak expression (also referred to as "weakly positive"). Strong positivity may be indicated with a superscript "high" (e.g., PDGFRA^{high}), and weak positivity may be indicated with a superscript "low" (e.g., PDGFRA^{low}). Whether the expression is strongly positive or weakly positive can be determined based on a chart obtainable by flow cytometry. Although the positions appearing in the chart may vary depending on the voltage settings and sensitivity settings of the device, the antibody clone used, the staining conditions, the dye used, and the like, those skilled in the art can appropriately set gating boundaries so as not to divide a cell population that is recognized as a single group in the obtained chart.

The fibroblasts included in the small intestine tissue of the present invention are positive for Vimentin, an intestinal fibroblast marker, and have the ability to produce collagen. The fibroblasts are typically PDGFRA-positive cells. As used herein, the term "collagen fibers" means fibers constructed by a plurality of collagen molecules assembled together. In one embodiment, the small intestine tissue of the present invention includes two or more types of fibroblasts. In a preferred embodiment, the small intestine tissue of the present invention includes fibroblasts that express R-spondin 3 (RSPO3) and fibroblasts that express bone morphogenetic protein 5 (BMP5).

The small intestine tissue of the present invention expresses one or more (preferably all) genes selected from the group consisting of TGFB1, Wnt5A, and IGF2. They are genes encoding growth factors. Typically, the small intestine tissue of the present invention exhibits higher expression levels of one or more (preferably all) of these genes as compared with those in normal adult human small intestine.

The small intestine tissue of the present invention expresses one or more (preferably all) genes selected from the group consisting of MMP1, COL3A1, and TIMP1. They are genes encoding extracellular matrix (ECM) and ECM degradation regulatory factors. Typically, the small intestine tissue of the present invention exhibits higher expression levels of one or more (preferably all) of these genes as compared with those in normal adult human small intestine.

In the present specification, the evaluation of the expression level of a specific gene can be performed by the method described in the Examples below, more specifically, by RNA-seq analysis using, as samples, total RNA isolated from the small intestine tissue of the present invention and total RNA of normal adult human small intestine (BioChain Institute, R1234226-50). Based on such RNA-seq analysis, when the TPM (transcripts per million) value of a given gene in the small intestinal tissue of the present invention is higher than that in the normal adult human small intestine, the gene is regarded as being more highly expressed in the small intestinal tissue of the present invention than in the normal adult human small intestine.

Examples of the drug-metabolizing enzyme activity described in the feature (F) include CYP3A4 activity, UGT1A1 activity, and CYP2C9 activity. Preferably, the small intestine tissue of the present invention has at least CYP3A4 activity. Having drug-metabolizing enzyme activity means that the drug-metabolizing enzyme activity is exhibited at least when measured by the method described in the Examples below.

Crypts are tubular invaginations located at the base of villi in the small intestine, and the term "crypt-like structure" as used herein refers to a structure similar to a crypt of the small intestine in appearance. Villi are protrusions present on the circular folds of the small intestinal wall, and the term "villus-like structure" as used herein refers to a structure similar to a villus of the small intestine in appearance.

### 3. Uses of small intestinal epithelial cells and small intestine tissue

The small intestinal epithelial cells and small intestine tissue of the present invention can be used in medical applications such as regenerative medicine. Accordingly, in another aspect, the present invention provides an agent for transplantation therapy including the small intestinal epithelial cells or small intestine tissue of the present invention (hereinafter may also be referred to as "the agent for transplantation therapy of the present invention"). The present invention also encompasses a method for treating or preventing a small intestinal disease, including administering or transplanting an effective amount of the small intestinal epithelial cells or small intestine tissue of the present invention into a mammal (e.g., human, mouse, rat, monkey, cow, horse, pig, or dog) as a subject for the treatment or prevention. Unless otherwise stated, a therapeutic or prophylactic medicament (or therapeutic or prophylactic method) for a disease encompasses a medicament (or method) capable of treating and preventing the disease.

Examples of the small intestinal disease include Crohn's disease, gastrointestinal polyposis, small intestinal tumors, protein-losing enteropathy, malabsorption syndrome, diverticular disease, and Meckel's diverticulum. The small intestinal epithelial cells and small intestine tissue of the present invention exhibit a therapeutic or prophylactic effect against a small intestinal disease by being transplanted into the affected area or a site resected during treatment of the disease.

The agent for transplantation therapy of the present invention can be administered or transplanted in vivo to a subject in need thereof. The small intestinal epithelial cells or small intestine tissue to be transplanted may be administered in a therapeutically or prophylactically effective amount. The amount to be administered is not particularly limited and may vary depending on various factors of the recipient, such as the age, the weight, the size of the transplant site, and the severity of the disease. For example, the number of cells to be administered may be approximately 10 × 10⁴ cells to 10 × 10¹¹ cells.

When the small intestinal epithelial cells or small intestine tissue of the present invention is used as an agent for transplantation therapy, it is desirable, from the viewpoint of preventing rejection, to use small intestinal epithelial cells or small intestine tissue derived from iPS cells established from somatic cells that have the same or substantially the same HLA genotype as the individual receiving the transplant. The term "substantially the same" in this context means that the HLA genotype matches to a degree sufficient to allow suppression of the immune response to the transplanted cells by an immunosuppressive agent, and examples of such somatic cells include those having an HLA type in which three loci, namely, HLA-A, HLA-B, and HLA-DR, or four loci including HLA-C in addition to these three loci, match those of the recipient. If it is difficult to obtain satisfactory cells owing to the age, the body constitution, or the like, the small intestinal epithelial cells or small intestine tissue may be transplanted in the state of being encapsulated in a capsule made of a material such as polyethylene glycol or silicone, a porous container, or the like to avoid the rejection.

The small intestinal epithelial cells or small intestine tissue of the present invention is produced in the form of a parenteral formulation such as an injection, suspension, or infusion by being mixed with a pharmaceutically acceptable carrier according to routine procedures. Therefore, in still another aspect, the present invention also provides a method for producing an agent for transplantation therapy, including formulating the small intestinal epithelial cells or small intestine tissue of the present invention. Such a production method may include the step of preparing the small intestinal epithelial cells or small intestine tissue of the present invention. Also, such a production method may further include the step of storing the small intestinal epithelial cells or small intestine tissue of the present invention.

Examples of a pharmaceutically acceptable carrier that may be contained in the parenteral formulation include aqueous solutions for injection, such as physiological saline and isotonic solutions containing dextrose and other auxiliary agents (e.g., D-sorbitol, D-mannitol, and sodium chloride). The small intestinal epithelial cells and small intestine tissue of the present invention may be blended with, for example, a buffer (e.g., phosphate buffer or sodium acetate buffer), a soothing agent (e.g., benzalkonium chloride or procaine hydrochloride), a stabilizer (e.g., human serum albumin or polyethylene glycol), a preservative, and/or an antioxidant.

The agent for transplantation therapy of the present invention may be supplied in a state of being cryopreserved under conditions normally used for cryopreservation of cells, and may be thawed at the time of using the agent. In this case, the agent may further contain serum or serum replacement, an organic solvent (e.g., DMSO), and the like. In this case, the concentration of serum or serum replacement is not particularly limited, and may be about 1% to about 30% (v/v) and preferably about 5% to about 20% (v/v). The concentration of the organic solvent is not particularly limited, and may be 0% to about 50% (v/v) and preferably about 5% to about 20% (v/v).

Furthermore, the small intestinal epithelial cells or small intestine tissue of the present invention can also be used in a method for screening candidate pharmaceutical agents useful for treating or preventing a small intestinal disease. Accordingly, in still another aspect, the present invention also provides a method for screening for a therapeutic or prophylactic medicament for a disease, including culturing the small intestinal epithelial cells or small intestine tissue of the present invention in the presence or absence of a test substance. Examples of such a small intestinal disease are the same as those given above as examples of the small intestinal disease to be treated or prevented by the agent for transplantation therapy of the present invention.

The small intestine tissue of the present invention typically expresses ANPEP (Alanyl aminopeptidase, membrane). ANPEP is known as an infection receptor for human coronavirus 229E (HCoV-229E). Also, the small intestine tissue of the present invention may express ACE2. ANPEP is known as an important infection receptor for several viruses belonging to the family *Coronaviridae,* such as SARS-CoV and SARS-CoV-2. Accordingly, the small intestinal epithelial cells and small intestine tissue of the present invention can serve as a useful tool both for studying the mechanism of infection of viruses that infect cells via ANPEP and/or ACE2, in particular, viruses belonging to the family *Coronaviridae,* and for pharmaceutical development.

Accordingly, in still another aspect, the present invention provides a method for screening a therapeutic or prophylactic medicament for a viral infection, including the following steps (I) to (III):
(1) contacting the small intestine tissue of the present invention with a virus in the presence or absence of a test substance and culturing the small intestine tissue;
(II) evaluating the degree of infection with the virus in the small intestine tissue; and
(III) when it is evaluated in the step (II) that the degree of infection with the virus is lower in the presence of the test substance than in the absence of the test substance, selecting the test substance as a candidate substance for a therapeutic or prophylactic medicament for the viral infection (hereinafter such a method is also referred to as "the screening method of the present invention").

Viruses to which the screening method of the present invention is applicable are not limited as long as they are capable of infecting the small intestine tissue of the present invention via a cell surface receptor such as ANPEP or ACE2. Examples of the viruses include those belonging to the family *Coronaviridae* (hereinafter also referred to as "coronaviruses"). Of these, viruses belonging to the Sarbecovirus subgenus, which infect cells via ANPEP or ACE2, are preferable, and in particular, viruses belonging to the family *Coronaviridae* (hereinafter also referred to as "coronaviruses") (e.g., HCoV-229E), novel coronavirus (SARS-CoV-2), and severe acute respiratory syndrome coronaviruses (SARS-CoV) are preferable.

When cells become infected with a virus, the immune response to the infection (e.g., increased expression of cytokines such as IL-1β, IL-6, IL-8, IFN-α, IFN-β, and IFN-γ, increased secretion of these cytokines, etc.) occur. Accordingly, by determining the degree of such immune response, the degree of viral infection of the small intestine tissue can be evaluated. Accordingly, one embodiment of the step (II) of the screening method of the present invention is the step of measuring the expression level and/or the secretion level of a cytokine in the small intestine tissue and/or in culture supernatant.

When cells become infected with a virus such as a coronavirus and the virus begins to replicate within the cell, characteristic changes appear in the appearance of the small intestine tissue (i.e., cell degeneration occurs). These phenomena are called "cytopathic effects (CPEs)" and serve as an indicator indicative of viral replication within the cells. Examples of CPEs include the formation of syncytia resulting from fusion of a large number of cells and detachment from the bottom of the culture vessel. The degree of viral infection can also be evaluated by analyzing these phenomena using a method such as microscopic observation or immunostaining.

In addition, any substance that can inhibit binding of, for example, a viral spike protein or envelope to cellular ANPEP and/or ACE2 can be as a therapeutic or prophylactic medicament for viral infectious diseases. In the presence of such a substance, a greater amount of virus is detected in culture supernatant as compared with that in the absence of the substance. Accordingly, the amount of virus in the culture supernatant serves as an indicator of the degree of viral infection. Accordingly, another embodiment of the step (II) of the screening method of the present invention is the step of measuring the amount of virus in culture supernatant.

Alternatively, the step (II) of the screening method of the present invention may be carried out by isolating the virus from the small intestine tissue of the present invention having been contacted with the virus, and comparing the amounts of the virus. In this case, when the amount of the virus in the small intestine tissue is lower in the presence of the test substance than in the absence of the test substance, the test substance is selected as a candidate substance for the therapeutic or prophylactic medicament for the viral infection.

The amount of the virus can be measured, for example, by performing quantitative PCR, such as competitive PCR or real-time PCR, using genomic RNA of the virus. Alternatively, the amount of the virus can also be measured by an immunological assay (e.g., CLEIA, ELISA, FIA, RIA, or Western blot) using an antibody that specifically recognizes a virus-derived peptide (e.g., a spike protein). Also, commercially available viral quantification kits may be used. The expression levels and the secretion levels of cytokines can also be measured in the same manner.

Furthermore, the small intestine tissue of the present invention can be used for evaluating the pharmacokinetics and toxicity of drugs. Specifically, the small intestine tissue of the present invention can be used, for example, to evaluate various properties of drugs, including the absorption or membrane permeability of test substances, drug interaction, induction of drug-metabolizing enzymes, induction of drug transporters, and toxicity.

The small intestine tissue of the present invention typically highly expresses drug-metabolizing enzymes. Accordingly, the degree of drug metabolism can be evaluated using the activities of the drug-metabolizing enzymes as indicators. In addition, the toxicity of a drug can also be evaluated by analyzing (e.g., identifying) metabolites of the drug generated as a result of an enzymatic reaction catalyzed by a drug-metabolizing enzyme, in which the drug is used as a substrate. Accordingly, in still another aspect, the present invention provides a method for evaluating the degree of metabolism of a test substance, including the following steps (i) to (iii):
(i) contacting a test substance with the small intestine tissue of the present invention;
(ii) measuring the activity level of a drug-metabolizing enzyme; and
(iii) based on the activity level of the drug-metabolizing enzyme measured in the step (ii), evaluating the degree of metabolism of the test substance catalyzed by the drug-metabolizing enzyme.

The step (i) can typically be performed by adding the test substance to the medium in which the small intestine tissue of the present invention is cultured, or by transferring the small intestine tissue of the present invention to a medium to which the test substance has been added in advance.

Examples of the drug-metabolizing enzyme used in the present invention include cytochrome P450s (e.g., CYP3A4 and CYP2C9), uridine diphosphate-glucuronosyltransferases (e.g., UGT1A1, UGT1A8, and UGT1A10), and sulfotransferases (e.g., SULT1A3). Of these, CYP3A4, UGT1A1, and CYP2C9 are preferable, and CYP3A4 is particularly preferable. Two or more types of drug-metabolizing enzymes may be used in combination.

Typically, the step (ii) is the step of measuring the expression level of the drug-metabolizing enzyme. The expression level of the drug-metabolizing enzyme can be evaluated based on the mRNA level or the protein level. For example, when the mRNA level of the drug-metabolizing enzyme is increased as compared with that before contact with the test substance, or as compared with that observed after contact with a negative control, it can be determined that the test substance has been metabolized at least partially. It is also possible to evaluate the degree of metabolism of the drug (e.g., the proportion of the drug metabolized, the metabolic rate, etc.) based on the expression level of the drug-metabolizing enzyme.

Also, the degree of metabolism of the drug can be evaluated by, in the step (i), further contacting the small intestine tissue of the present invention with a substance known to be metabolized by the drug-metabolizing enzyme (e.g., triazolam (metabolite: α-hydroxy-triazolam), diclofenac (metabolite: 4-hydroxy-diclofenac), or 7-hydroxycoumarin (metabolite: 7-hydroxycoumarin glucuronide)) and measuring the amount of metabolites of the substance. Alternatively, the measurement may be performed using a commercially available kit for measuring drug-metabolizing enzyme activity (e.g., P450-Glo^{™} CYP3A4 Assay).

The absorption of the test substance can be evaluated, for example, by measuring the residual amount of the test substance in the medium and evaluating the degree of absorption of the test substance based on the thus-measured residual amount.

The methods for measuring the expression level of drug-metabolizing enzymes, the amount of metabolites, and the residual amount of test substances are not particularly limited, and examples thereof include mass spectrometry, liquid chromatography, and immunological measurement.

The periods of the steps (I) and (i) are not particularly limited, and typically 1 hour to 10 days.

Examples of the test substance used in the present invention include cell extracts, cell culture supernatants, microbial fermentation products, extracts derived from marine organisms, plant extracts, purified or crude proteins, peptides, non-peptide compounds, synthetic low molecular weight compounds, and natural compounds.

The test substance can also be obtained using any one of a number of approaches in combinatorial library techniques known in the art, including (1) biological libraries, (2) synthetic library methods using deconvolution, (3) "one-bead one-compound" library methods, and (4) synthetic library methods using selection by affinity chromatography. Although biological library methods using selection by affinity chromatography are applicable only to peptide libraries, the other four approaches are applicable to libraries of peptides, non-peptide oligomers, or low molecular weight compounds (Lam (1997) Anticancer Drug Des. 12: 145-67). Examples of the method for synthesizing molecule libraries can be found in the art (DeWitt et al. (1993) Proc. Natl. Acad. Sci. USA 90: 6909-13; Erb et al. (1994) Proc. Natl. Acad. Sci. USA 91: 11422-6; Zuckermann et al. (1994) J. Med. Chem. 37: 2678-85; Cho et al. (1993) Science 261: 1303-5; Carell et al. (1994) Angew. Chem. Int. Ed. Engl. 33: 2059; Carell et al. (1994) Angew. Chem. Int. Ed. Engl. 33: 2061; Gallop et al. (1994) J. Med. Chem. 37: 1233-51). Compound libraries can be prepared in the form of solutions (see Houghten (1992) Bio/Techniques 13: 412-21), or in the form of beads (Lam (1991) Nature 354: 82-4), chips (Fodor (1993) Nature 364: 555-6), bacteria (U.S. Pat. No. 5,223,409), spores (U.S. Pat. Nos. 5,571,698, 5,403,484, and 5,223,409), plasmids (Cull et al. (1992) Proc. Natl. Acad. Sci. USA 89: 1865-9) or phages (Scott and Smith (1990) Science 249: 386-90; Devlin (1990) Science 249: 404-6; Cwirla et al. (1990) Proc. Natl. Acad. Sci. USA 87: 6378-82; Felici (1991) J. Mol. Biol. 222: 301-10; US-B-2002103360).

The present invention will be described more specifically with reference to Examples below. It is to be noted, however, that the present invention is not limited thereto by any means.

### EXAMPLES

### Materials and methods

### <Fabrication of microfluidic devices>

The microfluidic device consists of two layers of microchannels separated by a semipermeable membrane (Fig 2). The microchannel layers were fabricated from polydimethylsiloxane (PDMS) using a soft lithographic method (D. C. Duffy, J. C. McDonald, O. J. A. Schueller, G. M. Whitesides, Rapid prototyping of microfluidic systems in poly(dimethylsiloxane). Analytical Chemistry 70, 4974-4984 (1998).). PDMS prepolymer (Sylgard 184; Dow Corning) at a ratio of 10:1 base to curing agent was cast against a mold composed of SU-8 2150 (MicroChem) patterns formed on a silicon wafer. The size of the microchannels was 1 mm in width and approximately 300 µm in height. To introduce medium for cell culture into the microchannels, access holes were punched through the PDMS using a 6-mm biopsy punch (Kai Corporation). The upper and lower PDMS layers were bonded to a semipermeable polyethylene terephthalate (PET) membrane containing 3.0 µm pores (Cat. No. 353091; Corning) using a thin layer of liquid PDMS prepolymer as an adhesive (Chueh BH, Huh D, Kyrtsos CR, Houssin T, Futai N, Takayama S. Leakage-free bonding of porous membranes into layered microfluidic array systems. Anal Chem. 2007 May 1;79(9):3504-8. doi: 10.1021/ac062118p. Epub 2007 Mar 28. PMID: 17388566; PMCID: PMC2517097.). PDMS prepolymer was spin-coated (4,000 rpm for 60 sec) onto a glass slide. Subsequently, both the top and bottom channel layers were placed on the glass slide to transfer the thin layer of PDMS prepolymer onto the embossed PDMS surfaces. The PET membrane was placed on the top layer and bonded to the bottom layer. The combined layers were left at room temperature for 1 day to remove air bubbles and then put into an oven at 60°C overnight to cure the PDMS prepolymer. PDMS-based microfluidic devices were sterilized by placing them under ultraviolet (UV) light for 1 hr before cell culture.

<Maintenance of human pluripotent stem cells>

The human induced pluripotent stem (iPS) cell line, 1383D6 (provided by Dr. Masato Nakagawa, Kyoto University), and embryonic stem (ES) cell lines, KhES-3 (Kyoto University) and H9 (WA09, WiCell Research Institute), were maintained on 0.5 µg/cm² recombinant human laminin 511 E8 fragments (i-Matrix-511; Cat. No. 892 021; Nippi) with StemFit AK02N medium (Cat. No. RCAK02N; Ajinomoto). Cell passage was performed every 6 days. For cell passage, iPS cell colonies were treated with TrypLE Select Enzyme (Cat. No. 12563011; Thermo Fisher Scientific) for 10 min at 37°C and seeded with StemFit AK02N medium containing 10 µM Y-27632 (Cat. No. 034-24024; FUJIFILM Wako Pure Chemical). Human ES cells were used following the Guidelines for Derivation and Utilization of Human Embryonic Stem Cells of the Ministry of Education, Culture, Sports, Science and Technology of Japan, and the study was approved by an independent ethics committee.

### <Generation of small intestine tissue-on-a-chip>

Human iPS cells were seeded on Matrigel Growth Factor Reduced Basement Membrane (Cat. No. 354230; Corning)-coated cell culture plates. To perform definitive endoderm cell differentiation, human iPS cells were treated with 100 ng/mL Activin A (Cat. No. 338-AC-01M; R&D Systems) in RPMI1640 medium (Cat. No. R8758-500; Sigma-Aldrich) supplemented with 1× B-27 Supplement Minus Vitamin A (Cat. No. 12587001; Thermo Fisher Scientific), 1× GlutaMAX (Cat. No. 35050-079; Thermo Fisher Scientific), and 1× penicillin-streptomycin for 3 days. Next, human iPS cell-derived definitive endoderm cells were dissociated and suspended at 5 × 10⁶ cells/mL in the intestinal differentiation medium (advanced DMEM/F12 (Cat. No. 12634028; Thermo Fisher Scientific) supplemented with 2 % FBS and 1× GlutaMAX) supplemented with 100 ng/mL FGF2 and 10 µM Y-27632 (Cat. No. 034-24024; FUJIFILM Wako Pure Chemical). Then, a suspension medium (10 µL) was injected into the Matrigel-coated top channel of the microfluidic device. After 1 hr, the intestinal differentiation medium including 100 ng/mL FGF2 and 10 µM Y-27632 was added into the top channel of the microfluidic device and cultured for 24 hrs. To perform the midgut cell differentiation, human iPS cell-derived definitive endoderm cells were treated with 100 ng/mL FGF2 (Cat. No. 160-0010-3; KATAYAMA CHEMICAL INDUSTRIES Co., Ltd.) in the intestinal differentiation medium for 3 days.

To perform small intestinal progenitor cell differentiation, the cells were treated with 50 ng/mL EGF (Cat. No. AF-100-15; PeproTech) and 20 ng/mL IGF-1 (Cat. No. AF-100-11; PeproTech) in the intestinal differentiation medium containing 25% of Wnt-3A, R-spondin 3, and noggin-conditioned medium for 5 days. Wnt-3A, R-spondin 3, and noggin-conditioned medium was prepared by L-WRN cells (Cat. No. CRL-3276; American Type Culture Collection (ATCC)). To perform the small intestinal tissue differentiation, the cells were treated with 10 ng/mL EGF, 15 µM forskolin (Cat. No. 063-02193; FUJIFILM Wako Pure Chemical), 10 µM PD98059 (Cat. No. S1177; Selleck), 2.5 µM 5-aza-2'-dC (Cat. No. 018-20941; FUJIFILM Wako Pure Chemical), 0.25 µM A-83-01 (Cat. No. 035-24113; FUJIFILM Wako Pure Chemical), 2.5 µM (2'Z,3'E)-6-Bromoindirubin-3'-oxime (BIO) (Cat. No. 361550; Sigma-Aldrich), and 5 µM (3,5-Difluorophenylacetyl)-Ala-Phg-OBu^{t} (DAPT) (Cat. No. 3219-v; PEPTIDE INSTITUTE) in the intestinal differentiation medium containing 50 % of Hepatocyte Culture Media BulletKit (Cat. No. CC-3198; LONZA) for 12 days. The medium flow was maintained by a peristaltic pump (Programmable motor controller, Cat. No. RE-C200/C201 and Ring Pump, Cat. No. RP-TXP5F-P03A-DC3VS, Aqua Tech) at a flow rate of 30 µL/hr or a seesaw rocking shaker (Infinity Rocker Mini, Next Advance) for 20 days (day 4 to day 24).

To differentiate human iPS cells into small intestinal epithelial cells in the 24-well plate, human iPS cell-derived definitive endoderm cells were dissociated and suspended at 0.3 × 10⁶ cells/mL in the intestinal differentiation medium. Then, the suspension medium was seeded into the Matrigel-coated 24-well plate (500 µL/well). The growth factors or compounds used for intestinal differentiation in the 24-well plates are the same as those used in the microfluidic devices.

### <RT-qPCR>

Total RNA of small intestine tissue-on-a-chip was isolated using ISOGENE (Cat. No. 319-90211; NIPPON GENE). Total RNA from human small intestine was purchased from Takara Bio USA, Inc. (Cat. No. 636539). cDNA was synthesized with the Superscript VILO cDNA Synthesis Kit (Cat. No. 11754250; Thermo Fisher Scientific). RT-qPCR was performed with SYBR Green PCR Master Mix (Cat. N. 4385614; Thermo Fisher Scientific) using a StepOnePlus real-time PCR system, QuantStudio 1, or QuantStudio 3 Real-Time PCR System (Thermo Fisher Scientific). The relative quantification of target mRNA levels was performed using the 2^{-ΔΔCt} method. The values were normalized to the housekeeping gene glyceraldehyde 3-phosphate dehydrogenase (GAPDH). The primer sequences are summarized in Tables 1-1 and 1-2. For GAPDH, primers with sequences shown in Table 1-1 were used in all experiments except for Example 10, and only in Example 10 was a primer with sequences shown in Table 1-2 used.

**[Table 1-1]**

| Primers used in RT-qPCR analysis | | |
|---|---|---|
| Gene symbol | Primers (forward/reverse; 5'→3') | SEQ ID NO |
| GAPDH | GGTGGTCTCCTCTGACTTCAACA/GTGGTCGTTGAGGGCAATG | 1/2 |
| ALB | TGCAACTCTTCGTGAAACCTATG/ACATCAACCTCTGGTCTCACC | 3/4 |
| VIL1 | CTGAGCGCCCAAGTCAAAG/AGCAGTCACCATCGAAGAAGC | 5/6 |
| SI | TCCAGCTACTACTCGTGTGAC/CCCTCTGTTGGGAATTGTTCTG | 7/8 |
| LYZ | CTTGTCCTCCTTTCTGTTACGG/CCCCTGTAGCCATCCATTCC | 9/10 |
| MUC2 | GAGGGCAGAACCCGAAACC/GGCGAAGTTGTAGTCGCAGAG | 11/12 |
| CHGA | TAAAGGGGATACCGAGGTGATG/TCGGAGTGTCTCAAAACATTCC | 13/14 |
| LGR5 | CTCCCAGGTCTGGTGTGTTG/GTGAAGACGCTGAGGTTGGA | 15/16 |
| OSTA | CTGGGCTCCATTGCCATCTT/CACGGCATAAAACGAGGTGAT | 17/18 |
| OSTB | TCCAGGCAAGCAGAAAAGAAA/ACTGACAGCACATCTCTCTCT | 19/20 |
| ASBT | GGGAGAGGGACTTCCTCAAG/CCCTGACCATGGTGCTTACT | 21/22 |
| FGF19 | CGGAGGAAGACTGTGCTTTCG/CTCGGATCGGTACACATTGTAG | 23/24 |
| CYP3A4 | AAGTCGCCTCGAAGATACACA/AAGGAGAGAACACTGCTCGTG | 25/26 |
| CDX2 | TCCGTGTACACCACTCGATATT/GGAACCTGTGCGAGTGGAT | 27/28 |
| REG4 | CTGCTCCTATTGCTGAGCTG/GGACTTGTGGTAAAACCATCCAG | 29/30 |
| GP2 | AATCAAACCCATGCCATCTACAA/CACACTGACGTTCAGGGAACT | 31/32 |
| DCLK1 | ACTTCGACGAGCGGGATAAG/GGGCCTCAAAAGATCGGAACC | 33/34 |
| HCoV-229E | GTCGTCAGGGTAGAATACCTTA/CCCGTTTGCGCTTTCTAGT | 35/36 |
| IL-8 | TTTTGCCAAGGAGTGCTAAAGA/AACCCTCTGCACCCAGTTTTC | 37/38 |
| IL1B | ATGATGGCTTATTACAGTGGCAA/GTCGGAGATTCGTAGCTGGA | 39/40 |

**[Table 1-2]**

| Gene symbol | Primers (forward/reverse; 5'→3') | SEQ ID NO |
|---|---|---|
| *GAPDH* | GGAGCGAGATCCCTCCAAAAT/GGCTGTTGTCATACTTCTCAT GG | 41/42 |
| *CYP3A4* | AAGTCGCCTCGAAGATACACA/AAGGAGAGAACACTGCTCGT G | 43/44 |
| *UGT1A1* | TAAGTGGCTACCCCAAAACG/GCTTTGCATTGTCCATCTGA | 45/46 |
| *SULT1B1* | TTGAACAGTTCCATAGCAGACC/CAGGGAGAGTCATTTCCAAC ATT | 47/48 |
| *ABCB1*/*MDR1* | GCCAAAGCCAAAATATCAGC/TTCCAATGTGTTCGGCATTA | 49/50 |
| *ABCC1*/*MRP1* | CTCTATCTCTCCCGACATGACC/AGCAGACGATCCACAGCAAA A | 51/52 |
| *ABCC2*/*MRP2* | TGAGCAAGTTTGAAACGCACAT/AGCTCTTCTCCTGCCGTCTCT | 53/54 |
| *ABCG2*/*BCRP* | TGCAACATGTACTGGCGAAGA/TCTTCCACAAGCCCCAGG | 55/56 |
| *SLCO2B1*/*OATP2B1* | TTTGCCCACAACAGCAACTC/TGGTTAATGTCCACATAAAGGC G | 57/58 |
| *SLC15A1*/*PEPT1* | TCTTTGGTTATCCCCTGAGCA/GGCGGTGGACAGGTTATCATC | 59/60 |
| *FABP2* | ATGGCGTTTGACAGCACTTG/TCAGTTCCGTCTGCTAGATTGTA | 61/62 |

### <Immunofluorescence staining>

For immunofluorescence staining, the small intestine tissue-on-a-chip was fixed with 4% paraformaldehyde for 15 min. The small intestine tissue-on-a-chip was harvested and used for preparation of frozen sections (5 µm). For antigen retrieval, the sections were processed by heating at 70°C in HistoVT One (Cat. No. 06380-05; Nacalai Tesque) for 30 min. The sections were permeabilized using Tris Buffered Saline with 0.1%-Tween 20 Detergent (Cat. No. 34874-91; Nacalai Tesque) for 10 min and blocked using Blocking One Histo (Cat. No. 06349-64; Nacalai Tesque) for 45 min. The sections were incubated in Tris Buffered Saline with 0.1%-Tween 20 Detergent with or without primary antibodies for 2 hr at room temperature. The sections were washed with PBS and incubated in Tris Buffered Saline with 0.1%-Tween 20 Detergent containing Alexa Fluor 488- or 594-conjugated secondary antibodies for 45 min at room temperature. The sections were finally washed and mounted with Fluoro-KEEPER Antifade Reagent, Non-hardening Type with DAPI (Cat. No. 19178-91; Nacalai Tesque) and analyzed using a BZ-X700 (Keyence Corporation) or an FV3000 confocal microscope (Evident). Antibodies are summarized in Table 2.

**[Table 2]**

| Antibodies used in immunofluorescence staining | | |
|---|---|---|
| | Company | Cat. No |
| Villin | Abcam | ab109516 |
| Villin | Santa Cruz Biotechnology | sc-58897 |
| MUC2 | Santa Cruz Biotechnology | sc-515032 |
| LYZ | Santa Cruz Biotechnology | sc-518012 |
| CHGA | Santa Cruz Biotechnology | sc-393941 |
| CYP3A4 | Proteintech | 67110-1-Ig |
| Ki67 | Abcam | ab15580 |
| Vimentin | Santa Cruz Biotechnology | sc-6260 |
| ACTA2 | Proteintech | 14395-1-AP |
| COL1A1 | Proteintech | 14695-1-AP |

### <Quantitation of protein expression; Automated capillary electrophoresis immunoassay>

The small intestine tissue-on-a-chip was lysed in RIPA buffer (Cat. No. 89900; Thermo Fisher Scientific) containing a protease inhibitor mixture (Cat. No. P8340, Sigma-Aldrich). After the centrifugation, the supernatants were collected. The quantitation of protein expression was performed using a Jess (ProteinSimple) with 12-230 kDa Separation Module (Cat. No. SM-WO01, ProteinSimple) as instructed. Antibodies used for this analysis are summarized in Table 3. The data were analyzed and visualized using Compass for Simple Western software (ProteinSimple). The Jess protein images were used throughout the specification.

**[Table 3]**

| Antibodies used in protein quantification | | |
|---|---|---|
| | Company | Cat. No |
| Villin | Abcam | ab109516 |
| CYP3A4 | Santa Cruz Biotechnology | sc-27639 |
| ANPEP | Proteintech | 14553-1-AP |
| Vimentin | Santa Cruz Biotechnology | sc-6260 |
| ACTA2 | Proteintech | 14395-1-AP |
| β-Actin | Sigma-Aldrich | A5441 |

### <RNA-seq analysis>

RNA was isolated from small intestine tissue-on-a-chip cultured under the static or flow conditions. Human Small Intestine Total RNA was purchased from BioChain Institute (Cat. No. R1234226-50). RNA integrity was assessed with a 2100 Bioanalyzer (Agilent Technologies). The library preparation was performed using a TruSeq stranded mRNA sample prep kit (Illumina) according to the manufacturer's instructions. Sequencing was performed on an Illumina NextSeq500. The fastq files were generated using bcl2fastq-2.20. Adapter sequences and low-quality bases were trimmed from the raw reads by Cutadapt v3.4 (M. Martin, Cutadapt removes adapter sequences from high-throughput sequencing reads. 2011 17, 3 (2011).). The trimmed reads were mapped to the human reference genome sequences (hg38) using STAR v2.7.9a (A. Dobin et al., STAR: ultrafast universal RNA-seq aligner. Bioinformatics 29, 15-21

(2013).) with the GENCODE (release 36, GRCh38.p13) (A. Frankish et al., GENCODE reference annotation for the human and mouse genomes. Nucleic Acids Research 47, D766-D773 (2019).) gtf file. The raw counts for protein-coding genes were calculated using htseq-count v0.13.5 (S. Anders, P. T. Pyl, W. Huber, HTSeq-a Python framework to work with high-throughput sequencing data. Bioinformatics 31, 166-169 (2015).) with the GENCODE gtf file. Differentially expressed genes were identified with DEseq2 v1.30.1 (M. I. Love, W. Huber, S. Anders, Moderated estimation of fold change and dispersion for RNA-seq data with DESeq2. Genome Biology 15 (2014).), and gene expression levels were determined as transcripts per million (TPM).

### <H&E staining>

The small intestine tissue-on-a-chip was fixed with 4% paraformaldehyde for 15 min. The small intestine tissue on the chip was harvested and used to prepare paraffin sections. Paraffin-embedded tissue sectioning and H&E staining were performed by the Applied Medical Research Laboratory.

### <Alcian blue staining>

The small intestine tissue-on-a-chip was fixed with 4% paraformaldehyde for 15 min. The small intestine tissue on the chip was harvested and used to prepare paraffin sections. Paraffin-embedded tissue sectioning and Alcian blue staining were performed by the Applied Medical Research Laboratory.

### <Masson's trichrome staining>

The small intestine tissue-on-a-chip was fixed with 4% paraformaldehyde for 15 min. The small intestine tissue on the chip was harvested and used to prepare paraffin sections. Paraffin-embedded tissue sectioning and Masson's trichrome staining were performed by the Applied Medical Research Laboratory.

### <Transmission electron microscopy>

Micro-small intestine systems were fixed with 2% glutaraldehyde in 0.1 M phosphate buffer at 4°C. After fixation, ultrathin sectioning, resin embedding, and electron microscopy were performed by the Hanaichi UltraStructure Research Institute.

### <scRNA-seq analysis>

Single-cell cDNA libraries were prepared using the 10x Genomics Chromium Next GEM Single Cell 3' Reagent Kits v3.1 (10x Genomics) according to the manufacturer's instructions. Briefly, micro-small intestine systems were trypsinized to dissociate into single cells. These cells were mixed, and the cell concentration was adjusted to 1,000 cells/µL before being applied to a Chromium 10X Single Cell System (10x Genomics) to generate gel-bead in emulsions (GEMs). cDNA libraries were prepared using a Chromium Next GEM Single Cell 3' GEM, Library & Gel Bead Kit v3.1 (10x Genomics) following the manufacturer's instructions. Sequencing was performed using a NovaSeq 6000 (Illumina). The 10x Genomics Cell Ranger pipeline (version 7.1.0) was used to perform sample demultiplexing, alignment to the hg38 human reference genome, barcode/UMI processing, and gene counting for each cell. Gene count matrix data were processed as follows. Briefly, low-quality cells were excluded based on the following criteria: (I) the number of detected genes was ≤ 200; (II) the number of UMI molecules detected within a cell was ≥ 100,000; and (III) the percentage of mitochondrial genes was ≥ 12.5%. Filtered unique molecular identifiers (UMI) feature-barcode matrices were processed using ICARUS v.2.0 (Jiang, A.D., et al., Nar Genomics and Bioinformatics. 5:lqad032 (2023)). Gene count matrices were normalized and scaled by a factor of 10,000. Dimensionality reduction with principal component analysis was performed using a set of 2,000 top variable genes (Seurat::FindVariableFeatures) (Hao, Y.H., et al., Nature Biotechnology. 42(2):293-304 (2023)). Clustering was performed with 15 principal components and a k-nearest neighbor value of 20 using the Louvain clustering algorithm. Cell type annotation was performed by scType (Ianevski, A., et al., Nature Communications. 13(1):1246 (2022)). The accession number for scRNA-seq data reported in this study is GSE244013. scRNA-seq expression datasets of human fetal small intestines were obtained from a public database (ArrayExpress accession number E-MTAB-9536) (Elmentaite, R., et al., Nature. 597(7875):250-255 (2021)).

### <In situ gene expression analysis>

*In situ* RNA expression analysis at a single-cell level was performed using Xenium (10x Genomics). Micro-small intestine systems were fixed with 4% paraformaldehyde for 15 min. FFPE tissue sections (5 µm thickness) were prepared on Xenium slides (10x Genomics) (supported by Tokushima Molecular Pathology Institute). Fixation and tissue permeabilization were performed according to the manufacturer's protocol (Cat. No. CG000581, Rev A, 10x Genomics). Probe Hybridization Mix was prepared using pre-designed panels (Xenium Human Colon Gene Expression and Xenium Human Skin Gene Expression panels) according to the user guide (Cat. No. CG000582, Rev A, 10x Genomics). The prepared probes were hybridized at 50°C overnight. Post-hybridization wash (37°C for 30 min), ligation (37°C for 2 h), and amplification (30°C for 2 h) were performed according to the user guide. Autofluorescence quenching and nuclei staining were conducted in the dark. Using the prepared slide, fluorescent probe hybridization and imaging were conducted using the Xenium Analyzer (onboard analysis: version 1.8.2.1, software: version 1.7.1.0, 10x Genomics). The output images and expression profiles were evaluated by Xenium Explorer (version 2.0.0, 10x Genomics). After the Xenium run, H&E staining was performed on the Xenium slide. For quality control of Xenium analysis, we checked the output summary HTML file from the Xenium Analyzer and confirmed that there was no error reported. *In situ* gene expression profiling was supported by KOTAI Biotechnologies.

### <HCoV-229E infection>

Human coronavirus 229E (HCoV-229E) (Cat. No. VR-740) was purchased from American Type Culture Collection (ATCC). HCoV-229E were produced (was propagated?) in MRC-5 (Cat. No. CCL-171, ATCC) cells, and stored at -80°C until use. MRC-5 cells were cultured with RPMI-1640 (FUJIFILM Wako Pure Chemical Corporation) supplemented with 10% fetal bovine serum and 1% penicillin/streptomycin. HCoV-229E (0.1 MOI)-containing medium was injected into the top or bottom channel of small intestine tissue-on-a-chip. One day post-infection (1 dpi), the culture medium was replaced with fresh medium, and then cultured for 3 days.

### <RNA-seq analysis>

RNA was isolated from HCoV-229E-infected micro-small intestine systems. RNA integrity was assessed with a 2100 Bioanalyzer (Agilent Technologies). RNA-seq libraries were constructed using a TruSeq Stranded mRNA Sample Prep Kit (Illumina) according to the manufacturer's instructions. Sequencing was performed on an Illumina NextSeq 2000 with the paired-end mode. FASTQ files were generated using bcl2fastq-2.20. Adapter sequences and low-quality bases were trimmed from the raw reads using cutadapt ver v4.1 (Kechin, A., et al., Journal of Computational Biology. 24(11):1138-1143 (2017)). Trimmed reads were mapped to human reference genome sequences (hg38) using STAR ver 2.7.10a (Dobin, A., et al., Bioinformatics 29(1):15-21 (2019)) with GENCODE (release 32, GRCh38.p13) (Frankish, A., et al., Nucleic Acids Research. 47(D1):D766-D773 (2019)) GTF file. Uniquely and properly mapped reads were used for further analysis. Raw read counts were calculated using htseq-count ver. 2.0.2 (Anders, S., Pyl, P.T., and Huber, W. Bioinformatics. 31(2):166-169 (2015)) with the GENCODE GTF file. Differential expression analysis was performed by DESeq2 v1.34.0 (Love, M.I., Huber, W., and Anders, S. Genome Biology. 15 (12):550 (2014)) using the Wald test. p values were corrected for multiple testing using the Benjamini and Hochberg method and represented as the false discovery rate (FDR). Raw data from this study were submitted to the Gene Expression Omnibus (GEO) under accession number GSE263691.

### <Assay for drug-metabolizing enzyme activity>

To examine cytochrome P450 (CYP) activity, liquid chromatographic tandem mass spectrometric (LC-MS/MS) analysis was performed. LC-MS/MS analysis was performed using an LCMS-8060NX (Shimadzu Corporation). Before CYP substrate treatment, the small intestine tissue-on-a-chip was cultured with HBSS for 30 min. Then, the small intestine tissue-on-a-chip was cultured with HBSS containing 10 µM triazolam (Cat. No. 205-14221; FUJIFILM Wako Pure Chemical) (whose metabolite is α-hydroxy-triazolam, Cat. No. H2529; Sigma-Aldrich), diclofenac (Cat. No. D2508; Tokyo Chemical Industry) (whose metabolite is 4-hydroxy diclofenac, Cat. No. Cay10008518; Cayman Chemical) or 7-hydroxycoumarin (Cat. No. H0236, Tokyo Chemical Industry) (whose metabolite is 7-hydroxycoumarin glucuronide, Cat. No. Cay19882; Cayman Chemical). The half of medium was collected at 2, 4, and 6 hrs after treatment with the substrate. When collecting the supernatant, the same amount of culture medium containing the substrate was added. The collected supernatant was mixed with the same volume of acetonitrile. Samples were filtrated with Cosmonice Filter W of a pore size of 0.45 µm, then analyzed by LC-MS/MS to measure the concentration of substrate and their metabolites according to standard curve. Detailed information for the ionization mode and multiple-reaction monitoring (MRM) transition of the mass spectrometer is summarized in Table 4. The dwell time for each MRM transition was set at 100 msec. LC separations were carried out at 40°C with COSMOCORE(R) 2.6C18 Packed Column 2.1 mm I.D. × 50 mm (Cat. No. 12631-41; Nacalai Tesque). The mobile phase conditions are summarized in Table 5.

**[Table 4]**

| Detailed information of the ionization mode and multiple-reaction monitoring (MRM) transition | | | |
|---|---|---|---|
| | Ionization mode | MRM transition | Collision energy |
| triazolam | positive | 343.3/239.15 | -42 V |
| α-hydroxy-triazolam | positive | 359.1/176.1 | -28 V |
| diclofenac | negative | 294.9500>251.2000 | 11 V |
| 4'-hydroxydiclofenac | positive | 313.0000>232.2000 | -20 V |
| 7-hydroxycoumarin | positive | 163.1500>107.15 | -22 V |
| 7-hydroxycoumarin glucuronide | positive | 339.0000>163.1000 | -19 V |

**[Table 5]**

| Mobile phase conditions for LC-MS/MS | | |
|---|---|---|
| solvent A | 0.1% formic acid | |
| solvent B | acetonitrile | |
| T. flow: | 0.2 mL/min | |
| B. conc: | 10% | 0-1 min |
| | 10% → 30% | 1-1.5 min |
| | 30% | 1.5-2.5 min |
| | 30% → 95% | 2.5-3 min |
| | 95% | 3-4 min |
| | 95% → 10% | 4-4.5 min |
| | 10% | 4.5-5.5 min |
| sample injection | 5 µL | |

### <P450-Glo^{™} CYP3A4 Assay>

CYP3A4 activity was also measured by using P450-Glo^{™} CYP3A4 Assay Kits (Cat. No. V9001, Promega). The small intestine tissue-on-a-chip was treated with Luciferin-IPA and the luciferase intensity was measured with a luminometer (Lumat LB 9508, Berthold Technologies) according to the manufacturer's instructions.

### <Statistical analysis>

Assessment by statistical analysis was performed using an unpaired two-tailed Student's t-test. Statistical significance was assessed using one-way analysis of variance (ANOVA) followed by Tukey's post-hoc test.

### <Proteome analysis>

The small intestine tissue-on-a-chip was lysed in PTS buffer (12 mM SDC, 12 mM SLS, 100 mM Tris-HCl (pH 9.0)) containing a protease inhibitor mixture (Catalog No. P8340; Sigma-Aldrich). The cell lysate was subjected to ultrasonication and then centrifuged, and the resulting supernatant was collected. The supernatant, which was used as a protein sample, was treated by a modified protein aggregation capture method (TS Batth et al., Protein Aggregation Capture on Microparticles Enables Multipurpose Proteomics Sample Preparation. Mol Cell Proteomics 18,1027-1035 (2019)). Two types of Sera-Mag SpeedBead carboxylate-modified magnetic particles (hydrophilic particles, Catalog No. 45152105050250; hydrophobic particles, Catalog No. 65152105050250; Cytiva, Marlborough, MA, USA) were mixed in equal amounts and washed three times with distilled water. The beads were redissolved in distilled water to a concentration of 15 µg beads/µL. The protein sample was reduced and alkylated with tris(2-carboxyethyl)phosphine (TCEP) and 2-iodoacetamide (IAA) at final concentrations of 20 mM and 36 mM, respectively. The alkylated protein sample was mixed with 20 µL of the redissolved beads and ethyl alcohol at a final concentration of 75% (v/v). The resulting mixture was washed twice with 80% ethyl alcohol, and the supernatant was discarded. The sample was then suspended in 100 µL of 50 mM Tris-HCl (pH 8.0) containing 2 µL of trypsin/Lys-C Mix (Catalog No. V5072, Promega), and protein digestion was allowed to proceed overnight at 37°C. The digested sample was acidified with 20 µL of 5% trifluoroacetic acid (TFA) and desalted using an SDB-StageTip (J Rappsilber et al., Stop and go extraction tips for matrix-assisted laser desorption/ionization, nanoelectrospray, and LC/MS sample pretreatment in proteomics. Anal Chem 75, 663-670 (2003)). The peptide concentration was measured using a Quantitative Peptide Assay Kit (Catalog No. 23290, Thermo Fisher Scientific). 200 ng of desalted peptides in total were loaded onto an Aurora column (250 mm in length, 75 mm in inner diameter, IonOpticks) using nanoElute (Bruker) for separation, and analyzed using a timsTOF Pro2 (Bruker). As the mobile phase, 0.1% formic acid (solution A) and 0.1% formic acid-acetonitrile (solution B) were used, and the mobile phase was delivered at a flow rate of 400 nL/min with the following gradient: from 2% to 17% solution B for 60 min, from 17% to 25% solution B for 30 min, from 25% to 37% solution B for 10 min, from 37% to 80% solution B for 10 min, and 80% solution B for 10 min (120 min in total). The spray voltage was 1400 V, and the temperature of the interface heater was 180°C. To obtain MS and MS/MS spectra, PASEF (Parallel Accumulation Serial Fragmentation) combined with data-independent acquisition (DIA) was used (diaPASEF) (F Meier et al., diaPASEF: parallel accumulation-serial fragmentation combined with data-independent acquisition. Nat Methods 17, 1229-1236 (2020).). The diaPASEF settings were as follows: the cycle time of a single precursor ion scan was 1.7 sec, in which 16 diaPASEF scans were performed; the MS/MS isolation width was 25 m/z; the precursor ion ranges were 400 to 1200 m/z; and the ion mobility range was 0.57 to 1.47 V·s·cm⁻². The acquired DIA data were searched using DIA-NN (v1.81) (PMID: 31768060; Demichev et al., 2020) against the human data from UniProt/Swiss-Prot release 2022_03, with carbamidomethylation of cysteine set as a fixed modification and N-terminal acetylation of proteins and methionine oxidation set as variable modifications. Other DIA-NN parameters were as follows: Trypsin/P protease, one missed cleavage allowed, the peptide length set to 7-30 amino acids, the precursor m/z value range of 300-1800, precursor charge states of 1-4, fragment ion m/z range of 200-1800, and precursor FDR of 1%. "PG.MaxLFQ" was used as the representative protein area value for comparing protein expression levels.

### <Measurement of degree of orientation>

The small intestine tissue-on-a-chip stained with Vimentin was observed using a confocal laser scanning microscope FV3000 (Olympus Life Science). The captured images were binarized using ImageJ software, and thereafter, a fast Fourier transform was implemented using FiberOri8single03 to calculate the degree of orientation and the orientation intensity.

### Example 1: Generation of small intestine tissue-on-a-chip

To generate small intestine tissues-on-chips, protocols for inducing differentiation from human iPS cells to small intestinal epithelial cells were evaluated. Differentiation induction from human iPS cells to small intestinal epithelial cells was carried out in 24-well plates. Differentiation was induced using the following four protocols: condition 1 (protocol 1), which corresponds to a previously reported differentiation induction protocol using Activin A, FGF2, EGF, Forskolin, PD98059, 5-aza-2'-dC, and A-83-01 (Kabeya T, Mima S, Imakura Y, Miyashita T, Ogura I, Yamada T, Yasujima T, Yuasa H, Iwao T, Matsunaga T. Pharmacokinetic functions of human induced pluripotent stem cell-derived small intestinal epithelial cells. Drug Metab Pharmacokinet. 2020 Aug;35(4):374-382. doi: 10.1016/j.dmpk.2020.04.334. Epub 2020 May 16. PMID: 32651148.); condition 2 (protocol 2), which used HCM in combination with the basal medium of condition 1; condition 3 (protocol 3), which used Wnt-3A, R-spondin 3, noggin, BIO, and DAPT in addition to condition 1; and condition 4 (protocol 4), which used HCM in combination with the basal medium of condition 3. The overview of each protocol is shown in FIG. 1. The results revealed that the gene expression levels (as measured by RT-qPCR) of a small intestinal marker, Villin, observed under conditions 2 to 4 were all comparable to or higher than that observed under condition 1 (FIG. 3).

Differentiation induction of human iPS cells into small intestine tissue was carried out in microfluidic devices. Human iPS cells were cultured in a 12-well plate until they differentiated into definitive endoderm cells. Thereafter, the definitive endoderm cells were dissociated and seeded into the top channel of each microfluidic device, in which their differentiation into small intestine tissue was induced. FIG. 4 shows bright-field images of the cells cultured in the microfluidic devices, and FIG. 5 shows the amounts of RNA extracted from the cells collected after the differentiation induction. When the differentiation was induced under conditions 1 and 2, almost no cells survived in the microfluidic devices, and the amounts of collected RNA were small. In contrast, when differentiation was induced under conditions 3 and 4, the cells survived in the microfluidic devices. The cells cultured in the 24-well plate under condition 1 and the small intestine tissues-on-chips cultured in the microfluidic devices under conditions 3 and 4 were subjected to gene expression analysis (by RT-qPCR) (FIG. 6). The gene expression levels of Villin were comparable across all these three groups. The gene expression levels of SI, which is known to be expressed in mature small intestinal epithelial cells, and a drug-metabolizing enzyme CYP3A4 were high in the cells cultured in the microfluidic device under condition 4. These results suggest that condition 4 is optimal for generating human iPS cell-derived small intestine tissue in a microfluidic device.

### Example 2: Investigation on medium flow using small intestine tissues-on-chips

The effects of medium flow were investigated using small intestine tissues-on-chips. A peristaltic pump was connected to a bottom channel of each microfluidic device to cause medium to flow. As a result of gene expression analysis (by RT-qPCR), no differences were observed in the expression levels of markers of small intestinal epithelial cells, namely, an enterocyte marker (Villin), a goblet cell marker (MUC2), and an intestinal stem cell marker (LGR5) (FIG. 7). FIG. 8 shows bright-field images of small intestine tissues-on-chips. In statically cultured small intestine tissue-on-a-chip, cells did not acquire three-dimensional morphology, whereas, in the small intestine tissue-on-a-chip cultured under medium flow, cells formed raised structures. Observation of cross-sections of the small intestine tissues-on-chips after H&E staining revealed that the medium flow caused the raised structures like protrusions to be formed, and villus-like structures were identified (FIG. 9). The results of the gene expression analysis shown in FIG. 7 indicate that the medium flow did not affect the gene expression level of Villin, whereas the protein expression level of Villin (as measured by Jess) was significantly increased by the medium flow (FIG. 10). In contrast, the medium flow did not affect the protein expression level of CYP3A4 (as measured by Jess). The CYP3A4 activity was measured using P450-Glo^{™} CYP3A4 Assay, and the results revealed that the CYP3A4 activity was high with or without the medium flow (FIG. 11). Further, to examine the drug-metabolizing enzyme activities of CYP3A4, CYP2C9, and UGT1A1, substrates for the respective drug-metabolizing enzymes were applied to the small intestine tissues-on-chips, and the amounts of metabolites generated from the respective substrates were measured (by LC-MS/MS) (FIG. 12). The production levels of CYP3A4 and UGT1A1 metabolites were comparable between the static culture group and the medium flow group, whereas the production level of CYP2C9 metabolites was higher in the static culture group than in the medium flow group. These results suggest that the small intestine tissues-on-chips exhibit high CYP3A4, CYP2C9, and UGT1A1 activities regardless of whether medium flow is performed.

Markers of various cells constituting the small intestinal epithelium, namely, an enterocyte marker (Villin), a goblet cell marker (MUC2), a Paneth cell marker (lysozyme), a neuroendocrine cell marker (CHGA), and a proliferating cell marker (Ki67), were subjected to immunofluorescence staining (FIG. 13). The medium flow increased the expression of Villin and promoted its localization to the apical side. Under medium flow, MUC2-positive goblet cells, LYZ-positive Paneth cells, CHGA-positive enteroendocrine cells, and Ki67-positive proliferating cells were observed abundantly (FIG. 13). The expression of CYP3A4 was high in both the statically cultured small intestine tissue-on-a-chip and the flow-cultured small intestine tissue-on-a-chip (FIG. 13). Next, Alcian blue staining (to stain mucin) was performed (FIG. 14). In the small intestine tissue-on-a-chip cultured under medium flow, mucin-producing goblet cells stained in blue and a mucus layer (indicated by a black arrowhead) were observed. These results suggest that the small intestine tissue-on-a-chip cultured under medium flow includes a plurality of types of cells constituting the small intestinal epithelium and thus exhibits morphology similar to that of native small intestine tissue.

Next, whether small intestine tissue-on-a-chip can similarly be generated at different medium flow rates was examined (FIG. 15). The protein expression of Villin (as measured by Jess) remained high even at a slower flow rate (10 µL/hour) than the above-described flow rate (30 µL/hour), but decreased at a faster flow rate (100 µL/hour). The protein expression of CYP3A4 (as measured by Jess) was high at a faster flow rate (100 µL/hour). The protein expression levels of alanyl aminopeptidase, membrane (ANPEP) (as measured by Jess) were high at the above-described flow rate (30 µL/hour). The above examination revealed that the differentiation into the small intestine tissue-on-a-chip is feasible at any of the medium flow rates (10, 30, or 100 µL/hour). The examination also suggested that a low flow rate (10 µL/hour) is suitable for inducing the expression of the small intestinal marker Villin and a high flow rate (100 µL/hour) is suitable for inducing the expression of the drug-metabolizing enzyme CYP3A4.

### Example 3: Investigation of reason why medium flow promoted differentiation into small intestine tissue-on-a-chip

Further analysis was conducted to investigate the reason why medium flow promoted the differentiation into the small intestine tissue-on-a-chip. Masson's trichrome staining (mainly to stain collagen) was performed to examine localization of collagen fibers (FIG. 16). Collagen fibers, which support epithelial cells, were aligned on the basolateral side of the epithelial cell layer by the medium flow and exhibited a localization similar to that observed in native small intestine. Collagen, which is the main component of collagen fibers, is produced by fibroblasts. Thus, the presence of fibroblasts in the small intestine tissues-on-chips was examined. The protein expression level (as measured by Jess) of Vimentin, a fibroblast marker, did not change with or without medium flow. However, immunofluorescence staining revealed that Vimentin-positive cells were randomly distributed in the static culture group, whereas medium flow promoted their localization on the basolateral side of the epithelial cell layer (FIG. 17). The expression level of α-SMA, a myofibroblast marker, decreased under medium flow (immunofluorescence staining) (FIG. 17). Moreover, immunofluorescence staining of COL1A1 showed colocalization of COL1A1 with Vimentin (FIG. 18), suggesting that the Vimentin-positive fibroblasts localized on the basolateral side of the epithelial cell layer produced the COL1A1. GO enrichment analysis was performed, and the results revealed that the medium flow increased the expression levels of a group of genes associated with terms related to the regulation of extracellular secretions and structures in the small intestine tissue-on-a-chip (FIG. 19, right, underlined). These results suggest that, in the small intestine tissue-on-a-chip, the medium flow caused fibroblasts secreting collagen fibers to align on the basolateral side, whereby differentiation into the small intestine tissue was promoted.

### Example 4: Viral infection experiment using small intestine tissues-on-chips

A viral infection experiment was conducted using small intestine tissues-on-chips. Human coronavirus 229E (HCoV-229E) uses, as its infection receptor, aminopeptidase N (APN/CD13/ANPEP) highly expressed in the small intestine. The protein expression levels of ANPEP in the small intestine tissues-on-chips were examined, and it was found that ANPEP exhibited an increased expression level under medium flow (measured by Jess) (FIG. 20). The small intestine tissues-on-chips cultured under medium flow were infected with HCoV-229E by introducing HCoV-229E via either the top channel (from the apical side) or the bottom channel (from the basolateral side) (FIG. 21). The results revealed that the amount of viral genome (HCoV-229E mRNA) detected in the cells was higher when the cells were infected from the apical side than when the cells were infected from the basolateral side. The immune response to the viral infection (increased gene expression of IL-8 and IL-1β) was also higher in the group infected from the apical side than in the group infected from the basolateral side. These results demonstrate that the small intestine tissue-on-a-chip can also be used as an infection evaluation system for human coronavirus 229E (HCoV-229E).

### Example 5: Comparison between small intestine tissue-on-a-chip and native small intestine

Total RNA from small intestine tissue-on-a-chip cultured under medium flow and total RNA from native small intestine were compared by RNA-seq analysis. The results revealed that the expression levels of 4,510 genes were higher in the small intestine tissue-on-a-chip than in the total RNA from the native small intestine (FIG. 22). Enrichment analysis of the genes showing increased expression revealed that a group of genes associated with terms related to collagen fibrils, extracellular matrix (ECM), and negative regulation of apoptotic processes exhibited increased expression (FIG. 23). In the small intestine tissue-on-a-chip, growth factors (TGFB1, WNT5A, IGF2), ECM, and ECM degradation regulatory factors exhibited increased expression (FIG. 24). These results suggest that, in the small intestine tissue-on-a-chip, a group of genes related to ECM regulation tend to be expressed at higher levels than in total RNA from the native small intestine.

### Example 6: Proteome analysis

Proteome analysis was conducted for small intestine tissues-on-chips subjected to static culture (Static) or flow culture (Flow). The results revealed that medium flow increased the protein expression levels of 469 genes and decreased the protein expression levels of 344 genes (FIG. 25, left). Next, the Human Protein Atlas was used to determine in which organs the group of genes showing increased protein expression under flow culture (Flow) conditions are expressed (FIG. 25, right). (FIG. 25, right). As a result, the group of genes showing increased protein expression under flow culture (Flow) conditions were identified as a group of genes highly expressed in cell types present in the intestinal tract (gray columns).

Next, 3097 genes highly expressed in the normal small intestine were extracted from the Human Protein Atlas. The expression changes of these 3097 genes in the small intestine tissue-on-a-chip were visualized as a heatmap (FIG. 26, left). The protein expression levels of genes characteristic of the normal small intestine were increased by flow culture (Flow). 321 genes highly expressed in enterocytes of the normal small intestine were extracted from the Human Protein Atlas. The expression changes of these 321 genes in the small intestine tissue-on-a-chip were visualized as a heatmap (FIG. 26, right). The protein expression levels of many genes characteristic of enterocytes were increased by flow culture (Flow).

The protein expression changes of cytochrome P450s (CYPs), uridine diphosphate-glucuronosyltransferases (UGTs), sulfotransferases (SULTs), and carboxylesterases (CESs), which are drug-metabolizing enzymes, were visualized as a heatmap (FIG. 27, left). Flow culture (Flow) increased the protein expression levels of many drug-metabolizing enzymes. The ratio of the expression level of CES2, which is highly expressed in the small intestine, to that of CES1, which is highly expressed in the liver, is shown (FIG. 27, right). The protein expression level of CES2, which is a small intestine-specific carboxylesterase, was increased by flow culture (Flow).

The plot compares changes in mRNA expression and protein expression between the small intestine tissue-on-a-chip subjected to static culture (Static) and the small intestine tissue-on-a-chip subjected to flow culture (Flow) (FIG. 28, left). The mRNA expression change was calculated from the results of the RNA-seq analysis, and the protein expression change was calculated from the results of the proteome analysis. Flow culture (Flow) caused only minor changes in the mRNA expression, whereas it caused marked changes in the protein expression. The correlation coefficient between the mRNA expression change and the protein expression change was low (R²= 0.0794), suggesting the correlation between them is low. There were 703 genes (gray) for which protein expression levels were increased, whereas mRNA expression levels were not increased by flow culture (Flow). On the other hand, there were 401 genes (dark gray) for which protein expression levels were decreased, whereas mRNA expression levels were not decreased by flow culture (Flow).

An analysis using the Human Protein Atlas was conducted to identify the genes (703 genes, gray) for which protein expression levels were increased, whereas mRNA expression levels were not increased by flow culture (Flow) (FIG. 28, right). As a result, the group of genes showing increased protein expression under flow culture (Flow) were found to be a group of genes highly expressed in cells types present in the intestinal tract (gray columns).

### Example 7: Investigation of optimal culture conditions

The effects of the period of flow culture (Flow) on the structures and the functions of small intestine tissues-on-chips were investigated. FIG. 29 shows an overview of a differentiation induction protocol from pluripotent stem cells into small intestinal epithelial cells. The period of flow culture (Flow) in small intestine tissues-on-chips was shortened from the standard 20 days to 3, 7, or 12 days, and whether this affects the structures and the functions of the small intestine tissues-on-chips was examined. Bright-field images of the small intestine tissues-on-chips are shown in FIG. 30. In the small intestine tissue-on-a-chip subjected to flow culture (Flow) for 20 days, raised three-dimensional structures were observed, whereas such three-dimensional structures were not distinctly observed in the small intestine tissues-on-chips cultured under flow culture conditions for 3, 7, or 12 days. These results revealed that more than 12 days of culture are required to construct crypt-like and villus-like three-dimensional structures, and it is preferable to perform at least about 20 days of flow culture (Flow). The protein expression levels of Villin, ANPEP, and CYP3A4, which are highly expressed in small intestinal epithelial cells, were examined. The results are shown in FIG. 31. In particular, the protein expression levels of Villin and ANPEP were increased by 20 days of medium flow. In contrast, flow culture caused little change in the protein expression level of CYP3A4.

Next, conditions for culture medium used for inducing differentiation of intestinal progenitor cells into small intestine tissue were investigated. Flow culture (Flow) induced the formation of three-dimensional morphology in the small intestine tissues-on-chips under all of the following conditions: "standard medium used in combination with HCM (+HCM)", "medium without HCM (-HCM)", and "medium used in combination with William's E medium instead of HCM (+William's E Medium)" (FIG. 32). Accordingly, these results demonstrate that the use of hepatocyte culture medium (HCM or William's E medium) in combination with the basal medium used for culturing intestinal progenitor cells is not essential for forming three-dimensional morphology of small intestine tissue-on-a-chip.

### Example 8: Investigation of construction mechanism of small intestine tissue-on-a-chip

The small intestine tissue-on-a-chip was stained with Vimentin, which is a fibroblast marker (FIG. 33, upper right). In the group subjected to static culture (Static), Vimentin-positive cells were randomly distributed. On the other hand, in the group subjected to flow culture (Flow), Vimentin-positive cells were oriented along the flow of the medium (white arrow). Accordingly, the degree of orientation of Vimentin was calculated using FiberOri8single03 (FIG. 33, lower panels). The orientation intensity was 1.05 in the group subjected to static culture (Static) and 1.21 in the group subjected to flow culture (Flow), indicating that flow culture resulted in a higher intensity. These results demonstrate that medium flow (Flow) causes Vimentin-positive fibroblasts in the small intestine tissue-on-a-chip to be oriented along the flow of the medium.

Matrix metalloproteinases (MMPs) are enzymes that degrade extracellular matrix components such as collagen. Previous studies reported that MMPs promote fibroblast migration. Proteome analysis revealed that medium flow increased the protein expression level of MMP-1, which degrades type I collagen, in the small intestine tissue-on-a-chip (FIG. 34, upper panel). Investigation using GM6001, which is an MMP inhibitor, was conducted to examine the role of MMPs in the differentiation process of the small intestine tissue-on-a-chip (FIG. 34, lower panels). Although the medium flow induced construction of three-dimensional small intestine tissue in the microfluidic device, construction of three-dimensional small intestine tissue was inhibited when a medium containing GM6001 was used. Accordingly, this result suggests that MMPs play an important role in construction of three-dimensional small intestine tissue induced by medium flow. The above investigation suggests that flow culture (Flow) increases the MMP expression in the small intestine tissue-on-a-chip and causes fibroblasts to migrate to be oriented along the flow of the medium, thereby promoting the construction of three-dimensional small intestine tissue.

### Example 9: Gene expression analysis in small intestine tissue-on-a-chip cultured under medium flow

RT-qPCR analysis was conducted to examine the gene expression change over time in the small intestine tissues-on-chips (FIG. 35). POU class 5 homeobox 1 (POU5F1), which is an undifferentiated cell marker, and Mix paired-like homeobox (MIXL1), which is a mesendoderm cell marker, were highly expressed on day 0 and day 4 of differentiation induction and then their expression levels gradually decreased (FIGs. 35A and 35B). Subsequently, differentiation into endodermal cells and mesodermal cells was induced using medium containing 1-2% fetal bovine serum (FBS). Forkhead box A2 (FOXA2), which is an endoderm cell marker, transiently increased around day 8 of differentiation induction, followed by increase in the gene expression of Villin 1 (VIL1) and sucrase-isomaltase (SI), which are enterocyte markers (FIG. 35C). Similarly, T-box transcription factor T (TBXT), which is a mesodermal cell marker, transiently increased around day 8 of differentiation induction, followed by increase in the gene expression of Vimentin (VIM) and lymphatic vessel endothelial hyaluronan receptor 1 (LYVE1), which are mesenchymal cell markers (FIG. 35C). These results demonstrate that both small intestinal epithelial cells and mesenchymal cells were concurrently differentiated from human iPS cells in the small intestine tissues-on-chips cultured under medium flow.

### Example 10: Comparative analysis of small intestine tissue-on-a-chip, human native small intestine, and Caco-2 cells

RT-qPCR analysis was conducted to compare the gene expression in Caco-2 cells, which are widely used as a small intestinal epithelial cell model, and human native small intestine (FIG. 36). The gene expression of enterocyte markers was examined, and the results revealed that the expression levels of the enterocyte markers, VIL1 and fatty acid-binding protein 2 (FABP2), were higher in the small intestine tissue-on-a-chip than in the Caco-2 cells (FIG. 36A). Furthermore, mucin 2 (MUC2; a goblet cell marker), lysozyme (LYZ; a Paneth cell marker), regenerating family member 4 (REG4; a neuroendocrine cell marker), glycoprotein 2 (GP2; an M cell marker), and doublecortin-like kinase 1 (DCLK1; a tuft cell marker) showed negligible expression in the Caco-2 cells, whereas they were expressed in the small intestine tissue-on-a-chip. Still further, cytochrome P450 family 3 subfamily A member 4 (CYP3A4), CYP family 2 subfamily C member 9 (CYP2C9), UDP glucuronosyltransferase family 1 member A1 (UGT1A1), and sulfotransferase family 1B member 1 (SULT1B1), which are drug-metabolizing enzymes, showed low levels of expression in Caco-2 cells, whereas they exhibited high expression levels in the small intestine tissue-on-a-chip (FIG. 36B). The gene expression levels of ATP binding cassette subfamily B member 1 (ABCB1/MDR1), ABC subfamily C member 1 (ABCC1/MRP1), ABC subfamily C member 2 (ABCC2/MRP2), ABC subfamily G member 2 (ABCG2/BCRP), solute carrier organic anion transporter family member 2B1 (SLCO2B1/OATP2B1), and SLC family 15 member 1 (SLC15A1/PEPT1), which are transporters, in the small intestine tissue-on-a-chip were comparable to those in the Caco-2 cells (FIG. 36C). These results suggest that the small intestine tissue-on-a-chip can serve as a more functional model than the conventional intestinal model, Caco-2 cells.

### Example 11: Morphological analysis of small intestine tissue-on-a-chip

The effects of medium flow on the structure of enterocytes were more profoundly evaluated by electron microscopic analysis. In small intestine tissues-on-chips cultured under medium flow, the small intestinal epithelial cell layer was composed of uniform and columnar epithelial cells, and orderly arranged microvilli were observed on its surface (FIGs. 37A and 37B). Such mature epithelial cells were not observed in the static culture group. Also, in the small intestine tissues-on-chips cultured under medium flow, tight junctions (TJs), adherens junctions (AJs), and desmosomes (DSs) were orderly arranged, whereas they remained immature in the static culture group (FIG. 37C). These results suggest that the medium flow in the microfluidic device promoted maturation of the small intestinal epithelial cells.

### Example 12: In situ gene expression profiling in small intestine tissue-on-a-chip

Localization of various small intestinal epithelial cell markers in the small intestine tissue-on-a-chip cultured under medium flow was examined by in situ gene expression profiling at a single-cell level (FIGs. 38A and 38B). Solute carrier family 26 member 2 (SLC26A2) and solute carrier family 26 member 3 (SLC26A3), which are enterocyte markers, were expressed on the apical side of the small intestine tissue-on-a-chip, whereas SRY-box transcription factor 9 (SOX9), which is an intestinal progenitor cell marker, and leucine rich repeat containing G protein-coupled receptor 5 (LGR5), which is a stem cell marker, were expressed on the basolateral side (FIGs. 38C and 38D). Bestrophin 4 (BEST4), and CHGA, which is a neuroendocrine cell marker, were also expressed. These results indicate that polarized small intestinal epithelial layers could be constructed by the medium flow. Further, localization of stromal cell markers in the small intestine tissue-on-a-chip cultured under medium flow was also examined by in situ gene expression profiling at a single-cell level (FIGs. 39A and 39B). Cells positive for Vimentin (VIM), an intestinal fibroblast marker, were localized on the basolateral side of the small intestine tissue-on-a-chip (FIGs. 39C and 39D). In addition, platelet derived growth factor receptor (PDGFRA)^{high} cells that express PDGFRA, which is a telocyte marker, and PDGFRA^{low} cells positive for a frizzled-related protein (FRZB, a Wnt antagonist), which is a trophocyte marker, were also observed.

### Example 13: Gene expression analysis of fibroblasts constituting small intestine tissue-on-a-chip

The following analysis was conducted to investigate which intestinal fibroblast subtypes among the various fibroblasts constituting the intestinal tract are present in the small intestine tissue-on-a-chip. scRNA-seq analysis of the small intestine tissue-on-a-chip cultured under medium flow identified ten types of clusters including epithelial cells and stromal cells. Among these ten identified clusters, the stromal cell cluster was further subjected to subcluster analysis. The stromal cells were classified into three subclusters (FIG. 40A). These subclusters all expressed PDGFRA, a fibroblast marker (FIG. 40B). Subcluster 0 expressed R-spondin 3 (RSPO3), which plays an important role for maintaining intestinal stem cells. Subclusters 1 and 2 expressed bone morphogenetic protein 5 (BMP5), which plays an important role in small intestinal epithelial cell differentiation. These results demonstrate that fibroblasts that express growth factors important for maintenance and differentiation of small intestinal epithelial cells are present in the small intestine tissue-on-a-chip.

### Example 14: Viral infection experiment (HCoV-229E infection experiment) using small intestine tissue-on-a-chip

A human coronavirus 229E (HCoV-229E) infection experiment was conducted using small intestine tissues-on-chips, and the infected small intestine tissues-on-chips were subjected to RNA-seq analysis (FIG. 41). When the small intestine tissue-on-a-chip cultured under medium flow was infected with HCoV-229E from the apical side (Apical), changes in expression were observed in 182 genes, whereas no changes in gene expression were observed when infected from the basolateral side (FIG. 41A). The group of host genes associated with viral infection exhibited increased expression in the small intestine tissue-on-a-chip infected with HCoV-229E from the apical side (FIGs. 41B and 41C). These results demonstrate that the small intestine tissue-on-a-chip of the present invention can be used for evaluating viral infection.

### Example 15: Investigating using various human pluripotent stem (ES) cells

The present example examined whether small intestine tissue-on-a-chip having functions comparable to those obtained using human iPS cells could also be produced using human ES cells (FIGs. 42 and 43). In the case where a human ES cell line KhES-3 was used, small intestine tissues-on-chips having three-dimensional structures with villus-like protrusions (FIGs. 42A and 42B) could be constructed by the action of interstitial flow. The small intestine tissues-on-chips differentiated from KhES-3 exhibited high mucus production capacity and enterocyte marker expression (FIGs. 42C and 42D). In the small intestine tissues-on-chips differentiated from KhES-3, a fibroblast layer and collagen fibers were also formed beneath the epithelial cell layer (FIGs. 42E and 42F). Also, in the case where a human ES cell line H9 was used, small intestine tissues-on-chips having a three-dimensional structure with villus-like protrusions (FIGs. 43A and 43B) could be constructed by the action of interstitial flow. The small intestine tissues-on-chips differentiated from H9 exhibited high mucus production capacity and expressed enterocyte markers (FIG. 43C, FIG. 43D). In the small intestine tissues-on-chips differentiated from H9, a fibroblast layer and collagen fibers were also formed beneath the epithelial cell layer (FIGs. 43E and 43F). These results demonstrate that the small intestine tissue-on-a-chip of the present invention can be produced not only using human iPS cells but also using human ES cells.

### INDUSTRIAL APPLICABILITY

According to the present invention, small intestinal epithelial cells can be produced from midgut cells using a method distinct from conventional methods. Also, according to the method using a microfluidic device, small intestinal epithelial cells and mesenchymal cells such as fibroblasts can be concurrently differentiated from pluripotent stem cells within a single microfluidic device. The mesenchymal cells produce niche factors and extracellular matrix components such as collagen, thereby allowing the small intestinal epithelial cells to exhibit polarity and functions similar to those observed in vivo. By using small intestine tissue that can exhibit functions comparable to or even exceeding those of native tissue, it becomes possible to perform drug discovery trials (such as pharmacokinetic studies, drug toxicity testing, and infection experiments) with higher accuracy as compared with conventional small intestine models. Drug discovery trials with high clinical predictability are expected to contribute to more efficient and simplified animal experiments and clinical trials. In addition, the small intestine tissue of the present invention hold potential as a source of small intestine tissue for regenerative medicine.

The present application claims priority based on Japanese Patent Application No. 2023-132372 filed in Japan (filing date: August 15, 2023), the disclosure of which is incorporated herein in its entirety by reference.

## Claims

1. A method for producing a small intestinal epithelial cell or a small intestine tissue from a midgut cell, comprising the following steps (1) and (2):
(1) culturing a midgut cell in a medium containing a Wnt agonist, a BMP inhibitor, and a growth factor to obtain an intestinal progenitor cell; and
(2) culturing the intestinal progenitor cell obtained in step (1) in a medium containing a growth factor, an adenylate cyclase activator, an MEK inhibitor, a DNA methylation inhibitor, a TGF-β inhibitor, a GSK3β inhibitor, and a γ-secretase inhibitor to obtain a small intestinal epithelial cell or a small intestine tissue.

2. The method according to claim 1, wherein the Wnt agonist includes a Wnt protein and an R-spondin.

3. The method according to claim 1 or 2, wherein the Wnt agonist and the BMP inhibitor are obtained from a cell expressing them.

4. The method according to any one of claims 1 to 3, wherein the growth factor used in each of steps (1) and (2) includes EGF.

5. The method according to any one of claims 1 to 4, wherein steps (1) and (2) are performed in a microfluidic device.

6. The method according to claim 5, wherein steps (1) and (2) each includes a step of performing flow culture.

7. The method according to claim 6, wherein the flow culture is performed by a peristaltic pump or a shaker.

8. The method according to any one of claims 1 to 7, further comprising, prior to step (1), a step of inducing differentiation of a definitive endoderm cell into a midgut cell.

9. The method according to claim 8, further comprising a step of inducing differentiation of a pluripotent stem cell into a definitive endoderm cell.

10. A small intestinal epithelial cell or a small intestine tissue obtainable by the method according to any one of claims 1 to 9, or a microfluidic device supporting the small intestinal epithelial cell or the small intestine tissue.

11. A small intestine tissue having all of the following features (A) to (F) or a microfluidic device supporting the small intestine tissue:
(A) the small intestine tissue has an enterocyte;
(B) the small intestine tissue has a fibroblast;
(C) the small intestine tissue has a collagen fiber;
(D) the small intestine tissue expresses one or more genes selected from the group consisting of TGFB1, Wnt5A, and IGF2;
(E) the small intestine tissue expresses one or more genes selected from the group consisting of MMP1, COL3A1, and TIMP1; and
(F) the small intestine tissue has drug-metabolizing enzyme activity.

12. An agent for transplantation therapy, comprising the small intestinal epithelial cell or a small intestine tissue according to claim 10 or 11.

13. A method for screening a therapeutic or prophylactic medicament for a viral infection, comprising the following steps (I) to (III):
(I) contacting the small intestine tissue according to claim 10 or 11 with a virus in the presence or absence of a test substance, and culturing the small intestine tissue;
(II) evaluating the degree of infection with the virus in the small intestine tissue; and
(III) when it is evaluated in step (II) that the degree of infection with the virus is lower in the presence of the test substance than in the absence of the test substance, selecting the test substance as a candidate substance for a therapeutic or prophylactic medicament for the viral infection.

14. The method according to claim 13, wherein the virus belongs to the family *Coronaviridae.*

15. A method for evaluating the degree of metabolism of a test substance, comprising the following steps (i) to (iii):
(i) contacting a test substance with the small intestine tissue according to claim 10 or 11;
(ii) measuring the activity level of a drug-metabolizing enzyme; and
(iii) based on the activity level of the drug-metabolizing enzyme measured in step (ii), evaluating the degree of metabolism of the test substance catalyzed by the drug-metabolizing enzyme.

16. The method according to claim 15, wherein the drug-metabolizing enzyme is at least one selected from the group consisting of CYP3A4, UGT1A1, and CYP2C9.
